(19) Europäisches Patentamt
European Patent Office
Office européen des brevets

(11) EP 2 261 370 B1

(12) EUROPEAN PATENT SPECIFICATION

(45) Date of publication and mention of the grant of the patent:
**24.08.2016 Bulletin 2016/34**

(51) Int Cl.:
***G06F 19/20*** *(2011.01)* ***C12Q 1/68*** *(2006.01)*

(21) Application number: **10164051.4**

(22) Date of filing: **27.05.2010**

(54) **Method for detecting abnormal spots of nucleic acid microarray**

Verfahren zur Erkennung abnormaler Punkte eines Nukleinsäuremikroarrays

Procédé de détection de tâches anormales dans un microréseau d'acides nucléiques

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **27.05.2009 JP 2009128162**
**24.05.2010 JP 2010118362**

(43) Date of publication of application:
**15.12.2010 Bulletin 2010/50**

(73) Proprietors:
- **Fujifilm Corporation**
  **Minato-ku**
  **Tokyo (JP)**
- **National University Corporation**
  **Tokyo Medical and Dental University**
  **Bunkyo-ku**
  **Tokyo 113-8510 (JP)**

(72) Inventors:
- **Yoshida, Junya**
  **Kanagawa (JP)**
- **Kanehara, Hideyuki**
  **Kanagawa (JP)**
- **Ujihara, Dai**
  **Kanagawa (JP)**
- **Inazawa, Johji**
  **Tokyo (JP)**
- **Imoto, Issei**
  **Tokyo (JP)**

(74) Representative: **Hoffmann Eitle**
**Patent- und Rechtsanwälte PartmbB**
**Arabellastraße 30**
**81925 München (DE)**

(56) References cited:
**WO-A2-2007/033210**

- **PETERSON G ET AL: "A co-printed oligomer to enhance reliability of spotted microarrays" JOURNAL OF MICROBIOLOGICAL METHODS, ELSEVIER, AMSTERDAM, NL LNKD- DOI:10.1016/J.MIMET.2009.02.014, vol. 77, no. 3, 11 March 2009 (2009-03-11) , pages 261-266, XP026127244 ISSN: 0167-7012 [retrieved on 2009-03-11]**
- **RITARI JARMO ET AL: "Application of hybridization control probe to increase accuracy on ligation detection or minisequencing diagnostic microarrays" BMC RESEARCH NOTES, BIOMED CENTRAL LTD, GB, vol. 2, no. 1, 14 December 2009 (2009-12-14), page 249, XP021070089 ISSN: 1756-0500**
- **YIN B C ET AL: "A dual-probe hybridization method for reducing variability in single nucleotide polymorphism analysis with oligonucleotide microarrays" ANALYTICAL BIOCHEMISTRY, ACADEMIC PRESS INC, NEW YORK LNKD- DOI:10.1016/J.AB.2008.09.003, vol. 383, no. 2, 15 December 2008 (2008-12-15), pages 270-278, XP025585793 ISSN: 0003-2697 [retrieved on 2008-09-10]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 261 370 B1

## Description

BACKGROUND

1. Technical Field of the Invention

The invention relates to a method for detecting abnormal spots, which are unsuitable for being used in evaluation of a hybridization result, among spots being present on a nucleic acid microarray by using an abnormality detecting nucleic acid, which can be hybridized with all spots, at the time of using a nucleic acid microarray.

2. Description of the Related Art

A comparative genomic hybridization (CGH) method is a technique which can detect abnormality of the number of genomic copies in the entire chromosome within a short period of time (A. Kallioniemi et al., Science, 258, 818 - 821 (1992)). However, this CGH method has a problem in detection resolution because it cannot detect chromosomal aberration if the chromosomal aberration does not occur within a large region of generally from 5 to 10 Mb or more (J. Inasawa and M. Mizukuchi, Rinsho Kensa (Clinical Inspection), 49, 497 - 502 (2005)).

Contrary to this, an array CGH method has been developed as a new tool which can detect a genomic structural aberration occurring at a level of from some 10 kb to several Mb (D. Pinkel et al., Nat. Genet., 20, 207 - 211 (1998), and I. Inoue and J. Inasawa, Saibo Kogaku (Cell Engineering), 23, 355 - 361 (2004)).

In the array CGH method, CGH analysis is carried out using a microarray in which a large number of cloned DNA fragments are arrayed at a high density on a glass substrate or the like, instead of a chromosomal sample in the CGH method of the related art. By this technique, it becomes possible to detect abnormalities in the genome such as the number of copies of the genome exhaustively and at a high resolution, and to identify structural abnormality of the genome which causes a disease such as cancer.

A nucleic acid microarray which uses long chain nucleic acid (such as a cDNA or BAC clone) as a probe that cannot be chemically synthesized is produced by spotting probe nucleic acid on a substrate by a ink jet method, a pin array method or the like. There are cases in which the nucleic acid microarray produced by such a method is apt to cause variation in the amount of spot due to spotting error and the like and to generate an error of fluorescence signal caused by the gene to be measured.

On the other hand, quality inspection of the nucleic acid microarray is carried out by taking out the nucleic acid microarray for a certain number, actually carrying out hybridization using a standard sample or the like and detecting its fluorescence spot shape and labeled quantity value. This quality inspection is carried out mainly by a manufacturer who produces the nucleic acid microarray or a provider of the nucleic acid microarray.

As another quality inspection method, there is a method in which the nucleic acid microarray is stained with a dyestuff or the like. For example, PARAGON (TM) DNA Microarray Quality Control Staining Kit, manufactured by Molecular Probe, and the like are on the market. In this method, there is a problem in that it requires staining, scanning, washing and the like complex operations. Also, in order to use the nucleic acid microarray for the original purpose after staining the nucleic acid microarray, it is necessary to employ a washing step for removing the reacted dyestuff from the nucleic acid microarray and even the washing step is employed, it also poses a problem in that the dyestuff remains on the array.

In addition, the conventional quality inspection on the nucleic acid microarray is generally carried out by a sampling inspection. Since the inspection is carried out not on all of the nucleic acid microarrays is this method, there is a possibility of finding that they are rejected articles at the stage of using them by the user.

Narayanan et al. have reported (Journal of Microbiological Methods, 2009, 77, 261-266) that successful printing and hybridization is essential for efficient and reliable data acquisition in a spotted microarrays experiments. In this study it was demonstrated that printing a 25 mer (printed 25 mer) with a standard 70 mer probe in each spot followed by the use of a fluorescently labeled 25 mer complement in the hybridization mixture ensures monitoring overall printing quality of the chip. This system can also be used as a control to evaluate adequate hybridization, washing, and augment of spots to position the tracking grids during scanning. A print correction value incorporated in data analysis enhances consistency and reliability of results.

WO-A-2007/033210 concerns sequences and reagents for preparing microarrays with internal controls. Specifically, this document defines and provides sequences that are not present in the hybridizing mRNA or cDNA, and therefore can be used both as hybridization controls and for inter-spot normalization.

SUMMARY

In view of the above, the invention has been made. One exemplary embodiment of the invention may provide a nucleic acid analyzing method which reduces errors generated by variation in spotting amount on the nucleic acid microarray and the like.

Also, another exemplary embodiment of the invention may provide a method for inspecting the quality of nucleic acid microarrays, which can be carried out more simply and conveniently than the nucleic acid microarray quality inspection method of the related art.

Also, the inventors found a method which can carry out quality inspection by each spot unit by the use of a labeled abnormality detecting nucleic acid (labeled correction nucleic acid), and newly found a method which can carry out quality inspection by a new convenient method, to thereby achieve the invention.

According to a first aspect, the present invention provides a nucleic acid analyzing method for identifying pairs of spots comprising an abnormal spot in a nucleic acid microarray having a plurality of spots to which a probe nucleic acid is immobilized that is capable of detecting a target nucleic acid having a target sequence, a, wherein:

the plurality of spots includes a spot X1m on which is immobilised a probe nucleic acid including a probe sequence, a', complementary to the target sequence, a, and a sequence, b', different from the probe sequence, a', wherein m denotes an integer;
n spot pairs, each pair comprising a spot X1i and a spot X2i, the spots X1i and X2i each having a substantially identical probe nucleic acid and can be on the same nucleic acid array or different nucleic acid microarrays; and
wherein n is an integer of $\geq 2$, i is an integer, $1 \leq i \leq n$, and $1 \leq m \leq n$;

the method comprising:

hybridizing the sequence, b', with a sequence, b, by adding a labelled abnormality detecting nucleic acid, B, which is a nucleic acid having the sequence, b, which is capable of binding to the sequence, b', to the spot X1m on the nucleic acid microarray;
obtaining a labelled amount value Fc1m of the hybridized abnormality detecting nucleic acid, B, in the spot X1m;
obtaining a labelled amount value Fc2m of the abnormality detecting nucleic acid, B, from a spot X2m on which is immobilised a probe nucleic acid which is substantially identical to that of spot X1m and includes a sequence b' and which is present on the same array of the spot X1m or on a different array; and
determining whether or not the pair of spots X1m and X2m contains an abnormal spot, an abnormal spot being one having an abnormal hybridisation ability among spots present on the nucleic acid array, by:

calculating a comparative value $D_Bm$ which is a numerical value obtained by comparing the labelled amount value Fc1m and the labelled amount value Fc2m;
calculating a comparative value $D_Bi$ for each of the n spot pairs, the comparative value $D_Bi$ being a numerical value obtained by comparing an obtained labelled amount value Fc1i of the hybridized abnormality detecting nucleic acid, B, for the spot X1i on which is immobilised a probe nucleic acid including a sequence b'and an obtained labelled amount value Fc2i of the hybridized abnormality detecting nucleic acid, B, for the spot X2i on which is immobilised a probe nucleic acid including a sequence b'; and
comparing the comparative value $D_Bm$ with a representative value A, the representative value A being either an average value or median value of the n comparative values $D_B1$ to $D_Bn$;
wherein determining whether or not the pair of spots X1m and X2m contains an abnormal spot is based on the magnitude of the difference between the value $D_Bm$ and the representative value A.

Preferably the hybridizing of the sequence, b', with the sequence, b, is carried out simultaneously with hybridizing the probe sequence, a', with the target sequence, a; and
the obtaining of the labelled amount value Fc1m of the abnormality detecting nucleic acid, B, is carried out simultaneously with obtaining a labelled amount value F1m of the target nucleic acid.

Preferably each of the comparative values $D_Bi$ is calculated using the following Formula (1):

$$\text{Comparative Value } D_Bi = \text{Log2}\{(\text{labeled amount value Fc1i})/(\text{labeled amount value Fc2i})\} \quad (1)$$

wherein when the difference between the comparative value $D_Bm$ and the representative value, A, is larger than a standard deviation value of the n comparative values $D_B1$ to $D_Bn$, it is determined that the spot pair including the spots X1m and X2m is an abnormal spot pair.

In this case, it is further preferred that when the difference between the comparative value $D_Bm$ and the rep-

resentative value, A, is 0.6 or more, it is determined that the spot pair including the spots X1m and X2m is an abnormal spot pair.

Preferably the method further comprises the step of inspecting the quality of the nucleic acid microarray by indicating or eliminating pairs of spots detected as containing an abnormal spot.

Preferably the abnormality detecting nucleic acid, B, is a nucleic acid derived from a normal cell, a normal cell being one which has not undergone substantial mutation. In this case, the abnormality detecting nucleic acid, B, includes a repeating sequence.

Preferably the abnormality detecting nucleic acid, B, is a Cot-1 DNA.

Alternatively, the abnormality detecting nucleic acid, B, is a vector-derived nucleic acid.

Preferably 1 mol or more of the abnormality detecting nucleic acid, B, is used for 1 mol of the target nucleic acid.

According to a second aspect, the present invention provides a program that causes a computer to process data acquired in a method in accordance with the above first aspect, the date of processing comprising:

calculating each comparative value $D_B$i between the labelled amount value Fc1i and the labelled amount value Fc2i for the spot pair including the spot X1i and the spot X2i;
obtaining the representative value, A, based on the n comparative values $D_B$1 to $D_B$n; and
determining, based on the magnitudes of the comparative value $D_a$m and the representative value, A, as to whether or not the spot X1m is the abnormal spot.

In the above method, by comparing labeled amount values of the abnormality detecting nucleic acids which are hybridized with different spots and considering a comparison value thereof, it becomes possible to detect abnormal spots and reduce variation of data among nucleic acid microarrays. Thereby, an error in the labeled quantity value of the target nucleic acid can be reduced, and more accurate data can be obtained.

Further, with the above quality inspection method, for example, a manufacturer of the nucleic acid microarray carries out hybridization with the nucleic acid microarray using a labeled abnormality detecting nucleic acid and provides the user with the resulting labeled quantity values. Then, the user can know which spots are the spots having problems in view of quality inspection by comparing the data with the labeled quantity value of the labeled abnormality detecting nucleic acid hybridized together with the labeled target nucleic acid of the nucleic acid microarray used in the test. Thereby, the user can easily carry out quality inspection for each spot on the nucleic acid microarray. In addition, since the quality inspection method uses a labeled quantity value of the labeled abnormality detecting nucleic acid used in the test for the quality inspection, it can be carried out with adding only slight steps (most of them can be dealt with a computer program), without requiring a special reagent for the quality inspection and without adding a special step for the quality inspection.

BRIEF DESCRIPTION OF THE DRAWINGS

Fig. 1 is a schematic illustration, showing a nucleic acid microarray analyzing method according to an embodiment of the invention.
Figs. 2A to 2E are schematic illustrations showing aspects of the nucleic acid microarray analyzing method according to the invention.
Fig. 3 is a schematic illustration showing a quality control method according to an aspect of the invention.
Fig. 4 is a graph prepared by plotting Log Ratio (Female/Male) values obtained in Comparative Example 1 (the case of not eliminating abnormal spots).
Fig. 5 is a graph prepared by plotting Female/Male values obtained in Comparative Example 1 (the case of not eliminating abnormal spots).
Fig. 6 is a graph prepared by plotting Log Ratio (Female/Male) values obtained in Example 1 when abnormal spots were eliminated so that the abnormal spots became 3% of the whole.
Fig. 7 is a graph prepared by plotting the Log Ratio (Female/Male) values obtained in Example 1 when abnormal spots were eliminated so that the abnormal spots became 5% of the whole.
Fig. 8 is a graph prepared by plotting the Log Ratio (Female/Male) values obtained in Example 1 when abnormal spots were eliminated so that the abnormal spots became 10% of the whole.
Fig. 9 is a graph prepared by plotting the Log Ratio (Female/Male) values obtained in Example 1 when spot pairs having differences of 0.6 or more between comparative values and representative values were eliminated as abnormal spots.
Fig. 10 is a graph showing a relationship between an abnormal spot elimination ratio and a dispersion value (STDEV) in an autosomal region.

Fig. 11 is a graph prepared by plotting the Ratio (Female/Male) values obtained in Example 1 when abnormal spots were eliminated so that the abnormal spots became 3% of the whole.

Fig. 12 is a graph prepared by plotting the Log Ratio (Cot-1/Cot-1) of nucleic acid microarrays 1 and 2 in Example 2.

Fig. 13 is a graph showing a result of eliminating the spot pairs, which are problematic in view of quality control, from the plots of Fig. 12 (each eliminated spot pair has 0.6 or more in a difference between an average value of Log Ratio (Cot-1/Cot-1) and its Log Ratio (Cot-1/Cot-1)).

Fig. 14 is a graph prepared by plotting the Log Ratio (Female/Male) values calculated between the spot pairs of the nucleic acid microarrays 2 and 3 in Example 2.

Fig. 15 is a graph showing a result obtained by eliminating the spot pairs, which are problematic in view of quality inspection, calculated between the nucleic acid microarrays 1 and 2 and the nucleic acid microarrays 1 and 3, from the plots of Fig. 14.

Fig. 16 is a partially enlarged graph of Fig. 14.

Fig. 17 is a partially enlarged graph of Fig. 15.

Fig. 18 is a graph showing an example of the spot pair to be eliminated.

Fig. 19 is a graph prepared by plotting the Ratio (Female/Male) values obtained in Example 4 when abnormal spots were not eliminated.

Fig. 20 is a graph showing a result of eliminating abnormal spots in the same array or between different arrays in Example 4.

Fig. 21 is a graph prepared by plotting the Log Ratio (Female/Male) values in the case of not eliminating abnormal spots in Example 5 (in the case of using a normal cell-derived nucleic acid as the labeled abnormality detecting nucleic acid (labeled correction nucleic acid)).

Fig. 22 is a graph showing a result of eliminating abnormal spots from the plot of Fig. 21.

Fig. 23 is a graph showing a result of further eliminating abnormal spots from the plot of Fig. 22.

Fig. 24 is a graph prepared by plotting Log Ratio(Female/Male) values in the case of not eliminating abnormal spots in Example 6 (in the case of using a vector-derived nucleic acid as the labeled abnormality detecting nucleic acid (labeled correction nucleic acid)).

Fig. 25 is a graph showing a result of eliminating abnormal spots from the plot of Fig. 24.

Fig. 26 is a graph showing a result of further eliminating abnormal spots from the plot of Fig. 25.

DETAILED DESCRIPTION OF EMBODIMENTS OF THE INVENTION

(Description of Principle)

Exemplary embodiments of the invention will be described below with reference to the drawings. In this connection, the following embodiments are just exemplary embodiments of the invention, and the invention is not limited thereto.

In order to describe the nucleic acid analyzing method according to an embodiment of the invention, schematic illustrations are shown in Figs. 1 and 2.

Firstly, as shown in Fig. 1, a hybridization solution containing sample nucleic acids and an abnormality detecting nucleic acid is prepared.

In the embodiment of the invention, the nucleic acid analysis is carried out on two analytes (analyte (A1) and analyte (A2)). That is, as the sample nucleic acids, the sample nucleic acid (A1) is prepared from the analyte (A1), and the sample nucleic acid (A2) from the analyte (A2). The method for preparing sample nucleic acids will be described later. It is assumed that that a certain target nucleic acid (a) is contained in these sample nucleic acids (A1) and (A2).

In addition, an abnormality detecting nucleic acid (B) containing a sequence (b) different from the sequence of target nucleic acid (a) is prepared. It is preferable that the sequence (b) is a sequence which can bind to the probe nucleic acid immobilized to substantially all of the spots presenting on the nucleic acid microarray. Examples of such a sequence include a sequence containing a repeating sequence. It is preferable that the abnormality detecting nucleic acid (B) is a normal cell-derived nucleic acid, more preferably a vector-derived nucleic acid or Cot-1 DNA.

After carrying out labeling of these sample nucleic acids (A1) and (A2) and abnormality detecting nucleic acid (B) using a labeling compound which will be described later and further carrying out purification if required, hybridization solutions (A1) and (A2) containing the sample nucleic acids (A1) and (A2) which are labeled (labeled sample nucleic acids (A1) and (A2)) and the abnormality detecting nucleic acid (B) which are labeled (labeled abnormality detecting nucleic acid (B)) are respectively obtained.

Regarding the nucleic acid microarray, one array of the same type is prepared for each of the hybridization solutions. That is, the nucleic acid analysis according to the embodiment of the invention is based on the so-called monochromatic method. A nucleic acid microarray for carrying out analysis of the analyte (A1) is referred to as an array (A1), and a nucleic acid microarray for carrying out analysis of the analyte (A2) is referred to as an array (A2).

Each of these arrays (A1) and (A2) includes plural spots immobilized with a probe nucleic acid. In this connection, the same type of probe nucleic acid is arranged in the same manner in the arrays (A1) and (A2). Also, among the spots of arrays (A1) and (A2), an optional spot (X1) is immobilized with a probe nucleic acid containing a probe sequence (a') complementary to the target sequence (a). In addition, all of the spots including the optional spot (X1) are immobilized with a probe nucleic acid containing a sequence (b') different from the target sequence (a'). It is assumed that the sequence (b') can bind to the sequence (b) contained in the abnormality detecting nucleic acid (B).

Next, by respectively adding the labeled sample nucleic acids (A1) and (A2) to the arrays (A1) and (A2) and adding the labeled abnormality detecting nucleic acid (B) to both of the arrays (A1) and (A2), the target sequence (a) is hybridized with the probe sequence (a'), and the sequence (b) is hybridized with the sequence (b').

The adding method is not particularly limited, but it is preferable that the adding of the labeled sample nucleic acid (A1) and labeled abnormality detecting nucleic acid (B) to the array (A1) is carried out simultaneously, and the adding of the labeled sample nucleic acid (A2) and labeled abnormality detecting nucleic acid (B) to the array (A2) is carried out simultaneously.

In this embodiment, as shown in Fig. 1, a hybridization solution (A1) containing the labeled sample nucleic acid (A1) and labeled abnormality detecting nucleic acid (B) and a hybridization solution (A2) containing the labeled sample nucleic acid (A2) and labeled abnormality detecting nucleic acid (B), which are prepared in advance, are added to the arrays (A1) and (A2), respectively.

The hybridization can be carried out using a commercially available hybridizer. The temperature in carrying out the hybridization is preferably within the range of from 25 to 50°C, more preferably within the range of from 30 to 45°C, further preferably within the range of from 37 to 42°C.

Also, the hybridization time is set to a period of time of preferably from 8 to 96 hours, more preferably from 12 to 72 hours, further preferably from 16 to 48 hours.

After the hybridization, a washing may be carried out. The washing can be carried out using an SSC (Saline-sodium citrate solution; citrate buffer solution) solution, a formamide/SSC solution, an SSC/SDS (sodium dodecyl sulfate) solution and the like.

Regarding the hybridization, it is preferable that the target nucleic acid and probe nucleic acid hybridize under a stringent condition. As an illustrative hybridization condition, for example, there may be mentioned a hybridization reaction at 37°C for 16 hours and subsequent washing with (1) 5 ml of 2 x SSC solution at room temperature for 30 seconds, (2) 5 ml of 50% formamide/2 x SSC solution (pH 7.0) at 50°C for 15 minutes, (3) 5 ml of 2 x SSC/0.1% SDS solution (pH 7.0) at 50°C for 30 minutes and (4) 5 ml of 2 x SSC solution at room temperature for 5 minutes.

After the hybridization and washing, a labeled target nucleic acid-derived labeled amount contained in the labeled sample nucleic acid (A1), a labeled target nucleic acid-derived labeled amount contained in the labeled sample nucleic acid (A2) and a labeled amount derived from the labeled abnormality detecting nucleic acid (B) are read out from the respective spots of the arrays (A1) and (A2).

When a fluorescent compound is used as the label of the labeled abnormality detecting nucleic acid (B), respective values of the labeled amounts can be obtained, for example, by measuring fluorescence values using a fluorescence scanner or the like.

It is assumed that each of the arrays (A1) and (A2) has at least n spots (n denotes an integer). In the following description, the spots on the arrays (A1) and (A2) may be referred to as spots (X1i) and spots (X2i), respectively (i denotes an integer; $1 \le i \le n$).

The labeled sample nucleic acid (A1)-derived labeled amount value read out from a spot (X1i) on the array (A1) is referred to as (F1i), and the labeled abnormality detecting nucleic acid (B)-derived labeled amount value read out from the spot (X1i) on the array (A1) is referred to as (Fc1i).

On the other hand, the labeled sample nucleic acid (Al)-derived labeled amount value read out from a spot (X2i) on the array (A2) is referred to as (F2i), and the labeled abnormality detecting nucleic acid (B)-derived labeled amount value read out from the spot (X2i) on the array (A2) is referred to as (Fc2i).

Next, abnormal spots are detected by comparing the labeled amount values (Fc1i) of the labeled abnormality detecting nucleic acid (B) with the labeled amount values (Fc2i).

The abnormal spot means a spot which has an abnormal hybridization ability among spots being present on a nucleic acid microarray, which is unsuitable for being used in evaluation of a hybridization result and which is unsuitable for detecting the target sequence (a) contained in the sample nucleic acid. The abnormal spot is considered to be caused by having amounts of probe nucleic acid being unevenly immobilized in spots mainly at the time of producing the nucleic acid microarray. Comparing amounts of hybridized abnormality detecting nucleic acid between plural spots, it is originally desired that those values are constant, and if the amounts are different, it can be found that at least any one of the plural spots is an abnormal spot. Then, it can be estimated that a spot having a larger or smaller amount than a representative value or the like of the amounts of plural spots is an abnormal spot.

Comparison of the labeled amount value (Fc1i) and labeled amount value (Fc2i) is carried out between a spot pair having the same type of spots presenting on the array (A1) and array (A2). In the embodiment, it is assumed that the spots (X1i) and (X2i) on the array (A1) and array (A2) are the same type and constitute a spot pair. The same type of spots mean the spots immobilized with a substantially identical probe nucleic acid.

Illustratively, between a spot pair of the same type of spot (X1i) on the array (A1) and the same type of spot (X2i) on the array (X2), a comparative value ($D_B$i) is calculated by the following formula (1) for the labeled amount value (Fc1i) and labeled amount value (Fc2i). This comparison is carried out on all of the spot pairs on the arrays (Fig. 2A). That is, when there are n pairs (n is an integer of 2 or more) of spot pairs are present on the arrays (A1) and (A2), n comparative values are obtained.

$$\text{Comparative value } (D_B i) \text{ of abnormality detecting nucleic acid (B)} = \text{Log}_2\{(\text{labeled amount value (Fc1i)})/\text{labeled amount value (Fc2i)}\}$$

(Formula (1))

In this connection, the term "comparative value" as used in the embodiment of the invention means a numerical value obtained by comparing labeled amount values between spot pairs. In the embodiment, a Log value of a ratio of the labeled amount value (Fc1i) and the labeled amount value (Fc2i) is used as the comparative value. However, the method for obtaining the comparative value may be any method which can compare the labeled amount value (Fc1i) and the labeled amount value (Fc2i) by numerical values and can numerate a difference therebetween quantitatively. Examples of the other comparative values include a ratio of the labeled amount value (Fc1i) and the labeled amount value (Fc2i)., a difference between the labeled amount value (Fc1i) and the labeled amount value (Fc2i) and the like.

Next, a representative value (A) is calculated from the obtained n comparative values ($D_B$l) to ($D_B$n). Examples of the representative value include an average value of the n comparative values ($D_B$l) to ($D_B$n) and median value (median) of the n comparative values ($D_B$l) to ($D_B$n). The median value is a numerical value which is located in a position of a half of a set in which certain numerical values are arranged in order. In addition, a statistically calculated representative value or the like may be used.

Further, determining of an abnormal spot is carried out based on a difference from the representative value (A) of the comparative values ($D_B$l) to ($D_B$n) obtained as above.

For example, when a threshold value is set in advance and a difference between a comparative value ($D_B$i) of one spot pair and the representative value (A) is equal to or larger than the threshold value, it can be determined that the one spot pair is abnormal spot. The threshold value may be set, for example, to 0.6 or the like. Also, when the difference between the comparative value ($D_B$i) of one spot pair and the representative value (A) is larger than a standard deviation value of the n comparative values ($D_B$l) to ($D_B$n), it may be determined that the one pair is an abnormal spot.

Fig. 2 B is a graph schematically representing plotting of the comparative value ($D_B$) (Log ratio) calculated by the formula (1). In Fig. 2B, plots of the comparative values ($D_B$) when the representative value (A) is set to 0 are shown.

Since the same labeled abnormality detecting nucleic acid (B) is hybridized with substantially identical probes on the arrays (A1) and (A2), when immobilized amounts of the probe nucleic acid between the spots constituting the sot pair are the same, a hybridized amount of the labeled abnormality detecting nucleic acid (B) becomes the same between respective probe nucleic acids, and its Log ratio becomes zero, so that all of the points must be gathered on the axis X.

However, since there is variation in the amount of probe nucleic acid between spots in reality, there is a case in which a point separated from the axis X appears as shown in Fig. 2B.

This shows that both arrays are different in the hybridized amount of the labeled abnormality detecting nucleic acid. Assuming that hybridization of the labeled abnormality detecting nucleic acid (B) is substantially the same in the spots, this shows the nucleic acid microarrays are highly likely different in a spot amount of the probe nucleic acid.

In this connection, the range of becoming abnormal spots may be determined by limiting the number of abnormal spots based on the number of the whole spot pairs or the total number of spot pairs of the spots in interest, in determining the abnormal spots.

That is, in this case, the abnormal spots are determined by the following procedure.

Firstly, the representative value of the labeled amount values is calculated for the whole spot pairs or the spot pairs of the spots in interest, and a difference between the labeled amount value of each spot and the representative value is calculated.

Next, the differences are arranged in magnitude order. Based on the number of the whole spot pairs or the number of spot pairs of spots in interest, it may be determined that spots in order of difference from the largest difference or a certain ratio of spots are abnormal spots.

In the embodiment of the invention, the number of spots which corresponds to a ratio smaller than 30% may be regarded as the abnormal spots.

Also, it is more preferable that the differences are arranged in magnitude order, and that 10% of the number of the whole spot pairs or the total number of spot pairs of spots in interest are regarded as the abnormal spots in difference order from the largest difference.

Further, it is further more preferable that the differences are arranged in magnitude order, and that 5% of the number of the whole spot pairs or the total number of spot pairs of spots in interest are regarded as the abnormal spots in difference order from the largest difference.

In addition, it is most desirable that the differences are arranged in magnitude order, and that 3% of the number of the whole spot pairs or the total number of spot pairs of spots in interest are regarded as the abnormal spots in difference order from the largest difference.

In addition, the abnormal spots may also be determined by carrying out Fourier transformation on the whole spot pairs or a specified spot range and regarding the high frequency components thereof as the abnormal spots.

Next, the spots determined as abnormal spots are eliminated.

That is, the labeled amount values (1lm) and (F2m) of the target nucleic acid in the spot pairs containing the spots (X1m) and (X2m) determined as abnormal spots are not used in the subsequent data processing of the labeled amount values of the target nucleic acid, where m denotes a given integer.

Next, Log ratio is calculated based on the labeled amount values (F1i) of the labeled target nucleic acid on the array (A1) and the labeled amount values (F2i) of the labeled target nucleic acid on the array (A2) by the following formula (3) (see Fig. 2C). In this connection, the labeled amount values (F1m) and (F2m) of the target nucleic acid in the spot pairs containing the spots (X1m) and (X2m) determined as the abnormal spots are not included in this calculation as described above.

$$\text{Comparative value } (D_A i) \text{ of labeled target nucleic acid} = \text{Log}_2\{(\text{labeled amount value (F1i)})/(\text{labeled amount value (F2i)})\} \quad \text{(Formula (3))}$$

Fig. 2D is a graph schematically representing plots of the comparative values ($D_A$) (Log ratio) calculated by the formula (3).

In Fig. 2D, the comparative values ($D_A$m) of the labeled target nucleic acid in the spot pair containing the spots (X1m) and (X2m) which is determined as the abnormal spot is shown by a dotted circle. As shown in Fig. 2D, plot of the comparative value ($D_A$m) of the labeled target nucleic acid in the spot pair determined as the abnormal spot appears in most cases at a position deviated from the original position as it should be. That is, in the pattern of the schematic drawing shown in Fig. 2D, it is expected that the spot pair determined as the abnormal spot should be plotted originally on the axis X (namely, Log ratio (F2m/F1m) = zero).

In addition, for spots other than the spots (X1m) and (X2m) determined as the abnormal spots, labeled amount values (F1) and (F2) of the labeled target nucleic acid are corrected.

Illustratively, a corrected labeled amount value (F2'i) of the labeled amount value (F2i) is obtained by the following formula (2).

$$\text{Corrected labeled amount value (F2'i)} = \text{labeled amount value (F2i)} \times (\text{labeled amount value (Fc1i)}/\text{ labeled amount value (Fc2i)}) \quad \text{(Formula (2))}$$

The correcting is carried out by calculating the formula (2) on all of the spots or spots in interest on the nucleic acid arrays.

Fig. 2E is a drawing schematically representing plots of Log ratios of the labeled amount values (F1) and (F2') of the labeled target nucleic acid after the correction. By carrying out the correction, variation of data is reduced in comparison with Fig. 2D.

In this connection, in the embodiment of the invention, it is not always necessary to carry out such a correction of the labeled amount values (F1) and (F2). However, it is preferable to carry out such a correction in order to examine an amount of the target nucleic acid more accurately.

In the embodiment, correction of the labeled amount values of the labeled target nucleic acid is carried out after the determining of the abnormal spots. However, this order may be carried out in a reversed manner.

(Description of quality inspection method)

Next, a quality inspection method according to the embodiment of the invention will be described.

Fig. 3 is a drawing schematically representing procedure of the quality inspection method according to the embodiment.

As shown in Fig. 3, a nucleic acid microarray for quality inspection use (A0) is first prepared, and a hybridization solution containing the labeled abnormality detecting nucleic acid (B) is added to the nucleic acid microarray (A0) to hybridize the labeled abnormality detecting nucleic acid (B) with the probe nucleic acid.

Next, a labeled amount value Fc0 is obtained for each spot from the hybridized nucleic acid microarray (A0). For example, when a fluorescent compound is used as a label of the labeled abnormality detecting nucleic acid (B), the labeled amount value (Fc0) is obtained by measuring its fluorescence value using a fluorescence scanner or the like.

In the case of a nucleic acid microarray which is not for use in the calculation of labeled amount values for quality inspection use, namely, a nucleic acid microarray which hybridizes an actual sample (microarrays 1 to N (N is an integer of 2 or more) in the drawing), hybridization is carried out by the use of a labeled sample nucleic acid and a labeled abnormality detecting nucleic acid by the methods of Fig. 1 and Fig. 2 which use a labeled abnormality detecting nucleic acid (labeled correction nucleic acid). In the case of Fig. 3, differences from the spotted amount of the nucleic acid microarray 0 with the assumption that it is spotted normally can be obtained as numerical value data, by comparing the labeled amount value of the labeled abnormality detecting nucleic acid obtained therefrom with the labeled amount value derived from the labeled abnormality detecting nucleic acid for quality inspection use obtained from the nucleic acid microarray for quality inspection use, namely, the nucleic acid microarray 0. Thus, those in which such numerical data exceeded the range determined in advance can be eliminated from the subsequent data processing and determining of the results as non-standardized spots in terms of quality inspection.

It is preferable that the standard value is determined by the manufacturer of the nucleic acid microarrays or a provider of the nucleic acid microarrays. Alternatively, it may be determined by the user.

In this connection, it is preferable that the quality inspection is carried out on all of the spots on the nucleic acid microarray. Alternatively, it may be carried out on the spots in interest.

In addition, a numerical value obtained by testing several plates of nucleic acid microarrays may be used as the labeled amount value of the nucleic acid microarray 0. As the method for calculating numerical values in that case, it can be carried out by a method in which a representative value, such as the average value, is used, a method in which labeled amount values largely departing from the representative value are omitted and a representative value, such as the average value, is calculated from the remaining labeled amount values, and the like.

Also, the user can personally determine a labeled amount value of the labeled abnormality detecting nucleic acid for quality inspection use. For example, assuming that the nucleic acid microarrays 1 to n are used, the quality inspection can also be carried out by using the labeled amount value of the labeled abnormality detecting nucleic acid obtained from the nucleic acid microarray 1 as the standard value for quality inspection use and comparing the standard value with labeled amount values of respective labeled abnormality detecting nucleic acids obtained from the nucleic acid microarrays 2 to n.

In addition, like the cases shown in Fig. 1 and Fig. 2, after the quality inspection is performed, a microarray test may be carried out on the nucleic acid microarrays to be used for actual samples, namely, the nucleic acid microarrays 1 to n, abnormal spots among these nucleic acid microarrays are detected, and the detected abnormal spots may be eliminated or clearly indicated.

For example, spots having problems in view of quality inspection can be firstly eliminated by having the nucleic acid microarray 1 to undergo hybridization with a normal cell-derived labeled sample nucleic acid and a labeled abnormality detecting nucleic acid, having the nucleic acid microarray 2 to undergo hybridization with an abnormal cell-derived labeled sample nucleic acid and labeled a abnormality detecting nucleic acid and comparing the labeled amount values (fluorescence values) of the labeled abnormality detecting nucleic acids obtained from the respective nucleic acid microarrays, that is, by comparing labeled amount values of the labeled abnormality detecting nucleic acids between the nucleic acid microarray 0 and the nucleic acid microarray 1 and between the nucleic acid microarray 0 and the nucleic acid microarray 2.

Next, abnormal spots can be detected or clearly indicated by comparing the labeled amount values obtained from the labeled abnormality detecting nucleic acids of the nucleic acid microarray 1 and nucleic acid microarray 2. By doing this, in case where spotting amounts of the probe nucleic acid for certain specific spots vary in nucleic acid microarrays to be used for actual samples, namely, nucleic acid microarrays 1 to n from the standard value at the time of factory shipping these nucleic acid microarrays and where spotting amounts for the diversified spots are almost the same, there is a possibility that these cannot be detected as abnormal spots even if detection of abnormal spots was carried out by the comparison of labeled amount values of labeled abnormality detecting nucleic acids between nucleic acid microarrays 1 to n. However, according to the method of the embodiment, abnormal spots can be detected based on the variation in labeled amount value of labeled abnormality detecting nucleic acids at the time of factory shipping.

It is preferable that the labeled abnormality detecting nucleic acids to be used for the nucleic acid microarray 0 and those to be used for nucleic acid arrays 1 to n are those which were prepared by the same batch.

In the embodiment, the term "quality inspection" means that an inspection is carried out to determine as to whether or not the nucleic acid microarrays have the ability intended by the manufacturer when a nucleic acid microarray in which each spot on the nucleic acid microarray is assumed to be properly spotted is compared with a nucleic acid microarray under quality inspection, such as the nucleic acid microarray to be used by the user.

In the embodiment of the invention, inspection of a nucleic acid microarray can be carried out when the nucleic acid microarray is used by the user, namely, the quality inspection can be carried out on all nucleic acid microarrays. Also, the quality inspection can be carried out on each spot on the nucleic acid microarray. Thereby, an un-sampled nucleic acid microarray which could not be found by the usual sampling inspection can be inspected.

Also, in the related art, inspection of separate spots was possible, and inspection of all nucleic acid microarrays was possible by a method in which the probe nucleic acid bonded onto the nucleic acid microarrays is stained by a labeling compound having a staining action, but this method has various problems such as its requirement for staining reaction, washing reaction, frying step, read out of labeled amount values, elimination, washing and drying of staining compound from probe nucleic acid and the like considering many processes, as well as influence of labeled sample nucleic acid upon labeled amount values due to remaining of staining compound on the nucleic acid microarray, reduction of performance of the nucleic acid microarray due to leaving of probe nucleic acid from the nucleic acid microarray caused by the repetition of washing step, and the like.

However, since the quality inspection can be carried out at the time of use according to the quality inspection method of the embodiment, it is not necessary to stain the probe nucleic acid in advance with a labeling compound. Furthermore, the washing and drying for quality inspection are not necessary because the washing and drying are carried out together with the sample nucleic acid, so that the inspection can be carried out on each spot and on all of the nucleic acid microarrays. In addition, the labeled abnormality detecting nucleic acid used in the quality inspection can be used for the detection of abnormal spots and can also be used for the correction of labeled amount value of the labeled sample by using it as a labeled correction nucleic acid.

As shown in Fig. 3, a labeled abnormality detecting nucleic acid alone may be hybridized with the nucleic acid microarray 0, but the hybridization reaction may be carried out together with another nucleic acid different from the labeled abnormality detecting nucleic acid. For example, a labeled sample nucleic acid may be hybridized. In addition, among labeled sample nucleic acids, a normal cell-derived labeled sample nucleic acid can also be hybridized simultaneously. By doing this way, for example, labeled amount value of the normal cell-derived sample nucleic acid can be obtained together with the labeled amount value of the labeled abnormality detecting nucleic acid for quality inspection use. These labeled amount value can be supplied to the user as digital data, and by doing this manner, the user not only can carry out quality inspection but also can carry out a nucleic acid microarray test immediately without requiring preparation of a normal cell-derived labeled sample nucleic acid when an abnormal cell-derived sample nucleic acid alone is prepared. By this, sharp shortening of the operation steps becomes possible, and experimental cost is sharply reduced too, because using amount of nucleic acid microarrays is reduced and using amount of reagents is also reduced.

In addition, the quality inspection method of the embodiment can be used jointly with the sampling inspection. That is, for example, when the manufacturer of nucleic acid microarrays (the manufacturer includes a provider of nucleic acid microarrays) carries out sampling inspection of nucleic acid microarrays for certain numbers and produces and ships the passed batch together with labeled amount values of the labeled sample nucleic acid for quality inspection use, each user can carry out nucleic acid microarray tests by the method of the embodiment and also carry out the quality inspection.

(A case of containing a same type of probe)

For example, in the case of spotting two or more of the same probes, when all of them are determined as abnormal spots, all of them may be eliminated or these may be partially eliminated. However, when all of them are eliminated as abnormal spots, it becomes unable to obtain information regarding the probe. Then, it is preferable that, even if it is determined that all the spots are abnormal spots, at least one spot is not eliminated as an abnormal spot. In that case, it is preferable that the spot not being eliminated as an abnormal spot is included in a spot pair having the closest comparative value to the representative value among the comparative values of the same type of spot pairs. Also, when all the spots are eliminated, it is preferable to inform the users that all of the abnormal spots are eliminated. For example, the calculation of the embodiment can be automatically carried out by a software on a computer, and a summary of the abnormal spots can be displayed by the software on the screen as letters or images, and further, when all of the abnormal spots among them were eliminated or many spot pairs among spots prepared by spotting the same probe were eliminated as abnormal spots, it is preferable to display them by especially emphasizing them on the screen or on paper. Also, when many spot pairs among spots prepared by spotting the same probe were eliminated as abnormal spots or all of them were eliminated, it is preferable also to prepare in such a manner that threshold value of the spot pairs alone, for example threshold value for the Log ratio of the labeled amount values calculated from the calculate labeled abnormality detecting nucleic acid (labeled correction nucleic acid), can be changed. For example, it is preferable to prepare in such a manner that information can be obtained from spots by lowering threshold value for only the spot pairs spotted with a specific kind of probe nucleic acid, namely by reducing the number of abnormal spots.

Regarding combination of the spot pairs for carrying out comparison for detecting abnormal spots, most preferably, those in which the same probe nucleic acid is spotted and spotting positions inside the nucleic acid microarray are identical are desirable. Those which have the same spotting position are, for example, Column number and Row number having the same spot. However, according to the embodiment of the invention, it is not necessary to particularly stick to the spotting position with the proviso that the same probe nucleic acid is spotted. For example, when the same probe nucleic acid is spotted, this indicates for example that the spot pairs may be different Column numbers or different Row numbers. Also, when the same probe nucleic acid is spotted, hybridization conditions between two nucleic acid microarray may be different. Further, spotting may be carried out by changing concentration of the probe nucleic acid on purpose, within such a concentration range that it is not detected as an abnormal spot. The term "corresponds to the spot" as used in the embodiment means a combination of such spots.

In addition, it is not always necessary that the nucleic acid microarrays to be used have completely the same spot arrangement. That is, respective kinds of probe nucleic acids may be spotted by any arrangement as long as the same probe nucleic acid is spotted. That is, even in the case of nucleic acid microarrays having different arrangements, comparison can be carried out when the same probe nucleic acid pair is present.

Also, according to the embodiment of the invention, abnormal spots can be found out by carrying out the calculation on spots, but the calculation can also be carried out on an interested region or a group of spots. That is, mass production of a large number of nucleic acid microarrays is carried out by spotting predetermined probes in advance with the aim of improving the production efficiency, rather than producing separately designed nucleic acid microarrays individually. In such a case, there is a possibility that a probe the user does not require is contained and such a part becomes an object of detecting abnormal spot, so that it can be considered that many of abnormal spots are concentrated into such parts or, on the contrary, abnormal spots are concentrated into the regions of interest. Since such a case can be considered, it is not necessary in the embodiment to use all of the spots mounted on the nucleic acid arrays as the object of calculation, and a partial region can also be used as the object of calculation.

It is preferable that the labeled abnormality detecting nucleic acid to be used in the embodiment is of the same batch. This is because correction cannot be carried out correctly when concentration and labeling efficiency of the labeled abnormality detecting nucleic acid are different, for example in the case of carrying out the comparison between two nucleic acid microarrays. However, this is not necessary even when these concentration, labeling efficiency and the like physical property values of the labeled abnormality detecting nucleic acid (labeled correction nucleic acid) are different so long as contents of the differences are known.

According to the embodiment of the invention, representative values of the labeled sample nucleic acid and labeled abnormality detecting nucleic acid can be calculated and used in the subsequent treatment. For example, average values of their fluorescence values can be used. Also in the embodiment, it is able to use representative values of values in which labeled amount values of the labeled sample nucleic acid and labeled abnormality detecting nucleic acid (labeled correction nucleic acid) were numerically converted. For example, those in which fluorescence values of two labeled sample nucleic acids were divided and logarithmic values of the divided values, as well as average values of fluorescence values and the like of the labeled sample nucleic acid after correction with labeled correction nucleic acid, and the like can be used.

According to the embodiment of the invention, it is possible to use a monochromatic method (one color method) and it is also possible to use a two color method or a multicolor method of more than that.

For example, in the case of the monochromatic method, by allowing the labeled sample nucleic acid A1 and labeled abnormality detecting nucleic acid to hybridize with the nucleic acid microarray A and allowing the labeled sample nucleic acid B1 and labeled abnormality detecting nucleic acid to hybridize with the nucleic acid microarray B, it is possible to detect abnormal spots from labeled amount values of the two labeled abnormality detecting nucleic acids.

For example, in the case of the two color method, by allowing the labeled sample nucleic acid A1, labeled sample nucleic acid A2 and labeled abnormality detecting nucleic acid to hybridize with the nucleic acid microarray A and allowing the labeled sample nucleic acid B1, labeled sample nucleic acid B2 and labeled abnormality detecting nucleic acid to hybridize with the nucleic acid microarray B, it is possible to detect abnormal spots from labeled amount values of the two labeled abnormality detecting nucleic acids.

The case of a multicolor method of more than two colors can also be carried out in the same manner.

According to the embodiment of the invention, the detected abnormal spots can be eliminated or clearly indicated. The term elimination means that a step for eliminating abnormal spots-derived data is carried out during a period of from the time of detecting abnormal spots to a time of interpreting the data by the user, or that the abnormal spots-derived data are eliminated at least at the time when the user interprets the data. Even when data on the abnormal spots to be eliminated are internally present, it may be sufficient when data on the objects of elimination are not present on a graph in the case, for example of representing results of the nucleic acid microarray by the graph. Also, when the spots to be eliminated are handled on a computer, in general, this is handled in most cases by preserving as a variable, but the data may be eliminated from the variable or the spots to be eliminated may be handled by memorizing a eliminating position such as an index number or the like in other variable, namely by relying on the index number or the like without eliminating data from the variable.

The clear indication means an action to inform the user by a certain method that the data are derived from abnormal spots, and its means may be anything. For example, the presence of abnormal spots-derived data may be shown on the display connected to a computer or the screen reflected by a projector, or the presence of abnormal spots-derived data may be printed on a sheet of paper.

In addition, the abnormal spots and other spots (to be called normal spots) can be shown in the clear indication by different symbols, for example, the normal spots by open circle and the abnormal spots by closed circle. Also, the spots determined problematic by the quality inspection (to be referred sometimes to as abnormal spots in this case, too) can also be shown by the same symbol of abnormal spots, or can be shown by a symbol different from those of normal spots and abnormal spots, such as open triangle. In addition, by not indicating at least one or both of the abnormal spots and the spots determined problematic by the quality inspection, namely by indicating the normal spots alone, these can be distinguished from the normal spots, so that even such a case is regarded as clear indication in the embodiment.

The following describes details of the respective members, materials, treatments and the like to be used in this embodiment.

<Nucleic acid microarray>

The nucleic acid microarray to be called in the embodiment is the one in which a probe nucleic acid is bonded at a high density on a solid substrate. The solid material to be used in the substrate is not particularly limited with the proviso that it is a material to which the probe nucleic acid can be bonded, such as generally used slide glass and the like glass raw materials, as well as plastic materials. Shape of the substrate is not particularly limited, and it can be formed into a plane, the peak of a projection, a particulate, inside of a capillary and the like shapes. In addition, a shape in which a probe nucleic acid is sealed in the inside of a gelatinous substance can also be used.

Also, a DNA microarray produced by a spotter or the like and a DNA chip produced by a semiconductor technique are also included in the nucleic acid microarray.

In addition, even in the case of a multiple sample nucleic acid microarray in which two or more nucleic acid microarrays are arranged on one plate of substrate, each of them is called nucleic acid microarray. It is preferable that such an array is designed such that the hybridization solutions containing respective sample nucleic acids are not mixed when hybridization is carried out.

In this connection, a commercially available nucleic acid microarray may be used in the nucleic acid analyzing method of the embodiment.

<Probe nucleic acid>

The probe nucleic acid means a nucleic acid which is immobilized on the substrate of nucleic acid microarray. The probe nucleic acid of the embodiment comprises a probe sequence (a') complementary to the target sequence (a)and a sequence (b') which can bind to the sequence (b), and it is possible to hybridize a target nucleic acid comprising the target sequence (a) with a labeled abnormality detecting nucleic acid (B) comprising the sequence (b).

The probe nucleic acid and target nucleic acid (A) may be completely hybridized or partially hybridized. It is preferable that at least 50% or more the number of bases of the target nucleic acid are hybridized.

As the probe nucleic acid, a chemically synthesized nucleic acid, a cDNA, BAC (Bacterial Artificial Chromosomes), YAC (Yeast Artificial Chromosomes), PAC (P1-derived Artificial Chromosomes) and the like can be used. It is preferable that the probe nucleic acid is the BAI DNA or the cDNA. Further, a nucleic acid prepared by chemically modifying DNA, RNA or the like natural type nucleic acid or the like may be used as a probe nucleic acid. For example, a PNA (Peptide Nucleic Acid), a BNA (Bridged Nucleic Acid), a methyl phosphonate type DNA, a phosphorothioate type DNA, a boranophosphate type DNA and the like can be used. In addition, a chimeric type nucleic acid can also be used. For example, those in which a DNA moiety and an RNA moiety are mixed in a nucleic acid and a natural type nucleic acid and a modified type nucleic acid are mixed and the like can be used.

As the method for binding a probe nucleic acid onto a solid substrate, there may be used a method in which nucleotide is chemically synthesized one by one using an amidite monomer to which a photodesorption group typified by GeneChip (R) and using a mask, a method in which a desired sequence is chemically synthesized by spotting an amidite monomer on a solid substrate by an ink jet method, a method in which a nucleic acid is synthesized on a substrate by changing pH of a solution on an electrode and removing a protective group making use of the change in pH, a method in which a nucleic acid chemically synthesized in advance is purified and spotted on a substrate, a method in which a cDNA is spotted using a spotter, and the like. As the spotting method, an ink jet method, a pin array method and the like can be used.

<Immobilization of probe nucleic acid>

Immobilization of a probe nucleic acid on a nucleic acid microarray substrate can be carried out by chemically binding the probe nucleic acid onto a solid substrate. The binding includes covalent bond, ionic bond, electrostatic interaction, van der Waals binding, host-guest binding, metallic bond and the like. In addition, when a probe nucleic acid is bound onto a solid substrate, it may be bound via a linker.

<Spot pair>

The term spot air as used in the embodiment means a relationship between spots prepared by spotting the same type of probe nucleic acid. When applied to two or more nucleic acid arrays, it mainly means spots of the same type of probe, which are spots generally having the same block number, column number and row number. In this connection, it is not always necessary to have the same block number, column number and row number in this case as long as the same probe is spotted.

<complementary>

The term complementary as used in the embodiment means that the nucleic acid bases belonging to respective nucleic acids mutually undergo interaction via hydrogen bonds, and for example, it means that A (adenine) interacts with T (thymine) by two hydrogen bonds, and the like. Also, in addition to the complementation in which all of the base pairs of nucleic acids mutually undergo interaction, a case in which 50% or more of base pairs undergo interaction is also called complementary in the embodiment.

<Analyte>

The term analyte as used in the embodiment means a cell or the like which becomes the object of carrying out measurement of expressed amount and existing amount and the number of copies of a nucleic acid and the like.
As the analyte, for example, a normal cell or an abnormal cell can be used.
The normal cell means a cell which substantially did not undergo a mutation. Contrary to this, the abnormal cell means a cell having a gene in which the gene of a normal cell underwent a certain mutation.
For example, a cell obtained from a healthy person free from the onset of a cancer can be regarded as a normal cell, and a cell obtained from a patient developing a cancer as an abnormal cell. Also, even in the case of a patient who developed a cancer, a cell obtained from a normal region free from a cancer can also be regarded as a normal cell. In addition, a cell in which a gene in the cell was changed by adding a drug or the like reagent can be regarded as an abnormal cell, and a cell to which the drug was not added as a normal cell.
In addition, those to which an LCM (Laser Capture Microdissection) treatment was applied can also be used as an analyte.

<Sample nucleic acid>

This is a nucleic acid extracted from an analyte. For example, a nucleic acid extracted from a normal cell or a nucleic acid extracted from an abnormal cell (e.g., a cancer cell) can be used as the sample nucleic acid.

preparation of sample nucleic acid (purification and fragmentation and the like)>

When a sample nucleic acid is prepared from an analyte, it is necessary to remove protein, lipid and the like cell lysate by carrying out a purification treatment. This is because when purification is not carried out, various side reactions are generated during labeling with a fluorescent substance and in addition to this, the protein, lipid and the like cell lysate exert a great influence upon background noise so that performance of the nucleic acid microarray is considerably lowered.
Regarding the purification method, a method which uses a cartridge carrying silica, cellulose derivative or the like nucleic acid adsorbing film, ethanol precipitation or isopropanol precipitation, phenol-chloroform extraction and a method by a solid phase extraction cartridge, chromatography or the like which uses an ion exchange resin, a silica carrier to which octadecyl group or the like hydrophobic substituent group is bonded or a resin that shows a size exclusion effect can be used.

The purification treatment may be carried out after the following fragmentation treatment.

A fragmentation treatment may be carried out on a sample nucleic acid extracted from an analyte. As the fragmentation treatment, an enzyme treatment, an ultrasonic wave treatment, a mechanical fragmentation treatment, a chemical treatment and the like can be mentioned.

Treatment by a subtraction method may be carried out on a sample nucleic acid. The subtraction method is a method for efficiently isolating a gene when there is a difference in the number of copies or expression of the gene of interest, by subtracting a difference presenting in the gene by a knack of subtraction. As the subtraction method, for example, a PCR-Select method, an RDA (representational difference analysis) method, DsDD (Duplex-specific Direct Digestion) method and the like can be used.

By carrying out treatment by the subtraction method, accuracy of the nucleic acid microarray can also be improved. Particularly, when a cancer cell is used as the analyte, there is a case in which performance of the nucleic acid microarray is considerably lowered because a large amount of normal ells are contained in the cancer tissue together with the cancer cells, but the lowering of performance can be prevented to a certain degree by the subtraction method.

<Labeled sample nucleic acid>

The labeled sample nucleic acid means a sample nucleic acid in which a sample nucleic acid is labeled with a certain labeling compound. Those in which a purification treatment was carried out or not carried out after labeling are also called labeled sample nucleic acid in the embodiment.

<Unpurified labeled sample nucleic acid>

The unpurified labeled sample nucleic acid means a labeled sample nucleic acid obtained without carrying out a purification treatment after labeling of the sample nucleic acid. This purification treatment is a treatment for removing unreacted labeling compound, enzyme and the like after carrying out the labeling treatment, and a purification treatment for preparing the sample nucleic acid is not included therein.

Since purification is not carried out, various unreacted compounds added during the labeling step, such as a primer, an enzyme, a nucleotide, an ion contained in a buffer and the like, are contained in this unpurified labeled sample nucleic acid.

In addition, a nucleic acid after carrying out heating, EDTA addition and the like treatments of an unpurified labeled sample nucleic acid for the purpose of inactivating the coexisting enzyme is also called unpurified labeled sample nucleic acid.

<Purified labeled sample nucleic acid>

The purified labeled target nucleic acid means a sample nucleic acid obtained by carrying out a purification treatment after labeling of a sample nucleic acid on an unpurified labeled target nucleic acid.

<Abnormality detecting nucleic acid>

The abnormality detecting nucleic acid is a nucleic acid which has a sequence be able to be hybridized with at least a part of a probe nucleic acid in all the spots, which can be used to detect an abnormality in a hybridization ability in a spot in interest, specifically, to detect an abnormality caused by an amount of a probe nucleic acid immobilized to the spot in interest, and which further can be used to detect a spot amount of the probe nucleic acid between abnormal spots. Therefore, the abnormality detecting nucleic acid has any sequence and any chain length so long as it can be hybridized with any of the probe acids which are immobilized with all the spots on the nucleic acid microarray for which abnormality detection is carried out. Here, the expression "all the spots" means all of spots with which there is a possibility that an analyte of the same solution or a standard reacts. Normally, all the spots mean all spots on the nucleic acid microarray. However, meaning of the expression "all the spots" is not limited thereto.

As the abnormality detecting nucleic acid, a nucleic acid having a sequence which is considered to be present in all spots when an array is prepared, such as a repetition sequence, a vector or a genome of colibacilli.

Also, any nucleic acid may be used so long as it can be hybridized. A nucleic acid which is obtained by chemically modifying a nucleic acid of a native form such as DNA or RNA may be used. For example, PNA (Peptide Nucleic Acid), BNA (Bridged Nucleic Acid), a methyl-phosphonate type DNA, a phosphorothioate type DNA, a phosphoramidite type DNA, or a boranophosphate type DNA may be used. Also, for example, a chimera type nucleic acid. Examples of this type include one in which a DNA portion and an RNA portion are mixed or one in which the natural-type nucleic acid and a modified type nucleic acid are mixed.

Unlike the nucleic acid of the analyte, it is preferable that the abnormality detecting nucleic acid is a nucleic acid having a sequence on the probe nucleic acid. For example, it is more preferable that a nucleic acid which is a library itself being used as a probe or a nucleic acid having a seuqnce such as a vector or a cloning vector of the library which is used as a probe.

Examples of the vector include various vectors such as a plasmid, a BAC, a YAC, a PAC, a cosmid and a virus. Any nucleic acid may be used as an abnormality detecting nucleic acid so long as it is a nucleic acid of a vector which is used in preparation of an array or a nucleic acid having the same sequence as the vector.

A nucleic acid sequence of a library which is used in an immobilized probe or a normal cell-derived nucleic acid may be used as the abnormality detecting nucleic acid. It is preferable that the normal cell-derived nucleic acid has a sequence for which it has been known in advance that there is no abnormality in nucleic acid sequence. For example, when the sample nucleic acid includes a normal cell-derived one and an abnormal cell-derived one, the abnormal cell-derived labeled sample nucleic acid and the normal cell-derived labeled abnormality detecting nucleic acid are added to the first nucleic acid array (A1), and the normal cell-derived labeled sample nucleic acid and the normal cell-derived abnormality detecting nucleic acid are added to the second nucleic acid microarray. With the embodiment, abnormal spots can be detected from the labeled amount values of the normal cell-derived labeled abnormality detecting nucleic acid.

It is preferable that a repetition sequence is used as the abnormality detecting nucleic acid. The repetition sequence may be called a repeat sequence, and is a sequence which appears every certain number of bases repeatedly like a restriction site. Examples of the repetition sequence include a sequence in which a short base sequence pattern is repeated many times, such as poly d(AT) or poly d(GC), and a sequence in which a unit sequence being as long as several thousands of base sequences are repeated many times. It has been known that repetition sequences highly often appear in genome of higher organism such as human being. In the embodiment of the invention, Cot-1 DNA sold by Invitrogen Corporation may be used.

For example, in the case of a nucleic acid microarray using mammalian BAC-DNA, it is preferable that Cot-1 DNA is used. Cot-1 DNA is a nucleic acid which is generally used to black a repetition sequence of a sample which is obtained from mammalian at the time of measurement of the nucleic acid array. In the case of the nucleic acid microarray using a mammalian BAC probe, the porbe substantially includes many repetition sequences.

For example, in the case of the nucleic acid microarray using BAC-DNA having a repetition sequence in a probe, Cot-1 DNA is added, repetition sequences being present on the target nucleic acid and the probe nucleic acid are nonspecifically hybridized in advance and blocked. Thereby, it is prevented that a repetition sequence being present in the target nucleic acid is nonspecifically hybridized with the probe nucleic acid, and a fluorescence value derived from specific hybridization can be detected selectively. The inventors found that, by using the Cot-1 DNA with at least a part of the Cot-1 DNA being labeled (preferably, together with an unlabeled Cot-1 DNA), detection sensitivity for specific hybridization was enhanced, and a difference among spots can be reduced.

Also, synthetic oligonucleotide having a nucleic acid sequence which is substantially complementary to a nucleic acid sequence part which is commonly present in the probe nucleic acid may be used as the abnormality detecting nucleic acid.

In the embodiment of the invention, it is preferable that the abnormality detecting nucleic acid is used so that a molar ratio of the abnormality detecting nucleic acid to the sample nucleic acid is 1 or more. The molar ratio of 1 or more enhances an ability of the nucleic acid microarray.

Size of the abnormality detecting nucleic acid is preferably equal to more than 5 bp, more preferably equal to or more than 10 bp, further preferably equal to or more than 20 bp, and still further preferably equal to or more than 50bp. Also, the size of the abnormality detecting nucleic acid is preferably equal to or less than 1 Mbp, more preferably equal to or les than 500 kb, further preferably equal to or less than 2 kbp, and still further preferably equal to or less than 500 bp.

Using amount of the abnormality detecting nucleic acid is preferably 0.5 mol or more, more preferably 1 mol or more, further preferably 3 mol or more, still further preferably 8 mol or more, based on 1 mol of the target nucleic acid.

In this connection, upper limit of the using amount of abnormality detecting nucleic acid is preferably $10^{14}$ mol, more preferably $10^{10}$ mol, based on 1 mol of the target nucleic acid.

Concentration and the like physical property values of the abnormality detecting nucleic acids (B) may be different with the proviso that their concentration and the like physical property values are sufficiently known between the nucleic acid microarrays to be mutually used for comparison, but preferably, they may have the same concentration and originated fro the same batch.

<Labeled abnormality detecting nucleic acid (B)>

The labeled abnormality detecting nucleic acid is an abnormality detecting nucleic acid in which a labeling compound is linked to the abnormality detecting nucleic acid, and those in which a purification treatment was carried out or not carried out after labeling are also called labeled abnormality detecting nucleic acid in the embodiment.

<Unpurified labeled abnormality detecting nucleic acid and purified labeled abnormality detecting nucleic acid>

In the same manner as in the unpurified labeled sample nucleic acid and purified labeled sample nucleic acid, the unpurified labeled abnormality detecting nucleic acid is a labeled abnormality detecting nucleic acid in which a purification treatment was not applied to the labeled abnormality detecting nucleic acid and the purified labeled abnormality detecting nucleic acid is a labeled abnormality detecting nucleic acid obtained by applying a purification treatment to the labeled abnormality detecting nucleic acid.

<Unlabeled nucleic acid>

The unlabeled nucleic acid means a nucleic acid which is not labeled.
An unlabeled nucleic acid may be added to the hybridization solution which contains various nucleic acids.
As the unlabeled nucleic acid, for example, Cot-1 DNA for blocking repeating sequence and tRNA (transfer RNA), modified salmon sperm DNA, poly A, poly dA and the like, which mainly have the actin to lower background noise of nucleic acid microarray, and the like can be used. According to the embodiment of the invention, the Cot-1 DNA can be used suitably.

<Labeling>

The labeling means that a detectable substance is linked to the target nucleic acid. As the detectable substance, it is not particularly limited, but for example, a fluorescent material, an enzyme, a radioisotope, a compound which generates FRET (fluorescent resonance energy transfer), and the like can be mentioned. Among them, it is particularly desirable to use a fluorescent material.
As the fluorescent material, though not particularly limited, fluorescein isothiocyanate (FITC), Cy 3, Cy 5, Cy 7, green fluorescent protein (GFP), blue fluorescent protein (BFP), yellow fluorescent protein (YFP), red fluorescent protein (RFP), Alexa, Acridine, DAPI, ethidium bromide, SYBR Green, Texas Red, a rare earth fluorescence labeling agent (4,4'-bis(1",1",1",2",2",3",3"-heptafluoro-4",6"-hexanedion-6"-y1)-chlorosulfo-o-terphenyl (BHHCT), ethidium bromide, Acridine Orange, TAMRA, ROX and the like can be used.
In addition, digoxigenin (DIG), biotin and the like can also be used as a substance for labeling use. As an example of the use of biotin, when avidin is bonded to biotin linked to a probe, a biotin-linked alkaline phosphatase is bonded thereto and then nitroblue-tetrazolium and 5-bromo-4-chloro-3-indolyl phosphate are added thereto as substrates of the alkaline phosphatase, development of purple color can be observed, which can be used for the detection.
In addition, a non-enzymatic label can also be used. For example, there are ULS arrayCGH Labeling Kit (Kreatech Biotecgnology BV) and the like.
Regarding the labeling method, both labeling methods of direct labeling method and indirect labeling method may be used in the embodiment. The direct labeling method is a method in which, for example, the target nucleic acid is converted into single chain and hybridized with a short chain nucleic acid, a nucleotide derivative to which a fluorescent pigment is bonded is mixed in advance with nucleotides, and a labeled target nucleic acid is synthesized by one step. The indirect is a method in which the target nucleic acid is converted into single chain and hybridized with a short chain nucleic acid, a nucleotide derivative having a substituent group to which a fluorescent pigment can be bonded, such as a nucleotide derivative having aminoallyl group, is mixed in advance with natural type nucleotides, thereby synthesizing this substituent group-possessing target nucleic acid, and then the labeled target nucleic acid is obtained by a labeling compound via the aminoallyl group. According to the embodiment of the invention, it is preferable to use the direct labeling method because of its convenient procedure.
According to the embodiment of the invention, random primer method (primer extension method), nick translation method, PCR (polymerase chain reaction) method, terminal labeling method and the like can be used as the method for introducing a labeling compound into the target nucleic acid. Particularly, the random prier method can be suitably used in the embodiment.
In this connection, since an enzyme and the like are remained as such in the solution containing unpurified labeled sample nucleic acid and unpurified labeled abnormality detecting nucleic acid, it is preferable to deactivate activity of the enzyme remaining in the solution. This is because, for example when an unpurified labeled sample nucleic acid is labeled with Cy 3, and an unpurified labeled abnormality detecting nucleic acid with Cy 5, and both of them are mixed, and when an enzyme is remained in the solution, there is a possibility that the unpurified labeled sample nucleic acid is further labeled with Cy 5, and the unpurified labeled abnormality detecting nucleic acid with Cy 3, by the remaining enzyme.
As the enzyme deactivation method, it may be any method with the proviso that it is a method which can deactivate the enzyme, but it is preferable to carry out either one or both of a method in which EDTA is added and a method in which a heating treatment is carried out at 50°C or more. The heating temperature is preferably 50°C or more, further preferably 60°C or more. The heating time may be sufficient when it is 1 minute or more, but most preferably, it is preferable to carry out the heating treatment at 65°C or more for 5 minutes or more.

<Labeled amount value>

The term labeled amount value as used in the embodiment means a labeled value generated from a labeling compound, read out when a labeled nucleic acid is hybridized with a probe nucleic acid. More illustratively, for example, there may be mentioned a fluorescent value read out using a fluorescence scanner or the like, after carrying out hybridization of a labeled target nucleic acid and a labeled abnormality detecting nucleic acid (labeled correction nucleic acid) with the probe nucleic acid linked onto the nucleic acid microarray, subsequently removing mainly the labeled target nucleic acid, labeled abnormality detecting nucleic acid (labeled correction nucleic acid) and the like which hybridized only partially, for eliminating the unreacted labeled target nucleic acid and labeled correction nucleic acid under a predetermined washing condition, and then drying the nucleic acid microarrays.

In addition, regarding the labeled amount value, a numerical value after carrying out a mathematical treatment can also be used as a labeled amount value. For example, in the case of a fluorescence scanner GenePix (R) and the like, by dividing one spot into many regions and reading out data for every 10 μm square, their representative value such as average value, median value and the like is output. Like in this case, the labeled amount value read out using a read out device is generally a value after carrying out a mathematical treatment on the labeled amount values from many picture elements, and such a case is also regarded as a labeled amount value.

<Hybridization preparation solution>

The term hybridization preparation solution as used in the embodiment means a solution which has a role of suitably carrying out hybridization. It is preferable that a buffer solution is contained in the hybridization preparation solution, though not always necessary. In the case of a nucleic acid microarray for example, a buffer mixture consisting of dextran sulfate having an action to increase nucleic acid concentration, formamide having an action to lower melting temperature of nucleic acid and a buffer solution for constantly keeping pH of the solution can be mentioned as an example of the hybridization preparation solution.

In addition, it is preferable to use a solution consisting of a combination of at least two species among a compound having excluded volume, a compound which lowers melting temperature and a compound that keeps the solution at a constant pH, as a composition of the hybridization preparation solution.

As the substance having an excluded volume effect, polyethylene glycol (PEG, JP-A-2000-325099), dextran sulfate and the like can be used.

As the compound which lowers melting temperature, formamide, glycerol, formaldehyde, DMSO, DMF, GuSCN, iodine and the like can be used.

As the buffer solution, any buffer solution can be used with the proviso that it can keep pH at a constant level, but it is preferable to use those which are generally used for biochemistry. For example, it is preferable to use SSC or a good buffer solution. As the good buffer solution, Bis-Tris (N,N-bis(2-hydroxyethyl)iminotris-(hydroxymethyl)methane), MES (2-morpholinoethanesulfonic acid), HEPES (2-[4-(2-hydroxyethyl)-1-piperazinyl]ethanesulfonic acid), PIPES (piperazine-1,4-bis(2-ethanesulfonic acid)), ACES (N-(2-acetamino)-2-aminoethanesulfonic acid), CAPS (N-cyclohexyl-3-aminopropanesulfonic acid), TES (N-tris(hydroxymethyl) methyl-2-aminoethanesulfonic acid) and the like can be mentioned.

<Hybridization solution>

The term hybridization solution as used in the embodiment means a solution mixed with an unlabeled nucleic acid and other reagents, means a solution having a property to idealize hybridization of a labeled target nucleic acid and a labeled abnormality detecting nucleic acid with the probe nucleic acid on a nucleic acid microarray, and means a solution which has actions to accelerate a hybridization having high conformity of sequences of the probe nucleic acid with the labeled target nucleic acid and labeled abnormality detecting nucleic acid and, on the other hand, to inhibit a hybridization having low conformity of these sequences. For example, a hybridization solution of 6 x SSC, 5 x Denhardt solution, 0.2% SDS, human Cot-1 DNA, poly A and yeast tRNA is used as a general composition of hybridization solution for nucleic acid microarray use (M. Muramatsu and H. Nawa, Cell Engineering, Supplement, Genomic Science Series 1, DNA Microarray and Recent PCR Method (all written in Japanese)). In addition, for example, a hybridization solution containing formamide, dextran sulfate, 20 x SSC, Cot-1 DNA, yeast tRNA and 20% SDS is used in the case of nucleic acid microarray(I. Inoue and J. Inasawa, Cell Engineering, Vol. 123, p. 355, 2004).

In addition, a compound which improves noise and the like of nucleic acid microarray can also be contained.

As the compound having such an action, modified sermon sperm DNA, poly(A), poly(dA), tRNA, human Cot-1 DNA, mouse Cot-1 DNA and the like can be used.

In addition to these, as described in JP-A-2005-087109, phospholipid, Denhardt solution (a solution which contains Ficoll, polyvinyl pyrrolidone and bovine serum albumin as the main components), betaine as a quaternary ammonium salt (Biochemistry, 32, 137 - 144 (1997)), TMAC (tetramethyl ammonium chloride) (Proc. Natl. Acad. Sci. USA, 82, 1585 - 1588 (1985)), a commercially available hybridization solution such as ExpressHyb (mfd. by Clontech), PerfectHyb (mfd. by TOYOBO), ULTRAhyb (mfd. by Amicon), and the like can also be used in the embodiment as one of the compositions for hybridization solution.

As the surfactant, a cationic surfactant, an anionic surfactant and a nonionic surfactant can also be used. As the anionic surfactant, it is preferable to use sodium dodecyl sulfate. In addition to this, N-lauryl sarcoside, lithium lauryl sulfate and the like are used. As the nonionic surfactant, Tween (registered trademark), Triton X (registered trademark) and the like are known, and these surfactants can be used in the embodiment.

<Concentration of hybridization solution>

It is preferable that a hybridization solution which does not contain a labeled target nucleic acid but contains other nucleic acids is concentrated in advance. That is, this is because a labeled abnormality detecting nucleic acid, an unlabeled nucleic acid, a surfactant and the like are added to such a hybridization solution containing various nucleic acids, and further, the hybridization solution after mixing with a labeled target nucleic acid and the like has a possibility of resulting in the lowering of performance of nucleic acid microarray because of the lowered concentration of the labeled target nucleic acid, labeled abnormality detecting nucleic acid (labeled correction nucleic acid), unlabeled nucleic acid and the like due to increase of the solution volume. Thus, according to the embodiment of the invention, it is preferable to carry out concentration of the hybridization solution containing various nucleic acids before mixing with nucleic acids including the labeled target nucleic acid. This concentration operation may be carried out by the user, but it is preferable that the solution prepared in advance by the manufacture by carrying out its concentration is appended to a kit or the like. That is, this is because when it is prepared in advance by the manufacturer, the user does not necessary to carry out its concentration and can use it in the nucleic acid microarray only by mixing with this hybridization solution as long as the labeled target nucleic acid is prepared in advance. In addition, this is when a labeled abnormality detecting nucleic acid is contained in this hybridization solution, it is preferable to unify concentration, kind, batch number and the like of the labeled abnormality detecting nucleic acid (labeled correction nucleic acid) and the user can easily carry out a nucleic acid microarray test having high accuracy when the manufacturer provides the solution having good order of these factors.

<Purification>

The term purification as used in the embodiment is used as the same meaning of extraction, separation and fractionation. Also, as the means for this, a method which uses a cartridge carrying silica, cellulose derivative or the like nucleic acid adsorbing film, ethanol precipitation or isopropanol precipitation, phenolchloroform extraction and a method by a solid phase extraction cartridge, chromatography or the like which uses an ion exchange resin, a silica carrier to which octadecyl group or the like hydrophobic substituent group is bonded or a resin that shows a size exclusion effect can be included. In addition, solvent exchange is also called purification by a broad meaning in the embodiment. Because of this, ethanol precipitation and is opropanol precipitation are also called purification in the embodiment.

<Monochromatic method>

The monochromatic method according to the embodiment of the invention is a method called by another name one color method, which is a method in which substantially one kind of a labeled target nucleic acid labeled with one kind of a labeling compound and substantially one kind of a labeled abnormality detecting nucleic acid (labeled correction nucleic acid) labeled with a labeling compound different from the labeled target nucleic acid are used in one nucleic acid microarray, substantially one kind of another labeled target nucleic acid labeled with one kind of a labeling compound and substantially one kind of a labeled abnormality detecting nucleic acid (labeled correction nucleic acid) labeled with a labeling compound different from the labeled target nucleic acid are used in the other nucleic acid microarray, and labeled amount value of the labeled target nucleic acid obtained from the first nucleic acid microarray is compared with labeled amount value of the labeled target nucleic acid obtained from the second nucleic acid microarray. In addition, a method in which labeled amount values obtained by correcting the labeled amount values of labeled target nucleic acid using the labeled amount values of labeled correction nucleic acid, obtained from respective nucleic acid microarrays, are compared and examined is also called monochromatic method.

In this manner, according to the embodiment of the invention, a method in which the number of labeled compound for the samples mutually used as the object of comparison on one nucleic acid microarray is one is called monochromatic method. That is, two kinds of labeling compounds, labeled target nucleic acid and labeled correction nucleic acid, are used in the case, but since the substance directly according to the comparison is the labeled target nucleic acid alone, such a case is called monochromatic method in the embodiment even when the two kinds of labeling compounds, labeled target nucleic acid and labeled correction nucleic acid, are used.

<Two color method>

The two color method according to the embodiment of the invention is a method in which substantially one kind of a labeled target nucleic acid labeled with one kind of a labeling compound, a labeled target nucleic acid prepared from another target nucleic acid different from that of the substantially one kind, labeled with a different labeling compound, and substantially one kind of a labeled abnormality detecting nucleic acid labeled with a labeling compound different from the two kinds of labeled target nucleic acids are used in one nucleic acid microarray, and a result is obtained comparing the labeled target nucleic acids, and in carrying out this, elimination of abnormal spots can be carried out using labeled abnormality detecting nucleic acids (labeled correction nucleic acids, That is, by preparing another nucleic acid microarray, allowing at least the labeled abnormality detecting nucleic acid to hybridize therewith and comparing labeled amount value of this labeled abnormality detecting nucleic acid with labeled amount value of the labeled abnormality detecting nucleic acid obtained from the nucleic acid microarray, abnormal spots can be detected and the spots which correspond to the abnormal spots can be eliminated from the nucleic acid array or clearly indicated.

<Method for reading out labeled amount value>

Regarding the method for reading out labeled amount value using a nucleic acid microarray, various methods can be used. When a fluorescent material is used as the labeling compound, a fluorescence scanner is used most suitably. As the fluorescence scanner, there may be mentioned, for example, FLA-8000 (mf. by Fuji Photo Film), GenePix (R) 4000B (mfd. by Axon and the like.

<Provision of data>

According to the embodiment of the invention, it is able also to allow a labeled target nucleic acid and a labeled abnormality detecting nucleic acid (labeled correction nucleic acid) to hybridize in advance with a nucleic acid microarray and provide their labeled amount values as electronic data. For example, in the case of a nucleic acid microarray, it is possible to provide labeled amount value of a normal cell-derived labeled target nucleic acid as the target nucleic acid, and labeled amount value of labeled Cot-1 DNA or labeled vector-derived nucleic acid as the abnormality detecting nucleic acid, through electronic data.

By making use of these data, the user may prepare only a cell to be compared such as an abnormal cell without preparing comparing cell, and further regarding the labeling step and the like steps, may carry out only one kind of labeling step while it was necessary to carry out two labeling steps in the past, so that the steps are shortened and saving of the time and reduction of the amount of reagents to be used can be carried out. In this connection, it is preferable to use a labeled abnormality detecting nucleic acid which is common to both cases. In addition, it is preferable to pack electronic data on a labeled abnormality detecting nucleic acid and the labeled abnormality detecting nucleic acid in one kit.

(Program)

In treating the data obtained by carrying out a nucleic acid microarray measurement, it is possible to carry out a data processing using a program in order to carry out the data processing efficiently. This is particularly effective when the nucleic acid microarray has a large number of spots and a large number of comparative values ($D_B$) are calculated.

That is, the program of the embodiment of the invention may carry out

calculating each comparative value ($D_Bi$) between a labeled amount value (Fc1i) and a labeled amount value (Fc2i) for a spot pair including a spot (X1i) and a spot (X2i),

obtaining a representative value (A) based on the n comparative values ($D_Bl$) to ($D_Bn$), and

determining, based on the magnitude of the difference between the comparative value ($D_Bm$) and the representative value (A), as to whether or not the spot (X1m) is an abnormal spot.

The program is provided generally in the form of being recorded on a recording medium which can be read out by a computer. The recording medium is not particularly limited with the proviso that a computer can read it out. The recording medium of the invention includes both of the portable type and standing type media, and for example, a compact disk (CD), a digital video disk (DVD), a hard disk, a semiconductor memory and the like can be mentioned.

The program of the embodiment may be circulated by being recorded on the recording medium and also can be circulated in the form of being recorded on a recording device of a computer in advance and transferring it to other computer through a net work.

(Nucleic acid analyzing kit)

A nucleic acid analyzing kit of the embodiment comprises a nucleic acid microarray having plural spots to which a probe nucleic acid is immobilized and an abnormality detecting nucleic acid containing a sequence (b) that can bind to a sequence (b') contained in the probe nucleic acid immobilized on the nucleic acid microarray. The abnormality detecting nucleic acid may be labeled in advance, or each user may carry out the labeling at the time of its use. In addition, it is also desirable to provide said abnormality detecting nucleic acid as a hybridization solution containing an ionic strength-adjusting component, a surfactant and the like.

[Examples]

the embodiment of the invention will be described further in detail based on examples and comparative examples. It should be noted that the invention is not limited to the following examples.

In this connection, in the following examples, the Cy3-Female means a result of labeling Female DNA with Cy 3. In the same manner, the Cy5-Female means a result of labeling Female DNA (or male) with Cy 5. In addition, the Cy3-Male and Cy5-Cot-1 DNA also indicate in the same manner that these are nucleic acids labeled with respective labeling compounds.

[Comparative Example 1] Results of analysis of nucleic acid microarray in the case of not eliminating abnormal spots

<Preparation of unpurified labeled sample nucleic acid>

A 3 μl (0.75 μg) portion of Human Genomic DNA Female (mfd. by Promega, catalog number G152), 8 μl of water (Distilled Water, DNase, RNase Free, mfd. by GIBCO) and 20 μl of 2.5 x Random Primer Solution (mfd. by Invitrogen) of a labeling system for array CGH use (BioPrime (R) Array CGH Genomic Labeling System (mfd. by Invitrogen)) were put into a 1.7 ml capacity microtube (platinum tube, mfd. by B. M. Equipments) and heating treatment was carried out at 95°C for 5 minutes in a block incubator (BI-535A, mfd. by ASTEC). Five minutes thereafter, the microtube was taken out from the block incubator, put into a block incubator set to 37°C and allowed to stand still for 15 minutes.

By adding thereto 5 μl of 10 x dCTP Nucleotide Mix of BioPrime (R) Array CGH Genomic Labeling System (mfd. by Invitrogen), 3 μl of 250 nmol Cy3-dCTP Bulk Pack (mfd. by GE Healthcare Bioscience) and 1 μl of Exo-Klenow Fragment of BioPrime (R) Array CGH Genomic Labeling System (mfd. by Invitrogen), labeling reaction and amplification reaction were simultaneously carried out at 37°C for 2 hours on the BLOCK INCUBATOR BI-535A. After 2 hours thereof, the microtube was taken out from the incubator and the Exo-Klenow Fragment contained in the reaction solution was deactivated by carrying out a heating treatment on the BLOCK INCUBATOR BI-535A set to 65°C.

The operation was carried out also on Hyman Genomic DNA Male (mfd. by Promega, catalog number G147) and 2 types of unpurified labeled sample nucleic acids were obtained by the above operations. In this connection, Cy3-dCTP (mfd. by GE Healthcare Bioscience) was also used as a labeling compound on the Male DNA.

<Preparation of unpurified labeled abnormality detecting nucleic acid>

A 6 μg (6 μl) portion of Cot-1 DNA Female (mfd. by Invitrogen, catalog number 15279-011), 5 μl of water and 20 μl of 2.5 x Random Primer Solution were put into a 1.7 ml capacity microtube (platinum tube, mfd. by B. M. Equipments) and heating treatment was carried out at 95°C for 5 minutes on Block Incubator BI-535A. Five minutes thereafter, the microtube was taken out, put into Block Incubator BI-535A set to 37°C and allowed to stand still for 15 minutes. By adding thereto 5 μl of 10 x dCTP Nucleotide Mix, 3 μl of 250 nmol Cy5-dCTP Bulk Pack (mfd. by GE Healthcare Bioscience) and 1 μl of Exo-Klenow Fragment, labeling reaction and amplification reaction were simultaneously carried out at 37°C for 2 hours on the BLOCK INCUBATOR BI-535A. Two hours thereafter, the microtube was taken out from the incubator and the Exo-Klenow Fragment contained in the reaction solution was deactivated by carrying out 15 minutes of a heating treatment on the BLOCK INCUBATOR BI-535A set to 65°C.

<Preparation of hybridization solution containing a labeled abnormality detecting nucleic acid Cot-1 DNA>

A 20 μl portion of an unpurified labeled abnormality detecting (correcting) nucleic acid, Cy5-Cot-1 DNA and 62 μl (62 μg) of an unlabeled nucleic acid, Cot-1 DNA (mfd. by Invitrogen), were put into a 1.7 ml capacity microtube, and 8 μl of 3 M sodium acetate (pH 5.2) and 360 μl of ethanol cooled at -20°C were added thereto and mixed and then allowed to stand still at -80°C for 10 minutes. Thereafter, centrifugation was carried out at 15,000 rpm and at 4°C for 30 minutes using a centrifuge MX-300 manufactured by TOMY SEIKO. After the centrifugation, since precipitate goes to the bottom of the centrifugation tube, the supernatant was discarded while taking care of not sucking the precipitate. Next, the centrifugation tube was allowed to stand still for 10 minutes while opening its cap, and the remaining ethanol was removed. Ten minutes thereafter, 52 μl of a hybridization preparation solution consisting of 40% formamide, SSC and 0.075 mg/μl of dextran sulfate and 8 μl of 10% SDS were added thereto and allowed to stand still for 30 minutes. After 30 minutes thereof, the precipitate was dissolved by carrying out its stirring and then thoroughly stirred.

<Preparation of hybridization solution containing labeled sample nucleic acid and labeled abnormality detecting nucleic acid>

A 20 μl portion of the unpurified labeled sample nucleic acid of Female DNA prepared in <Preparation of unpurified labeled sample nucleic acid> and 60 μl of the solution prepared in <Preparation of hybridization solution containing a labeled abnormality detecting (correcting) nucleic acid Cot-1 DNA> were put into a 1.7 ml capacity microtube and stirring was carried out by thoroughly carrying out a Vortex treatment. Also in the same manner, 20 μl of the unpurified labeled sample nucleic acid of Male DNA prepared in <Preparation of unpurified labeled sample nucleic acid> and 60 μl of the solution prepared in <Preparation of hybridization solution containing a labeled abnormality detecting nucleic acid Cot-1 DNA> were put into a 1.7 ml capacity microtube and stirring was carried out by thoroughly carrying out a Vortex treatment.

<Pretreatment of nucleic acid microarray>

The nucleic acid microarray used herein has a total of 1,600 spots prepared by spotting two spots for each of 800 probe DNA species prepared from a BAC clone (RPCI, RP11 BAC Library) on a glass substrate. The following describes the nucleic acid microarray preparation method. Firstly, BAC DNA was obtained by mass-culturing the BAC clone and then extracting and purifying it using an ion exchange column (Plasmid Midi Kit 100, Cat. No. 12145, mfd. by QIAGEN). Thereafter, the BAC DNA was digested with four base-recognizing enzymes *Rsa*I*, Dpn*I and *Hae*III and subjected to ligation by adding an adapter. Next, a probe DNA was obtained by amplifying it by a PCR method using a primer having the same sequence of one oligonucleotide of the adapter. Length of the probe DNA was approximately from 1,000 to 3,000 bp. GENESHOT (NGK Insulators, Nagoya) was used for the spotting of probe DNA on a glass substrate.

About 200 ml of a blocking solution (mfd. by Matsunami Glass) was put into a glass container, and a nucleic acid microarray was put into the same to carry out 20 minutes of blocking reaction using SLIDE WASHER, SW-4 (mfd. by JUJI FIELD), while shifting the slide glass up and down. After 20 minutes thereof, the nucleic acid microarray was taken out from the blocking solution and put into a container charged with 200 ml of water. This was washed for 3 minutes using SLIDE WASHER and, 3 minutes thereafter, again put into a container charged with 200 ml of water and washed using SLIDE WASHER. Three minutes thereafter, this was transferred into a container charged with 200 ml of ethanol and again washed using SLIDE WASHER. Three minutes thereafter, the nucleic acid microarray was taken out, and the nucleic acid microarray was dried by carrying out its centrifugation using a table top centrifuge Spin Dryer mini 2350 (mfd. by TOMY SEIKO).

After the blocking, the nucleic acid microarray was soaked in boiling water for 2 minutes and subsequently soaked in 70% ethanol of -20°C for 2 minutes, 85% ethanol of -20°C for 2 minutes and 100% ethanol of -20°C for 2 minutes, and then the nucleic acid microarray was dried by carrying out 1 minute of centrifugation by the Spin Dryer. In this connection, the operation was carried out by simultaneously treating two nucleic acid microarrays.

<Hybridization]

The two sample solutions prepared in <Preparation of hybridization solution containing labeled sample nucleic acid and labeled abnormality detecting nucleic acid> were respectively subjected to 16 minutes of heating treatment at 75°C on the BLOCK INCUBATOR BI-535A. Thereafter, the heat treatment was carried out at 42°C for 30 minutes or more. Hybridization of the respective solutions was carried out using a hybridizer.

As the hybridizer, HYBRIMASTER HS-300 (mfd. by Aloka) was used. This was carried out at a hybridization temperature of 37°C for a hybridization time of 16 hours. During the hybridization, it was set to carry out the stirring with a roller. The hybridization solutions containing Cy3-Female DNA and Cy5-Cot-1 DNA were added dropwise to respective nucleic acid microarrays and the start button of hybridizer was pressed. The washing conditions were set to (1) 5 ml of 2 x SSC solution at room temperature for 30 seconds, (2) 5 ml of 50% formamide/2 x SSC (pH 7.0) solution at 50°C for 15 minutes, (3) 5 ml of 2 x SSC/0.1% SDS (pH 7.0) solution at 50°C for 30 minutes and (4) 5 ml of 2 x SSC solution at room temperature for 5 minutes, and it was set to carry out the stirring using a roller during the washing. After carrying out hybridization by the hybridizer, the microarray was taken out from the hybridizer, the microarray was put into a 50 ml capacity SUMILON tube charged with 2 x SSC, and several minutes thereafter, the microarray was taken out and the nucleic acid microarray was dried by carrying out 1 minute of centrifugation by the Spin Dryer-Mini Model 2350.

<Data uptake>

The thus washed two microarrays were subjected to scanning using GenePix (R) 4000B (mfd. by Axon Instruments).

<Data processing>

Data processing was carried out using the following formula.

By regarding the unpurified labeled sample nucleic acid Cy3-Female-derived fluorescence value as FF, and the unpurified labeled sample nucleic acid Cy3-Male-derived fluorescence value as FM, fluorescence value of the Female side labeled abnormality detecting (correcting) nucleic acid as Fc1 and the Male side labeled abnormality detecting (correcting) nucleic acid as Fc2, the labeled amount value FM' of Cy3-Male DNA after correction was calculated by the following formula.

$$FM' = FM \times Fc1/Fc2$$

In addition, Log Ratio(Female/Male) was calculated by the fooling formula.

$$\text{Log Ratio(Female/Male)} = \text{Log}_2(FF/FM')$$

In this connection, regarding the spot pair to be compared between arrays, Log Ratio(Female/Male) was calculated on spots arranged on the nucleic acid microarray using the same Block Number, the same Column Number and the same Row Number.

In addition, the value of Log Ratio(Female/Male) {the Ratio(Female/Male) is ratio of fluorescence value of the Female side spot to fluorescence value of the Male side spot} was also calculated using the following formula.

$$\text{Ratio(Female/Male)} = FF/FM'$$

Next, average value of the Log Ratio or Ratio(Female/ Male) of two spots prepared by spotting the same type of probe nucleic acid was calculated.

In this connection, global normalization was carried out as a normalization method.

<Results>

A graph prepared by plotting Log Ratio values in order of chromosome numbers is shown in Fig. 4, and that of Female/Male values in Fig. 5. In this connection, the plots in Figs. 4 and 5 are average values of two spots prepared by spotting the same type of probe. The open circle shows a part which corresponds to autosome, and the closed circle a part that corresponds to X-chromosome (the same shall apply subsequent drawings).

[Example 1] Results of analysis of nucleic acid microarray in the case of eliminating abnormal spots

On the results of Comparative Example 1, abnormal spots were detected through numerical calculation by the following method.

By regarding the Cy5-Cot-1 DNA-derived fluorescence value obtained from the Cy3-Female-hybridized nucleic acid microarray as Fc1, and the Cy5-Cot-1 DNA-derived fluorescence value obtained from the Cy3-Male-hybridized nucleic acid microarray as Fc2, their Log Ratio {Log Ratio(Cot-1/Cot-1)) was calculated as comparative value ($D_B$) by the following formula.

$$\text{Log Ratio(Cot-1/Cot-1)} = \text{Log}_2(Fc2/Fc1)$$

In this connection, the comparative value ($D_B$) was calculated between spots having the same Block Number, the same Column Number and the same Row Number of the Cy3-Female-hybridized nucleic acid microarray and the y3-Male-hybridized nucleic acid microarray, and the comparative value ($D_B$) was calculated on all of the spots spotted on the nucleic acid microarrays.

Next, average value was calculated as intermediate value (M) of the comparative value ($D_B$) calculated in the above, and all of the comparative values ($D_B$) were corrected so that the intermediate value becomes zero.

Next, After calculating the difference between intermediate value (N) and comparative value ($D_B$), absolute value ABS{Log Ratio(Cot-1/Cot-1)} was calculated for the calculated value on all spots. Since the intermediate value was corrected to zero, larger numerical value of this value shows larger difference from the intermediate value, so that in order of this difference, 3%, 5% and 10% of the number of spot pairs, based on the number of the total spot pairs namely 1,600 pairs in the case of this Example, that is, 48, 80 and 160 spot pairs, were respectively determined as abnormal spots. Also, all of the spot pairs showing this value of exceeding 0.6 were regarded as abnormal spots.

After detection of the abnormal spots, the values of Log Ratio(Female/Male) calculated in Comparative Example 1, which correspond to the abnormal spots, were eliminated.

In this connection, when both of the two spots spotted with the same type of probe nucleic acid were detected as abnormal spots in eliminating the abnormal spots, not eliminating both of them, the value of Log Ratio(Female/Male) having large value of ABS{Log Ratio(Cot-1/Cot-1)} was eliminated and the small value of Log Ratio(Female/Male) was not eliminated.

For example, when the number of eliminated abnormal spots was 10% of the total spots, there were some cases in which both of the two spots spotted with the same probe nucleic acid were detected as abnormal spots, but in such case, the spot far distant from the comparative value ($D_B$) among these two spots was determined as abnormal spot.

More illustratively, both of the $110^{th}$ and $111^{th}$ spot pairs spotted with the same probe nucleic acid were detected as abnormal spots. Although the both are regarded naturally as abnormal spots because the comparative value ($D_B$) of the $110^{th}$ was 1.45 while that of the $111^{th}$was 0.413, only the $110^{th}$ spot having the comparative value ($D_B$) far distant from the intermediate value was eliminated. Since the Log Ratio(Female/Male) value of the $110^{th}$ was -0.467 while that of the $111^{th}$ was -0.191, and these values more close to zero are the right answer, this result shows the effectiveness of saving the one having a comparative value ($D_B$) closer to the intermediate value.

Next, in the same manner as in Comparative Example 1, average value of Log Ratio(Female/Male) of two spots spotted with the same type of probe nucleic acid was calculated.

<Results>

Fig. 6 shows a case of plotting in which, of the whole 1,600 spot pairs, the sot pairs having comparative values that correspond to the higher-rank 3% (48 spot pairs) having larger difference between comparative value and intermediate value were eliminated as abnormal spots.

Fig. 7 shows a case of plotting in which, in the same manner, the sot pairs having comparative values that correspond to the higher-rank 5% (80 spot pairs) in order of the difference were eliminated as abnormal spots.

Fig. 8 shows a case of plotting in which, in the same manner, the sot pairs having comparative values that correspond to the higher-rank 10% (160 spot pairs) in order of the difference were eliminated as abnormal spots.

In comparing Figs. 6 to 8, though spots having a Log Ratio of outside the range of $\pm$ 0.25 were confirmed in a large number in Fig. 4, these are reduced to several spots in Fig. 6 to 8.

Also, it can be seen that variation in the autosomal moiety (theoretically, all of the spots concentrate on the axis X) is reduced by the elimination of abnormal spots.

In addition, in Figs. 7 and 8 in which 5% and 10% of the abnormal spots were eliminated, separation of the autosome and X-chromosomal moiety became good. That is, while comparative values in Fig. 4 where abnormal spots were not eliminated, in which the open circle parts of Log Ratio of autosomal moiety and the closed circle parts of the X-chromosomal moiety were calculated from many spot pairs, are overlapped, only one point is overlapped according to the case of Fig. 6 in which 3% of abnormal spots were eliminated, and in the case of Fig. 7 and Fig. 8 in which 5% of abnormal spots were eliminated, lower limit of Log Ratio value of the X-chromosomal moiety and upper limit of Log Ratio value the autosomal moiety are sufficiently separated.

Assuming that the X-chromosomal moiety corresponds to the amplified or deleted moiety, these results show that a nucleic acid microarray having high diagnostic accuracy can be obtained by eliminating abnormal spots.

Fig. 9 shows a case in which a method for setting a threshold value in advance and regarding those having outside of the range as abnormal spot is used in eliminating abnormal spots based on the difference between comparative value and intermediate value (that is, in this case, the number of abnormal spots varies depending on each test). In this case, a spot pair having a comparative value of 0.6 or more as the difference between intermediate value and comparative value was determined as an abnormal spot. When compared with Fig. 4, it can be seen that variation in autosomal moiety became small and separation between autosome and X-chromosome became good.

Further, a relationship between the abnormal spot-eliminating ratio and dispersion value (STDEV) of the autosomal region is shown in Fig. 10. When abnormal spots were not eliminated, the STDEV became about 0.13 and it became a small value of from 0.9 to 1.2 when abnormal spots were eliminated.

These results show that variation of data is reduced and a nucleic acid microarray having further high accuracy can be obtained by eliminating abnormal spots.

In addition, Fig. 11 shows a result of plotting the Ratio(Female/Male) value when 3% of spots among the entire spots were eliminated as abnormal spots.

When compared with Fig. 5, it can be seen that the autosomal moiety and the X-chromosomal moiety are more clearly separated by eliminating abnormal spots.

[Example 2] Quality inspection of nucleic acid microarray using labeled abnormality detecting nucleic acid

In this inventive example, it is assumed that a nucleic acid microarray 1 is possessed by the manufacturer and nucleic acid microarrays 2 and 3 are possessed by the user.

<Preparation of unpurified labeled sample nucleic acid>

Cy3-Female DNA and Cy5-Male DNA were prepared by the same method of the <Preparation of unpurified labeled sample nucleic acid> of Comparative Example 1.

<Preparation of unpurified labeled abnormality detecting (correcting) nucleic acid>

Cy5-Cot-1 DNA was prepared by the same method of the <Preparation of unpurified labeled abnormality detecting (correcting) nucleic acid> of Comparative Example 1. In this connection, all of the labeled abnormality detecting (correcting) nucleic acid Cy5-Cot-1 DNA samples to be hybridized with the nucleic acid microarrays 1 to 3 were used by subdividing from the same preparation batch.

<Preparation of hybridization solution containing labeled abnormality detecting (correcting) nucleic acid Cy5-Cot-1 DNA>

This was prepared by the same method of the preparation of hybridization solution containing labeled abnormality detecting (correcting) nucleic acid Cy5-Cot-1 DNA> of Comparative Example 1.

<Preparation of hybridization solution containing labeled sample nucleic acid or labeled abnormality detecting (correcting) nucleic acid>

This was prepared by the same method of the <Preparation of hybridization solution containing labeled sample nucleic acid or labeled abnormality detecting (correcting) nucleic acid> of Comparative Example 1. However, the hybridization solution containing labeled abnormality detecting (correcting) nucleic acid was prepared by adding and mixing, instead of the unpurified labeled sample nucleic aid, 20 $\mu$l of water to and with a hybridization solution containing labeled abnormality detecting (correcting) nucleic acid.

<Pretreatment of nucleic acid microarray>

Pretreatment was carried out by the same method of the <Pretreatment of nucleic acid microarray> of Comparative Example 1. In this connection, a nucleic acid microarray onto which 3 spots for each of 576 species of BAC probe DNA were spotted was used in this case.

<Hybridization>

Hybridization was carried out by the <Hybridization> of Comparative Example 1. However, a hybridization solution containing Cy5-Cot-1 was allowed to effect hybridization on the nucleic acid microarray 1. A hybridization solution containing Cy5-Cot-1 DNA and Cy3-Female DNA was allowed to effect hybridization on the nucleic acid microarray 2. A hybridization solution containing Cy5-Cot-1 DNA and Cy3-Male DNA was allowed to effect hybridization on the nucleic acid microarray 3.

<Data uptake>

Uptake of data was carried out by the same method of <Data uptake> of Comparative Example 1.

<Data processing and results>

By regarding the fluorescence value derived from Cy5-Cot-1 DNA of the nucleic acid array 1 as Fc1, and the fluorescence value derived from Cy5-Cot-1 DNA of the nucleic acid array 2 as Fc2 and the fluorescence value derived from Cy5-Cot-1 DNA of the nucleic acid array 3 as Fc3, the comparative value {Log Ratio(Cot-1/Cot-1)} between each spot pair constituted from the same Block Number, the same Column number and the same Row Number was calculated using the following formula, between nucleic acid arrays 1 and 2 and the nucleic acid arrays 1 and 3.

Log Ratio(Cot-1/Cot-1) = $Log_2$(Fc1/Fc2) for between nucleic acid arrays 1 and 2
Log Ratio(Cot-1/Cot-1) = $Log_2$ (Fc1/Fc3) for between nucleic acid arrays 1 and 3

Next, average value of Log Ratio(Cot-1/Cot-1) between respective arrays was calculated and all of the Log Ratio(Cot-1/Cot-1) values were corrected in such a manner that the average value became zero. Next, difference in Log Ratio(Cot-1/Cot-1) was obtained by calculating absolute value of difference between each spot pair and average value on all spot pairs. Fig. 12 shows illustration of the results.

Fig. 12 shows illustration of the difference in Log Ratio(Cot-1/Cot-1) between the nucleic acid microarrays 1 and 2 obtained by calculating in the manner in order of chromosomes, (in this connection, the difference in Log Ratio(Cot-1/Cot-1) between the nucleic acid microarrays 1 and 3 obtained by calculating in the manner was also calculated in the same manner, though not illustrated).

In this inventive example, those which have a difference in Log Ratio(Cot-1/Cot-1) of 0.6 or more were defined as problematic spots in view of quality control, and spot pair-constituting spots on the nucleic acid microarrays 2 or nucleic acid microarray 3, having a difference in Log Ratio(Cot-1/Cot-1) of more than this value, were eliminated.

Fig. 13 shows a result of eliminating spot pairs having a difference in Log Ratia(Cot-1/Cot-1) of 0.6 or more from the results of Fig. 12. As a result, about 20 spots were problematic spots in view of quality inspection. Though not illustrated, the same calculation was carried out between the nucleic acid microarrays 1 and 3.

Next, comparison was carried out between nucleic acid microarrays 2 and 3. Firstly, correction of fluorescence value of Cy3-Male side allowed to carry out hybridization on the nucleic acid microarray 3 was carried out using the following formula, between nucleic acid microarrays 2 and 3 using the labeled abnormality detecting nucleic acid as the labeled correction nucleic acid. In this connection, the fluorescence value of Cy5-Cot-1 DNA of nucleic acid microarray 2 was regarded as Fc2, and the fluorescence value of Cy3-Female DNA of nucleic acid microarray 2 as F2, the fluorescence value of Cy5-Cot-1 DNA of nucleic acid microarray 3 as Fc3 and the fluorescence value of Cy3-Male DNA of nucleic acid microarray 3 as F3.

$$\text{Fluorescence value of Cy3-Male after correction} = F3 \times Fc2/Fc3$$

Using the mathematical expression, calculation was carried out on all of the spots on the nucleic acid microarrays constituted from the same Block Number, the same Column Number and the same Row Number.

Next, using the fluorescence value of Cy3-Female DNA and the fluorescence value of Cy3-Male DNA after correction obtained in the above, Log Ratio(Female/Male) was calculated by the following formula, between all of the spot pairs which can be formed between the nucleic acid microarrays 2 and 3.

$$\text{Log Ratio(Female/Male)} = \text{Log}_2\text{(fluorescence value of Cy3-Female DNA/fluorescence value of Cy3-Male DNA after correction)}$$

The Log Ratio(Female/Male) calculated in the above was illustrated in order of chromosomes, with the result shown in Fig. 14. Since autosome and X-chromosome were sufficiently separated by this result too, this can be said a good result.

Next, the spot pairs having a Log Ratio(Female/Male) constituting problematic spot pairs in view of quality control, obtained between the nucleic acid microarrays 1 and 2 and nucleic acid microarrays 1 and 3, were eliminated. In this connection, even when the problematic spot pairs in view of quality control were not present only in one pair of arrays, among certain spot pairs between the arrays, these were regarded as the object of elimination.

Fig. 15 is a result of eliminating the spot pairs problematic in view of quality inspection calculated between the nucleic acid microarrays 1 and 2 and the nucleic acid microarrays 1 and 3, from the results of Fig. 14. In comparison with Fig. 14, variation in the autosomal moiety was further reduced. For example, there were 17 cases having a Log Ratio(Female/Male) of 0.2 or more in Fig. 14 but was reduced to 7 in Fig. 15, and these results show that these are nucleic acid microarrays having further high accuracy.

In addition, a result obtained by jointly using the method for eliminating the abnormal spots detected between nucleic acid microarrays 2 and 3 is shown in Fig. 17. By regarding the labeled value of Cy3-Female side Cy5-Cot-1 DNA as Fc2, and the labeled value of Cy3-Male DNA side Cy5-Cot-1 DNA as Fc3, calculation was carried out between all of the spot pairs on the nucleic acid microarrays by the following calculation formula using Log Ratio(Cot-1/Cot-1), between spot pairs constituted from the same Block Number, the same Column Number and the same Row Number between the nucleic acid microarrays 2 and 3.

$$\text{Log Ratio(Cot-1/Cot-1)} = \text{Log}_2(\text{Fc2/Fc1})$$

Next, average value was calculated from the Log Ratio(Cot-1/Cot-1) values between all of the spot pairs calculated, and the Log Ratio(Cot-1/Cot-1) values between all of the spot pairs calculated were corrected in such a manner that the average value calculated by calculation became zero. Next, absolute value of the Log Ratio(Cot-1/Cot-1) after correction was calculated, those having the value of 0.6 or more were regarded as abnormal spots, and calculation results of the Log Ratio

(Female/Male) derived from the corresponding spot pairs were eliminated from the results of Fig. 16. Illustration of the results is Fig. 17.

In this connection, Fig. 16 is a result of enlarged representation of from the 1100$^{th}$ to 1300$^{th}$ in Fig. 14, and Fig. 17 is also an illustration of the same region of Fig. 16. When Fig. 16 and Fig. 17 are compared, since the moiety surrounded by a circle, namely this moiety, is an autosomal moiety, it shows that the nucleic acid microarray is an array which shows smaller noise as the ratio come up to zero and has high performance, and in comparison with Fig. 16, the spot pairs having the Log Ratio(Female/Male) value further distant from the axis X are reduced in the case of Fig. 17, so that the above results show that results of a nucleic acid microarray having reduced variation and further high accuracy can be obtained by the method of this inventive example.

[Example 3] In the case of keeping at least one spot when all spots of the spots spotted with the same probe were determined as abnormal spots

<Preparation of unpurified labeled sample nucleic acid>

Cy3-Sample A and Cy3-Sample B were prepared by the same method of the <Preparation of unpurified labeled sample nucleic acid> of Comparative Example 1. The sample A is DNA which was obtained from a cell which was obtained by immortalizing and cultivating patient specimen who is female and has defect in the chromosome 7. The sample B is DNA which was obtained from blood of a person who is female and has no genetic mutation.

<Preparation of unpurified labeled abnormality detecting nucleic acid (labeled correction nucleic acid)>

Cy5-Cot-1 DNA was prepared by the same method of the <Preparation of unpurified labeled abnormality detecting (correcting) nucleic acid> of Comparative Example 1. In this connection, all of the labeled abnormality detecting (correcting) nucleic acid Cy5-Cot-1 DNA samples to be hybridized with the nucleic acid microarrays 1 and 2 were used by subdividing from the same preparation batch.

<Preparation of hybridization solution containing labeled abnormality detecting (correcting) nucleic acid Cy5-Cot-1 DNA>

This was prepared by the same method of the <Preparation of hybridization solution containing labeled abnormality detecting (correcting) nucleic acid Cy5-Cot-1 DNA> of Comparative Example 1.

<Preparation of hybridization solution containing labeled sample nucleic acid or labeled abnormality detecting (correcting) nucleic acid>

This was prepared by the same method of the <Preparation of hybridization solution containing labeled sample nucleic acid or labeled abnormality detecting (correcting) nucleic acid> of Comparative Example 1.

<Pretreatment of nucleic acid microarray>

Pretreatment was carried out by the same method of the <Pretreatment of nucleic acid microarray> of Comparative Example 1. In this connection, two nucleic acid microarrays onto which 3 spots for each of 632 species of BAC probe DNA were spotted was used in this case.

<Hybridization>

Hybridization was carried out by the method of the <Hybridization> of Comparative Example 1.

<Data uptake>

Uptake of data was carried out by the same method of the <Data uptake> of Comparative Example 1.

<Data processing and results>

By regarding the fluorescence value derived from Cy3-Sample A of the nucleic acid array 1 as F1, and the fluorescence value derived from Cy5-Cot-1 DNA of the same as Fc1, the fluorescence value derived from Cy3-Sample A of the nucleic acid array 2 as F1 and the fluorescence value derived from Cy5-Cot-1 DNA of the same as Fc2, fluorescence value of Cy3-Sample B after correction was obtained using the following formula.

$$\text{Fluorescence value of Cy3-Sample B after correction } (F2') = F2 \times c1/Fc2$$

Using this mathematical expression, fluorescence value of Cy3-Sample B after correction was obtained between each of all spot pairs constituted from the same Block Number, the same Column number and the same Row Number, between the nucleic acid microarray 1 and nucleic acid microarray 2.

Next, using the fluorescence value of Cy3-Sample B after correction obtained by the calculation, comparative values between all of the spot pairs on the nucleic acid microarrays, namely Log Ratio(Sample A/Sample B), were obtained using the following mathematical expression.

$$\text{Log Ratio(Sample A/Sample B)} = \text{Log}_2(F1/F2')$$

Next, average value of the thus calculated Log Ratio(Sample A/Sample B) values was calculated, and all of the Log Ratio(Sample A/Sample B) values were corrected in such a manner that their average value becomes zero.

Next, between nucleic acid microarrays 1 and 2, Log Ratio(Cot-1/Cot-1) was calculated for all of the spot pairs on arrays between nucleic acid microarrays 1 and 2, using the following formula between spot pairs constituted from the same Block Number, the same Column Number and the same Row Number.

$$\text{Log Ratio(Cot-1/Cot-1) for between nucleic acid microarrays 1 and 2} = \text{Log}_2(Fc1/Fc2)$$

Those in which this Log Ratio(Cot-1/Cot-1) value was 0.6 or more were regarded as abnormal spots.

Next, an example of spot pairs as the object of elimination is shown in Fig. 18. The chromosome ID 834 to 836 (numbers starting from number zero) are spot pairs prepared by spotting the same probe nucleic acid, and all of their Log Ratio(Cot-1/Cot-1) values were 0.6 or more so that all of them were abnormal spots to be eliminated.

As an example of the method for keeping at least one spot pairs among them, keeping of the most smaller value of the Log Ratio(Cot-1/Cot-1) values is carried out in this inventive example, and in the case, the chromosome ID 834 is kept in this inventive example.

The Log Ratio(Sample A/Sample B) values having these Log Ratio(Cot-1/Cot-1) values are 0.058, 0.201 and 0.169 in order of younger chromosome ID, showing that it is close to the right answer when the region for the chromosome number is close to the region free from deletion and amplification, and the number 834 selected in this inventive example shows the most small value of the Log Ratio(Sample A/Sample B) among the three candidates, so that these results show desirability of the method carried out in this inventive example for keeping only the Log Ratio(Sample A/Sample B) value having the most smaller Log Ratio(Cot-1/Cot-1) value and eliminating the other spot pairs determined as abnormal spots.

In addition, ID numbers 1536 to 1538, 1704 to 1706 and 1863 to 1865 are also preventing disappearance of the probe nucleic acid-derived information by keeping the Log Ratio(Cot-1/Cot-1) value most close to the intermediate value.

[Example 4] Results of analysis when abnormal spots were eliminated inside the same array or between different arrays

<Preparation of unpurified labeled sample nucleic acid>

Cy3-Sample A DNA and Cy3-Sample B DNA were prepared by the same method of the <Preparation of unpurified labeled sample nucleic acid> of Comparative Example 1. In this connection, a substance collected from a patient analyte having a deleted region was used as the Sample A DNA, and an analyte collected from a normal person as the Sample B DNA.

<Preparation of unpurified labeled abnormality detecting (correcting) nucleic acid>

Cy5-Cot-1 DNA was prepared by the same method of the <Preparation of unpurified labeled abnormality detecting (correcting) nucleic acid> of Comparative Example 1. In this connection, all of the labeled abnormality detecting (correcting) nucleic acid Cy5-Cot-1 DNA samples to be hybridized with the nucleic acid microarrays 1 and 2 were used by subdividing from the same preparation batch.

<Preparation of hybridization solution containing labeled abnormality detecting (correcting) nucleic acid Cy5-Cot-1 DNA>

This was prepared by the same method of the <Preparation of hybridization solution containing labeled abnormality detecting (correcting) nucleic acid Cy5-Cot-1 DNA> of Comparative Example 1.

<Preparation of hybridization solution containing labeled sample nucleic acid or labeled abnormality detecting (correcting) nucleic acid>

This was prepared by the same method of the <Preparation of hybridization solution containing labeled sample nucleic acid or labeled abnormality detecting (correcting) nucleic acid> of Comparative Example 1.

<Pretreatment of nucleic acid microarray>

Pretreatment was carried out by the same method of the <Pretreatment of nucleic acid microarray> of Comparative Example 1. In this connection, two nucleic acid microarrays onto which 3 spots for each of 662 species of BAC probe DNA were spotted was used in this case.

<Hybridization>

Hybridization was carried out by the method of the <Hybridization> of Comparative Example 1.

<Data uptake>

Uptake of data was carried out by the same method of the <Data uptake> of Comparative Example 1.

<Data processing and results>

Log Ratio(Cot-1/Cot-1) was calculated using Cy5-Cot-1 DNA-derived fluorescence values between spot pairs constituted from combinations thinkable from the spots in which the same probe nucleic acid was spotted on the first nucleic acid array. For example, since the number of spots prepared by spotting the same probe nucleic acid is 3 in the case of this inventive example, these were regarded as spots A, B and C and Log Ratio(Cot-1/Cot-1) was calculated using the following formula. In this connection, the fluorescence value was used by subtracting the background fluorescence value.

Between spots A and B Log Ratio(Cot-1 A/Cot-1 B)
Between spots A and C Log Ratio(Cot-1 A/Cot-1 C)
Between spots B and C Log Ratio(Cot-1 B/Cot-1 C)

Next, the Log Ratio(Cot-1/Cot-1) was calculated by the formula for all of the spot pairs presenting on the nucleic acid microarrays, all of the calculated values were then corrected in such a manner that their average manner becomes zero, and then absolute values of these values were calculated.

Those in which the value of Log Ratio(Cot-1/Cot-1) calculated by the calculation formula is 0.6 or more were regarded as the spots to be eliminated (in this inventive example, in order to avoid confusion between the elimination of abnormal spots inside a nucleic acid microarray and the elimination of abnormal spots between nucleic acid microarrays, the former was called positively the spot to be eliminated). For example, when both of the value of Log Ratio(Cot-1 A/Cot-1 B) between spots A and B and the value of Log Ratio(Cot-1 A/Cot-1 C) between spots A and C are 0.6 or more, the spot to be eliminated was determined by taking into consideration the spots which take the most large value of Log Ratio(Cot-1/Cot-1) among the remaining combinations. For example, when the value of Log Ratio(Cot-1 A/Cot-1 B) between spots A and C is the second largest value, spot A was regarded as the spot to be eliminated. Also, when Log Ratio(Cot-1/Cot-1) values of all of the thinkable combinations of spot pairs are 0.6 or more, all spots were regarded as the spot to be eliminated. In addition, the Cy3-Sample side fluorescence value derived from the spot to be eliminated was eliminated.

The spot to be eliminated was determined and eliminated also on the other nucleic acid microarray by the same process.

Next, by regarding the Cy3-Sample A DNA-derived fluorescence value of the nucleic acid microarray 1 as F1, and the Cy5-Cot-1 DNA-derived fluorescence value of the same as Fc1, the Cy3-Sample B DNA-derived fluorescence value of the nucleic acid microarray 2 as F2 and the Cy5-Cot-1 DNA-derived fluorescence value of the same as Fc2, fluorescence value of the Cy3-Sample B DNA after correction was obtained using the following formula.

Fluorescence value of Cy3-Sample B DNA after correction (F2') = F2 x Fc1/Fc2

Using this mathematical expression, fluorescence value of the Cy3-Sample B DNA after correction was obtained on all of the spot pairs constituted from the same Block Number, the same Column Number and the same Row Number, between the nucleic acid microarray 1 and the nucleic acid microarray 2.

Next, using the fluorescence value of the Cy3-Sample B DNA after correction obtained by the calculation, comparative value between all of the spot pairs on the nucleic acid microarrays, namely Log Ratio (Sample A/Sample B), was obtained using the following mathematical expression.

$$\text{Log Ratio(Sample A/Sample B)} = \text{Log}_2(\text{F1/F2'})$$

Next, average value of the calculated Log Ratio (Sample A/Sample B) was calculated and all of the Log Ratio(Sample A/Sample B) values were corrected in such a manner that the average value becomes zero.

Fig. 19 is a result of plotting chromosome numbers on the axis X, and the Log Ratio(Sample A/Sample B) values calculated by the formula on the axis Y. In this connection, Fig. 19 shows a result of spotting average values of comparative values of 3 spots spotted with the same type of probe. In addition, the open circle shows a part which corresponds to autosome, and the closed circle to X-chromosome (the same in the following drawings).

Next, Log Ratio(Cot-1/Cot-1) was calculated on all of the spot pairs on arrays between the nucleic acid microarrays 1 and 2 using the following formula, on spot pairs constituted from the same Block Number, the same Column Number and the same Row Number between the nucleic acid microarrays I and 2.

$$\text{Log Ratio(Cot-1/Cot-1) for between nucleic acid microarrays 1 and 2} = \text{Log}_2(\text{Fc1/Fc2})$$

Next, average value of the Log Ratio (Cot-1/Cot-1) obtained from the calculation result was calculated and all of the Log Ratio(Cot-1/Cot-1) values were corrected in such a manner that the average value becomes zero. The spot pairs in which this value exceed 0.6 were regarded as abnormal spots. In addition, the spot pairs derived from spots having the spot to be eliminated on one or both of the nucleic acid microarrays were also regarded as abnormal spots. Next, the Log Ratio

(Sample A/Sample B) values derived from these abnormal spots were eliminated.

Fig. 20 shows a result of plotting chromosome numbers on the axis X and Log Ratio(Sample A/Sample B) values, in which abnormal spots were eliminated by this method, on the axis Y.

In comparison with Fig. 20, Fig. 19 seems to be frequent in the variation in autosome part plotted with open circle (it is suitable that this part is close to zero).

In addition, while value of the degree of dispersion (STDEV) of Fig. 19 is 0.134, degree of dispersion of Fig. 20 is 0.126, so that variation of data id evidently reduced by eliminating abnormal spots.

The above results show that variation is reduced and results of nucleic acid microarray having further high accuracy are obtained by the method of this inventive example.

[Example 5] Results of analysis when a normal cell-derived nucleic acid was used as the labeled abnormality detecting nucleic acid

<Digestion of DNA with restriction enzyme>

A 1.5 μg portion of a sample nucleic acid (normal sample nucleic acid (female); it has been known in advance that the sample nucleic acid is normal) was put into a 1.5 ml capacity microtube (platinum tube, mfd. by BM Equipment Corporation), 5 μl of a buffer for a restriction enzyme *Dpn*II and 1.5 μl (20 U) of the restriction enzyme *Dpn*II were added thereto and the total volume was adjusted to 50 μl by further adding water. By putting this microtube into a block incubator BI-535A (mfd. by ASTEC) set to 37°C, the enzyme reaction was carried out for 2 hours. Two hours thereafter, the microtube was taken out from the block incubator.

<Purification of restriction enzyme-treated DNA>

The purification was carried out using NucleoSpin Kit (mfd. by Japan Genetics). A 100 μl portion of NT buffer was added to 50 μl in total volume of the DNA fragmented by the restriction enzyme and mixed, and then spin down was lightly carried out. Total volume of this solution was added to the cartridge of NucleoSpin and put into a micro volume high speed cooling centrifuge MX-300 (mfd. by TOMY SEIKO), and centrifugation was carried out at 11,000 rpm for 1 minute. By discarding the eluate, 600 μl of the washing liquid NT-3 buffer was added to the cartridge and 1 minute of centrifugation was carried out again at 11,000 rpm using MX-300. By discarding the eluate, centrifugation was carried out again at 11,000 rpm for 2 minutes using MX-300 in order to remove the solution remaining in the column completely. The cartridge was taken out from the centrifuge, 46 μl of water was added to the cartridge, 1 minute of incubation was carried out and then centrifugation was carried out at 11,000 rpm for 1 minute.

<Preparation of labeled DNA>

BioPrime Array CGH Genomic Labeling System (mfd. by Invitrogen) was used in the labeling. A 21 μl portion of the DNA after carrying out the restriction enzyme treatment and purification was put into a 1.5 ml capacity microtube, 20 μl of the 10 x dCTP nucleotide mix attached to the BioPrime kit was added thereto and the mixture was heated for 5 minutes on a block incubator heated to 95°C. Five minutes thereafter, the microtube was taken out from the block incubator and rapidly cooled on ice. After 10 minutes of the rapid cooling, 5 μl of the 10 x dCTP nucleotide mix attached to the BioPrime kit, 3 μl of 1 mM Cy dCTP (mfd. by G E Healthcare Bioscience) and 1 μl of the Exo-Klenow attached to the BioPrime kit were added thereto and lightly mixed and then, after carrying out spin down, labeling reaction was carried out for 2 hours on a block incubator heated to 37°C.

<Purification of labeled DNA>

The purification was carried out by the same method of the purification of restriction enzyme-treated DNA. However, 60 μl of water was used as the desorption solution of DNA.

The process was carried out on two sample nucleic acids (analyte sample and normal sample, that is, the samples A and B) and correction nucleic acid. However, the sample nucleic acid was labeled with Cy3, and abnormality detecting (correcting) nucleic acid was labeled with Cy5. In this connection, according to this inventive example, the samples in which the sample nucleic acids (Samples A and B) were labeled correspond to the labeled sample nucleic acids, and the sample in which a normal sample nucleic acid (female) was labeled to the abnormality detecting (correcting) nucleic acid.

<Preparation of hybridization solution containing purified labeled DNA>

A 28 μl portion of each of the purified labeled DNA derived from the sample nucleic acids and abnormality detecting (correcting) nucleic acid was put into a 1.5 ml capacity microtube, 65 μl of human Cot-1 DNA, 12 μl of 3 M sodium acetate (pH 5.2) and 310 μl of ethanol of -20°C were added thereto and lightly mixed, this was allowed to stand still at -80°C for 10 minutes and then centrifugation was carried out at 15,000 rpm and at 4°C for 30 minutes using MX-300. After centrifugation, the microtube was taken out to discard the supernatant by taking care of not discarding the precipitate and then left in the dark for 10 minutes while opening the cap of the microtube in order to evaporate ethanol in the microtube and precipitate. Next, 63 μl of Hybridization Buffer MM # 1, 9 μl of 100 mg/μl yeast tRNA and 18 μl of 20% SDS were added to the microtube and allowed to stand still for 30 minutes in the dark. After 30 minutes thereof, stirring was carried out until the precipitate was completely dissolved.

By doing this, hybridization solutions containing two kinds of purified labeled DNA, namely a mixture of analyte sample-derived labeled sample nucleic acid and abnormality detecting (correcting) nucleic acid-derived labeled abnormality detecting (correcting) nucleic acid and a mixture of normal sample-derived labeled sample nucleic acid and abnormality detecting (correcting) nucleic acid-derived labeled abnormality detecting (correcting) nucleic acid, were obtained.

<Pretreatment of nucleic acid microarray>

Pretreatment of nucleic acid microarray was carried out by the same method of the <Pretreatment of nucleic acid microarray> of Comparative Example 1. In this connection, as the nucleic acid microarray, a nucleic acid microarray having the number of spots of a total of 1,986, in which 662 species of probe DNA were spotted three spots for each, wherein the probe DNA species prepared from the BAC clone were spotted at NGK Insulators, was used.

<Pre-hybridization>

Pre-hybridization was carried out by heating the hybridization solution containing purified labeled DNA at 75°C for 8 minutes and heating at 42°C for 30 minutes.

<Hybridization using hybridizer>

HYBRIMASTER HS-300 (mfd. by Aloka) was used as the hybridizer. After arranging the nucleic acid microarray on the hybridizer, the hybridized solution was coated on the nucleic acid microarray. This was carried out at a hybridization temperature of 42°C for a hybridization time of 48 hours. The hybridizer was set such that stirring was carried out with a roller during the hybridization. The hybridization solutions containing Cy3-Sample A DNA and Cy3-Sample B DNA were added dropwise onto respective nucleic acid microarrays and start button of the hybridizer was pressed. The washing condition was set to (1) 5 ml of 2 x SSC solution at room temperature for 30 seconds, (2) 5 ml of 50% formamide/2 x SSC solution (pH 7.0) at 50°C for 15 minutes, (3) 5 ml of 2 x SSC/0.1% SDS solution (pH 7.0) at 50°C for 30 minutes and (4) 5 ml of 2 x SSC solution at room temperature for 5 minutes, and the hybridizer was set such that stirring was carried out using a roller during the washing. Hybridization was carried out by the hybridizer and the nucleic acid microarray was dried by carrying out 1 minute of centrifugation by Spin Dryer-Mini Model 2350.

<Data uptake>

Uptake of data was carried out by the same method of the <Data uptake> of Comparative Example 1.

<Data processing and results>

Between the fluorescence value derived from the Cy3-Sample A of nucleic acid microarray 1 and the fluorescence value derived from the Cy3-Sample B of nucleic acid microarray 2, Log Ratio(Sample A/Sample B) was calculated on all of the spot pairs on arrays between nucleic acid microarrays 1 and 2 using the following formula, between spot pairs constituted from the same Block Number, the same Column Number and the same Row Number. Fig. 21 is a result of plotting the chromosome numbers on the axis X, and the Log Ratio(Sample A/Sample B) values on the axis Y. That is, a result of the case of not carrying out any correction was shown.

By regarding the fluorescence value derived from the Cy3-Sample A DNA of nucleic acid microarray 1 as F1, the fluorescence value derived from the Cy5-Female DNA of the same array as Fc1, the fluorescence value derived from the Cy3-Sample B DNA of nucleic acid microarray 2 as F2 and the fluorescence value derived from the Cy5-Female DNA of the same array as Fc2, fluorescence value of the Cy3-Sample B DNA after correction was obtained using the following formula.

$$\text{Fluorescence value of Cy3-Sample B DNA after correction } (F2') = F2 \times Fc1/Fc2$$

Using this mathematical formula, fluorescence values of the Cy3-Sample B DNA after correction were obtained on the spot pairs constituted from the same Block Number, the same Column Number and the same Row Number, between the nucleic acid microarrays 1 and 2.

Next, using the fluorescence value of Cy3-Male DNA after correction obtained by the calculation, Log Ratio(Sample A/Sample B) was obtained using comparative values between all of the spot pairs on the nucleic acid microarrays, namely the following mathematical formula.

$$\text{Log Ratio(Sample A/Sample B)} = \text{Log}_2(\text{F1/F2'})$$

Next, average value of the calculated Log Ratio(Sample A/Sample B) values was calculated and all of the Log Ratio(Sample A/Sample B) values were corrected in such a manner that their average value becomes zero.

Fig. 22 is a result of plotting the chromosome numbers on the axis X, and the Log Ratio(Sample A/Sample B) values, wherein average values were calculated between spot pairs spotted with the same probe nucleic acid, on the axis Y.

Next, between nucleic acid microarray 1 and nucleic acid microarray 2, Log Ratio(Cot-1/Cot-1) was calculated on all of the spot pairs on arrays between nucleic acid microarrays 1 and 2 using the following formula, between spot pairs constituted from the same Block Number, the same Column Number and the same Row Number.

$$\text{Log Ratio(Cot-1/Cot-1) for nucleic acid microarrays 1 and 2}$$
$$= \text{Log}_2(\text{Fc1/Fc2})$$

Next, average value of the Log Ratio(Cot-1/Cot-1) values obtained from the calculation results was calculated, and all of the Log Ratio(female/ male) values were corrected in such a manner that the average value becomes zero.

The Log Ratio(Sample A/Sample B) values constituted from spot pairs having corrected Log Ratio(Female/Male) values in which the corrected Log Ratio(Cot-1/Cot-1) value obtained from the results exceeded 0.6 were regarded as abnormal spots and eliminated from the graph of Fig. 22, with the results shown in Fig. 23.

When Fig. 21 and Fig. 22 are compared, it can be seen that variation of data is sharply reduced by the correction of labeled values using labeled abnormality detecting (correcting) nucleic acid.

In addition, when Fig. 22 and Fig. 23 are compared, it can be seen that variation of data is further reduced by the method for eliminating abnormal spots using labeled abnormality detecting (correcting) nucleic acid.

The above results show that results of nucleic acid microarray with reduced variation and further high accuracy can be obtained by the method of this inventive example.

[Example 6] Results of analysis when a vector-derived nucleic acid was used as the labeled abnormality detecting nucleic acid

<Preparation of unpurified labeled sample nucleic acid>

The preparation was carried out in the same manner as that described in <preparation of unpurified labeled sample nucleic acid> of the comparative example 1.

<Preparation of abnormality detecting nucleic acid>

1 clone was selected from RPCI-11 vector, chloramphenicol was added to Luria-Bertani broth so as to be 100 mg/mL, and it was cultivated at 37 °C for one night using BE-43FL (an incubator manufactured by TITEC Corporation). Then, BACJ was extracted using QIAampl miniprep kit of Qiagen. The extracted BAC was digested using restriction enzyme NotI manufactured by Nippon Gene Co., Ltd., and was supplied to agarose electrophoresis. A fragment about 7 kb was cut out, and was purified from the agarose using Nucleospin of MN Corporation. Ligation is carried out therefor for one night at 4 °C using T4 DNA ligase manufactured by Invitrogen Corporation. Thereby, pBAC10L was obtained.

pBAC10L was added to DH-10B chemically competent cell manufactured by Invitrogen Corporation. After being left on ice for 30 minutes, it was humidified at 42°C for 30 seconds, and then left again on ice for 2 minutes. SOC broth of 600μL was added thereto, and it was inoculated in the chloramphenicol-containing LB broth of 100mg/mL. It was cultivated for one night at 37 °C, and then, BAC DNA was extracted using QIAprep spin mini prep kit to obtain the vector-derived abnormality detecting nucleic acid.

<Preparation of unpurified labeled abnormality detecting nucleic acid>

6 μg (6 μl) of the vector-derived abnormality detecting nucleic acid which was prepared in <preparation of abnormality detecting nucleic acid>, 5 μl of water, 20 μl of 2.5xRandom Primers Solution were put into a 1.7 ml capacity micro tube (platinum tube manufactured by BM Equipment Corporation) and were subjected to a heating treatment on BLOCK INCUBATOR BI-535A for 5 minutes at 95 °C. 5 minutes later, the micro tube was taken out, put into BLOCK INCUBATOR BI-535A which was set at 37 °C and then left for 15 minutes. 5 μl of 10xdCTP Nucleotide Mix, 3 μl of Cy5-dCTP Bulk Pack 250nmol (manufactured by GE healthcare bio science), 1 μl of Exo-Klenow Fragment were added thereto, and labeling reaction and amplification reaction were carried out at 37 °C for two hours in BLOCK INCUBATOR BI-535A. Two hours later, the micro tube was taken out from INCUBATOR, and was subjected to heating treatment for 15 minutes on BLOCK INCUBATOR BI -535A which was set at 65 °C, and Exo-Klenow Fragment contained in the reaction solution was deactivated

<Preparation of hybridization solution containing the labeled abnormality detecting nucleic acid>

20 μl of the unpurified labeled abnormality detecting nucleic acid Cy5-vector DNA which was prepared in the <preparation of unpurified labeled abnormality detecting nucleic acid>, 62 μl (62 μg; invitrogen) of unlabeled Cot-01 DNA were put into the 1.7 ml capacity micro tube. 8 μl of 3M sodium acetate (pH 5.2) and 360 μl of ethanol at -20 °C were added, and after mixing, it was left for 10 minutes at -80 °C. Then, centrifuging is carried out at 15,000 rpm for 30 minutes at 4 °C using the centrifuge MX-300 manufactured by TOMY Seiko Co., Ltd.. After centrifuging, precipitation remains on the bottom of the centrifuge tube. Thus, supernatant is eliminated with taking care of not suctioning the precipitation. Then, it was left for 10 minutes with a lid of the centrifuge tube being opened, and the remaining ethanol was eliminated. 10 minutes later, 52 μl of the hybridization preparation solution which was prepared so that formamide is 40%, SSC, and dextran sulfate sodium was 0.075mg/μl and 8 μl of 10% SDS were added, and were left for 30 minutes. 30 minutes later, the precipitation was solved by stirring, and then further stirring was carried out. <Preparation of hybridization solution containing labeled sample nucleic acid and labeled abnormality detecting nucleic acid>

20 μl of unpurified labeled sample nucleic acid of Female DNA which was prepared in the <unpurified labeled sample nucleic acid> and 60 μl of the solution which was prepared in the <preparation of hybridization solution containing labeled abnormality detecting nucleic acid> were put into the 1.7 ml capacity micro tube, and stirring was carried out by sufficiently carrying out the Vortex treatment. Similarly, 20 μl of unpurified labeled sample nucleic acid of Male DNA which was prepared in the <unpurified labeled sample nucleic acid> and 60 μl of the solution which was prepared in the <preparation of hybridization solution containing labeled abnormality detecting nucleic acid Cot-1 DNA> were put into the 1.7 ml capacity micro tube, and stirring was carried out by sufficiently carrying out the Vortex treatment.

<Pretreatment of CGH array>

Pretreatment of CGH array was carried out in a similar manner to the <pretreatment of nucleic acid microarray> of Comparative Example 1. As the CGH array, used was a nucleic acid micro array having 2,136 spots in total in which 3 sets of 712 types of probe DNA which were prepared from BAC clone were spotted by NGK Insulators, Ltd.

<Hybridization>

Hybridization was carried out in a similar manner to that of the <Hybridization> of Comparative Example 1.

<Data uptake>

Data uptake was carried out in a similar manner to that of the <Data uptake> of Comparative Example 1.

<Data processing and result>

Log Ratio(Female/Male) was calculated for each of all spot pairs on the CGH arrays 1 and 2, based on the fluorescence value derived from Cy3-Male of the CGH array 1 and the fluorescence value derived from Cy3-Female of the CGH array 2. Specifically, the following formula was used in the calculation:

$$Log\ Ratio(Female/Male) = Log_2\left(\frac{Cy3\text{-}Female\ derived\text{-}fluorescence\ value}{Cy3\text{-}Male\ derived\text{-}fluorescence\ value}\right)$$

Each spot pair included a spot on the CGH array I and a spot on the CGH array 2 which have the same Block number, the same Column number and the same Row number. Fig. 24 is a graph prepared by plotting the Log Ratio values in which the X axis denotes the chromosome number and the Y axis denotes the Log Ratio value. That is, Fig. 24 shows the results without any correction.

It is assumed that F1 denotes the fluorescence value derived from Cy3-Male of the CGH array 1, Fc1 denotes the fluorescence value derived from Cy5-vector DNA of the CGH array 1, F2 denotes the fluorescence value derived from Cy3-Female of the CGH array 2, Fc2 denotes the fluorescence value derived from Cy5-vector DNA of the CGH array 2. Then, corrected Cy-3 Female-derived fluorescence values were obtained by the following formula.

$$Corrected\ Cy3 - Female\ derived - fluorescence\ value(F2') = F2 \times \frac{Fc1}{Fc2}$$

Using the formula, the corrected Cy3-Female derived-fluorescence value was obtained for each of all the spot pairs.

Then, the comparison value of each of all the spot pairs on the CGH arrays, that is, Log Ratio(Female/Male) was obtained by the following formula based on the thus-calculated corrected Cy3-Female derived-fluorescence value.

$$Log\ Ratio(Female/Male) = Log_2\left(\frac{F1}{F2'}\right)$$

Then, an average value of the calculated Log Ratio(Female/Male) was obtained, and all the calculated Log Ratio(Female/Male) were corrected so that the average value became zero.

Fig. 25 is a graph prepared by plotting the corrected Log Ratio(Female/Male) values in which the X axis denotes the chromosome number and the Y axis denotes the Log Ratio value.

Then, Log Ratio(vector DNA/vector DNA) was calculated for each of all the spot pairs on the CGH arrays 1 and 2. It is noted that each spot pair included the spot on the CGH array 1 and the spot on the CGH array 2 which have the same Block number, the same Column number and the same Row number.

$$Log\ Ratio(vector\ DNA/vector\ DNA)\ between\ CGHarrays\ 1\ and\ 2 = Log_2\left(\frac{Fc1}{Fc2'}\right)$$

Then, an average values of the Log Ratio(vector DNA/vector DNA) was calculated, and all the Log Ratio(vector DNA/vector DNA) were corrected so that the average value became zero.

Spot pairs having 0.6 or more in the corrected Log Ratio(vector DNA/vector DNA) were treated as abnormal spot pairs, and the Log Ratio(Female/Male) of the abnormal spot pairs were eliminated from the graph of Fig. 25. The result is shown in Fig. 26.

Comparing Figs. 24 and 25, it can be seen that variation in data was drastically improved by correction of labeled values using the labeled abnormality detecting nucleic acid.

Furthermore, comparing Figs. 25 and 26, eliminating the abnormal spots (abnormal spot pairs) using the labeled abnormality detecting (correction) nucleic acid improved the variation in data.

The above results show that the method of the embodiment reduces the variation and that the more accurate CGH array results were obtained.

## Claims

1. A nucleic acid analyzing method for identifying pairs of spots comprising an abnormal spot in a nucleic acid microarray having a plurality of spots to which a probe nucleic acid is immobilized that is capable of detecting a target nucleic acid having a target sequence, a, wherein:

the plurality of spots includes a spot X1m on which is immobilised a probe nucleic acid including a probe sequence, a', complementary to the target sequence, a, and a sequence, b', different from the probe sequence, a', wherein m denotes an integer;
n spot pairs, each pair comprising a spot X1i and a spot X2i, the spots X1i and X2i each having a substantially identical probe nucleic acid and can be on the same nucleic acid array or different nucleic acid microarrays; and wherein n is an integer of $\geq 2$, i is an integer, $1 \leq i \leq n$, and $1 \leq m \leq n$;

the method comprising:

hybridizing the sequence, b', with a sequence, b, by adding a labelled abnormality detecting nucleic acid, B, which is a nucleic acid having the sequence, b, which is capable of binding to the sequence, b', to the spot X1m on the nucleic acid microarray;
obtaining a labelled amount value Fc1m of the hybridized abnormality detecting nucleic acid, B, in the spot X1m;
obtaining a labelled amount value Fc2m of the abnormality detecting nucleic acid, B, from a spot X2m on which is immobilised a probe nucleic acid which is substantially identical to that of spot X1m and includes a sequence b' and which is present on the same array of the spot X1m or on a different array; and
determining whether or not the pair of spots X1m and X2m contains an abnormal spot, an abnormal spot being one having an abnormal hybridisation ability among spots present on the nucleic acid array, by:

calculating a comparative value $D_B$m which is a numerical value obtained by comparing the labelled amount value Fc1m and the labelled amount value Fc2m;
calculating a comparative value $D_B$i for each of the n spot pairs, the comparative value $D_B$i being a numerical value obtained by comparing an obtained labelled amount value Fc1i of the hybridized abnormality detecting nucleic acid, B, for the spot X1i on which is immobilised a probe nucleic acid including a sequence b'and an obtained labelled amount value Fc2i of the hybridized abnormality detecting nucleic acid, B, for the spot X2i on which is immobilised a probe nucleic acid including a sequence b'; and

comparing the comparative value $D_B$m with a representative value A, the representative value A being either an average value or median value of the n comparative values $D_B$1 to $D_B$n;
wherein determining whether or not the pair of spots X1m and X2m contains an abnormal spot is based on the magnitude of the difference between the value $D_B$m and the representative value A.

**2.** A method according to Claim 1, wherein:

the hybridizing of the sequence, b', with the sequence, b, is carried out simultaneously with hybridizing the probe sequence, a', with the target sequence, a; and
the obtaining of the labelled amount value Fc1m of the abnormality detecting nucleic acid, B, is carried out simultaneously with obtaining a labelled amount value F1m of the target nucleic acid.

**3.** A method according to Claim 1, wherein each of the comparative values $D_B$i is calculated using the following Formula (1):

$$\text{Comparative Value } D_B i = \mathrm{Log}_2\{(\text{labeled amount value Fc1i})/(\text{labeled amount value Fc2i})\} \quad (1)$$

wherein when the difference between the comparative value $D_B$m and the representative value, A, is larger than a standard deviation value of the n comparative values $D_B$1 to $D_B$n, it is determined that the spot pair including the spots X1m and X2m is an abnormal spot pair.

**4.** A method according to Claim 3, wherein when the difference between the comparative value $D_B$m and the representative value, A, is 0.6 or more, it is determined that the spot pair including the spots X1m and X2m is an abnormal spot pair.

**5.** A method according to Claim 1, further comprising the step of inspecting the quality of the nucleic acid microarray by indicating or eliminating pairs of spots detected as containing an abnormal spot.

**6.** A method according to any preceding claim, wherein the abnormality detecting nucleic acid, B, is a nucleic acid derived from a normal cell, a normal cell being one which has not undergone substantial mutation.

7. A method according to Claim 6, wherein the abnormality detecting nucleic acid, B, includes a repeating sequence.

8. A method according to any preceding claim, wherein the abnormality detecting nucleic acid, B, is a Cot-1 DNA.

9. A method according to any one of Claims 1 to 5, wherein the abnormality detecting nucleic acid, B, is a vector-derived nucleic acid.

10. A method according to any preceding claim, wherein 1 mol or more of the abnormality detecting nucleic acid, B, is used for 1 mol of the target nucleic acid.

11. A program that causes a computer to process data acquired in a method defined in any preceding claim, the data processing comprising:

calculating each comparative value $D_B$i between the labelled amount value Fc1i and the labelled amount value Fc2i for the spot pair including the spot X1i and the spot X2i;
obtaining the representative value, A, based on the n comparative values $D_B$1 to $D_B$n; and
determining, based on the magnitudes of the comparative value $D_B$m and the representative value, A, as to whether or not the spot X1m is the abnormal spot.

**Patentansprüche**

1. Nukleinsäure-Analyseverfahren zum Identifizieren von Paaren von Spots, umfassend einen abnormalen Spot, in einem Nukleinsäure-Mikroarray mit einer Vielzahl von Spots, woran eine Proben-Nukleinsäure immobilisiert ist, die in der Lage ist, eine Ziel-Nukleinsäure mit einer Zielsequenz a zu detektieren, worin:

die Vielzahl der Spots einen Spot X1m umfasst, auf dem eine Proben-Nukleinsäure immobilisiert ist, enthaltend eine Probensequenz a', die zu der Zielsequenz a komplementär ist, und eine Sequenz b', die sich von der Probensequenz a' unterscheidet, worin m eine ganze Zahl bezeichnet;
n Spot-Paare, wobei jedes Paar eine Spot X1i und einen Spot X2i umfasst, worin die Spots X1i und X2i jeweils eine im wesentlichen identische Proben-Nukleinsäure aufweisen und auf dem gleichen Nukleinsäurearray oder verschiedenen Nukleinsäure-Mikroarrays vorliegen können; und
worin n eine ganze Zahl vorn $\geq 2$ ist, i eine ganze Zahl ist, $1 \leq i \leq n$ ist und $1 \leq m \leq n$ ist;

worin das Verfahren umfasst:

Hybridisieren der Sequenz b' mit einer Sequenz b durch Zugeben einer markierten, Abnormalität-detektierenden Nukleinsäure B, welche eine Nukleinsäure mit der Sequenz b ist, die in der Lage ist, an die Sequenz b' zu binden, zu dem Spot X1m auf dem Nukleinsäure-Mikroarray;
Erhalten eines Markierungsmengenwerts Fc1m der hybridisierten Abnormalität-detektierenden Nukleinsäure B in dem Spot X1m;
Erhalten eines Markierungsmengenwerts Fc2m der Abnormalität-detektierenden Nukleinsäure B von einem Spot X2m, worauf eine Proben-Nukleinsäure immobilisiert ist, die im Wesentlichen identisch ist zu derjenigen des Spots X1m und eine Sequenz b' umfasst, und welche auf dem gleichen Array des Spots X1m oder auf einem anderen Array vorliegt; und
Bestimmen, ob das Paar Spots X1m und X2m einen abnormalen Spot enthält oder nicht, wobei ein abnormaler Spot einer ist, der eine abnormale Hybridisierungsfähigkeit unter den Spots, die auf dem Nukleinsäurearray vorliegen, ist, durch:
Berechnen eines Vergleichswerts $D_B$m, welcher ein numerischer Wert ist, der durch Vergleichen des Markierungsmengenwerts Fc1m und des Markierungsmengenwerts Fc2m erhalten wird;
Berechnen eines Vergleichswert $D_B$i für jedes der n Spot-Paare, wobei der Vergleichswert $D_B$i ein numerischer Wert ist, der durch Vergleichen eines erhaltenen Markierungsmengenwerts Fc1i der hybridisierten Abnormalität-detektierenden Nukleinsäure B für den Spot X1i, auf welchem eine Proben-Nukleinsäure, die eine Sequenz b' enthält, immobilisiert ist, und eines erhaltenen Markierungsmengenwerts Fc2i der hybridisierten Abnormalität-detektierenden Nukleinsäure B für den Spot X2i, auf der eine Proben-Nukleinsäure immobilisiert ist, die eine Sequenz b' umfasst, erhalten wird; und

Vergleichen des Vergleichswerts $D_B$m mit einem repräsentativen Wert A, wobei der repräsentative Wert A entweder ein Mittelwert oder ein Medianwert der n-Vergleichswerte $D_B$1 bis $D_B$n ist;
worin das Bestimmen, ob das Paar Sports X1m und X2m einen abnormalen Spot enthält oder nicht, auf der Größe des Unterschieds zwischen dem Wert $D_B$m und dem repräsentativen Wert A beruht.

**2.** Verfahren gemäß Anspruch 1, worin:

das Hybridisieren der Sequenz b' mit der Sequenz b simultan mit dem Hybridisieren der Probensequenz a' mit der Zielsequenz a durchgeführt wird; und
das Erhalten des Markierungsmengenwerts Fc1m der Abnormalität-detektierenden Nukleinsäure B simultan mit dem Erhalten eines Markierungsmengenwerts F1m der Ziel-Nukleinsäure durchgeführt wird.

**3.** Verfahren gemäß Anspruch 1, worin jeder der Vergleichswerte $D_B$i unter Verwendung der folgenden Formel (1) berechnet wird:

$$\text{Vergleichswert } D_B i = \text{Log}_2 = \text{Log}_2\{(\text{Markierungsmengenwert Fc1i})/(\text{Markierungsmengenwert Fc2i})\} \quad (1)$$

worin, wenn der Unterschied zwischen dem Vergleichswert $D_B$m und dem repräsentativen Wert A größer ist als ein Standard-Abweichungswert der n-Vergleichswerte $D_B$1 bis $D_B$n, es bestimmt wird, dass das Spot-Paar, das die Sports X1m und X2m enthält, ein abnormales Spot-Paar ist.

**4.** Verfahren gemäß Anspruch 3, worin bestimmt wird, dass das Spot-Paar, das die Spots X1m und X2m enthält, ein abnormales Sport-Paar ist, wenn der Unterschied zwischen dem Vergleichswert $D_B$m und dem repräsentativen Wert A 0,6 oder mehr beträgt.

**5.** Verfahren gemäß Anspruch 1, ferner umfassend den Schritt des Inspizierens der Qualität des Nukleinsäure-Mikroarrays durch Anzeigen oder Eliminieren von Spot-Paaren, die als einen abnormalen Spot enthaltend detektiert werden.

**6.** Verfahren gemäß irgendeinem vorhergehenden Anspruch, worin die Abnormalität-detektierende Nukleinsäure B eine Nukleinsäure ist, die aus einer normalen Zelle erhalten ist, wobei eine normale Zelle eine ist, die keine substantielle Mutation eingegangen ist.

**7.** Verfahren gemäß Anspruch 6, worin die Abnormalität-detektierende Nukleinsäure B eine Wiederholungssequenz umfasst.

**8.** Verfahren gemäß irgendeinem vorhergehenden Anspruch, worin die Abnormalität-detektierende Nukleinsäure B Cot-1-DNA ist.

**9.** Verfahren gemäß irgendeinem der Ansprüche 1 bis 5, worin die Abnormalität-detektierende Nukleinsäure B eine aus einem Vektor erhaltene Nukleinsäure ist.

**10.** Verfahren irgendeinem vorhergehenden Anspruch, worin 1 mol oder mehr der Abnormalität-detektierenden Nukleinsäure B für 1 mol der Ziel-Nukleinsäure verwendet wird.

**11.** Programm, das verursacht, dass ein Computer Daten verarbeitet, die in einem Verfahren gewonnen werden, wie es in gemäß irgendeinem vorhergehenden Anspruch definiert ist, worin die Datenverarbeitung umfasst:

Berechnen von jedem Vergleichswert $D_B$i zwischen dem Markierungsmengenwert Fc1i und dem Markierungsmengenwert Fc2i für das Spot-Paar, das den Spot X1i und den Spot X2i umfasst;
Erhalten des repräsentativen Werts A auf Grundlage der n-Vergleichswerte $D_B$1 bis $D_B$n; und
Bestimmen, ob der Spot X1m der abnormale Spot ist oder nicht, auf Grundlage der Größen des Vergleichswerts $D_B$m und des repräsentativen Werts A.

## Revendications

1. Procédé d'analyse d'acide nucléique pour identifier des paires de spots comprenant un spot anormal dans une micropuce à acide nucléique comportant une pluralité de spots sur lesquels est immobilisée une sonde d'acide nucléique qui est capable de détecter un acide nucléique cible ayant une séquence cible, a, dans lequel :

   la pluralité de spots comprend un spot X1m sur lequel est immobilisée une sonde d'acide nucléique comprenant une séquence de sonde, a', complémentaire de la séquence cible, a, et une séquence, b', différente de la séquence de sonde, a', où m indique un nombre entier ;
   n paires de spots, chaque paire comprenant un spot X1i et un spot X2i, les spots X1i et X2i ayant chacun une sonde d'acide nucléique essentiellement identique et pouvant être sur la même puce à acide nucléique ou sur des micropuces à acide nucléique différentes ; et
   où n est un nombre entier $\geq 2$, i est un nombre entier, $1 \leq i \leq n$, et $1 \leq m \leq n$ ;
   le procédé comprenant :

      l'hybridation de la séquence, b', avec une séquence, b, en ajoutant un acide nucléique détectant une anomalie marqué, B, qui est un acide nucléique ayant la séquence, b, qui est capable de se lier à la séquence, b', sur le spot X1m sur la micropuce à acide nucléique ;
      l'obtention d'une valeur de quantité marquée Fc1m de l'acide nucléique détectant une anomalie hybridé, B, dans le spot X1m ;
      l'obtention d'une valeur de quantité marquée Fc2m de l'acide nucléique détectant une anomalie, B, à partir d'un spot X2m sur lequel est immobilisée une sonde d'acide nucléique qui est essentiellement identique à celle du spot X1m et qui comprend une séquence b', et qui est présente sur la même puce du spot X1m ou sur une puce différente ; et

   la détermination que la paire de spots X1m et X2m contient ou pas un spot anormal, un spot anormal étant un spot présentant une capacité d'hybridation anormale parmi les spots présents sur la puce à acide nucléique, par :

      le calcul d'une valeur comparative $D_B$m qui est une valeur numérique obtenue en comparant la valeur de quantité marquée Fc1m et la valeur de quantité marquée Fc2m ;
      le calcul d'une valeur comparative $D_B$i pour chacune des n paires de spots, la valeur comparative $D_B$i étant une valeur numérique obtenue en comparant une valeur de quantité marquée obtenue Fc1i de l'acide nucléique détectant une anomalie hybridé, B, pour le spot X1i sur lequel est immobilisée une sonde d'acide nucléique comprenant une séquence b' et une valeur de quantité marquée obtenue Fc2i de l'acide nucléique détectant une anomalie hybridé, B, pour le spot X2i sur lequel est immobilisée une sonde d'acide nucléique comprenant une séquence b' ; et
      la comparaison de la valeur comparative $D_B$m avec une valeur représentative A, la valeur représentative A étant soit une valeur moyenne soit une valeur médiane des n valeurs comparatives $D_B$1 à $D_B$n ;

   dans lequel la détermination que la paire de spots X1m et X2m contient ou pas un spot anormal est basée sur l'ampleur de la différence entre la valeur $D_B$m et la valeur représentative A.

2. Procédé selon la revendication 1, dans lequel :

   l'hybridation de la séquence, b', avec la séquence, b, est réalisée simultanément à l'hybridation de la séquence de sonde, a', avec la séquence cible, a ; et
   l'obtention de la valeur de quantité marquée Fc1m de l'acide nucléique détectant une anomalie, B, est réalisée simultanément à l'obtention d'une valeur de quantité marquée F1m de l'acide nucléique cible.

3. Procédé selon la revendication 1, dans lequel chacune des valeurs comparatives $D_B$i est calculée en utilisant la formule (1) suivante :

$$\text{Valeur comparative } D_B i = \log_2 \{(\text{valeur de quantité marquée Fc1i})/(\text{valeur de quantité marquée Fc2i})\} \quad (1)$$

dans lequel lorsque la différence entre la valeur comparative $D_B$m et la valeur représentative, A, est supérieure à une valeur d'écart-type des n valeurs comparatives $D_B$1 à $D_B$n, il est déterminé que la paire de spots comprenant les spots X1m et X2m est une paire de spots anormale.

**4.** Procédé selon la revendication 3, dans lequel lorsque la différence entre la valeur comparative $D_B$m et la valeur représentative, A, est de 0,6 ou plus, il est déterminé que la paire de spots comprenant les spots X1m et X2m est une paire de spots anormale.

**5.** Procédé selon la revendication 1, comprenant en outre l'étape d'inspection de la qualité de la micropuce à acide nucléique en indiquant ou en éliminant des paires de spots détectées comme contenant un spot anormal.

**6.** Procédé selon l'une quelconque des revendications précédentes, dans lequel l'acide nucléique détectant une anomalie, B, est un acide nucléique dérivé d'une cellule normale, une cellule normale étant une cellule qui n'a pas subi de mutation essentielle.

**7.** Procédé selon la revendication 6, dans lequel l'acide nucléique détectant une anomalie, B, comprend une séquence répétitive.

**8.** Procédé selon l'une quelconque des revendications précédentes, dans lequel l'acide nucléique détectant une anomalie, B, est un ADN Cot-1.

**9.** Procédé selon l'une quelconque des revendications 1 à 5, dans lequel l'acide nucléique détectant une anomalie, B, est un acide nucléique dérivé d'un vecteur.

**10.** Procédé selon l'une quelconque des revendications précédentes, dans lequel 1 mol ou plus de l'acide nucléique détectant une anomalie, B, est utilisée pour 1 mol de l'acide nucléique cible.

**11.** Programme qui amène un ordinateur à traiter des données acquises dans un procédé défini dans l'une quelconque des revendications précédentes, le traitement des données comprenant :

le calcul de chaque valeur comparative $D_B$i entre la valeur de quantité marquée Fc1i et la valeur de quantité marquée Fc2i pour la paire de spots comprenant le spot X1i et le spot X2i ;
l'obtention de la valeur représentative, A, basée sur les n valeurs comparatives $D_B$1 à $D_B$n ; et
la détermination, en se basant sur les ampleurs de la valeur comparative $D_B$m et de la valeur représentative, A, que le spot X1m est ou n'est pas le spot anormal.

FIG. 1

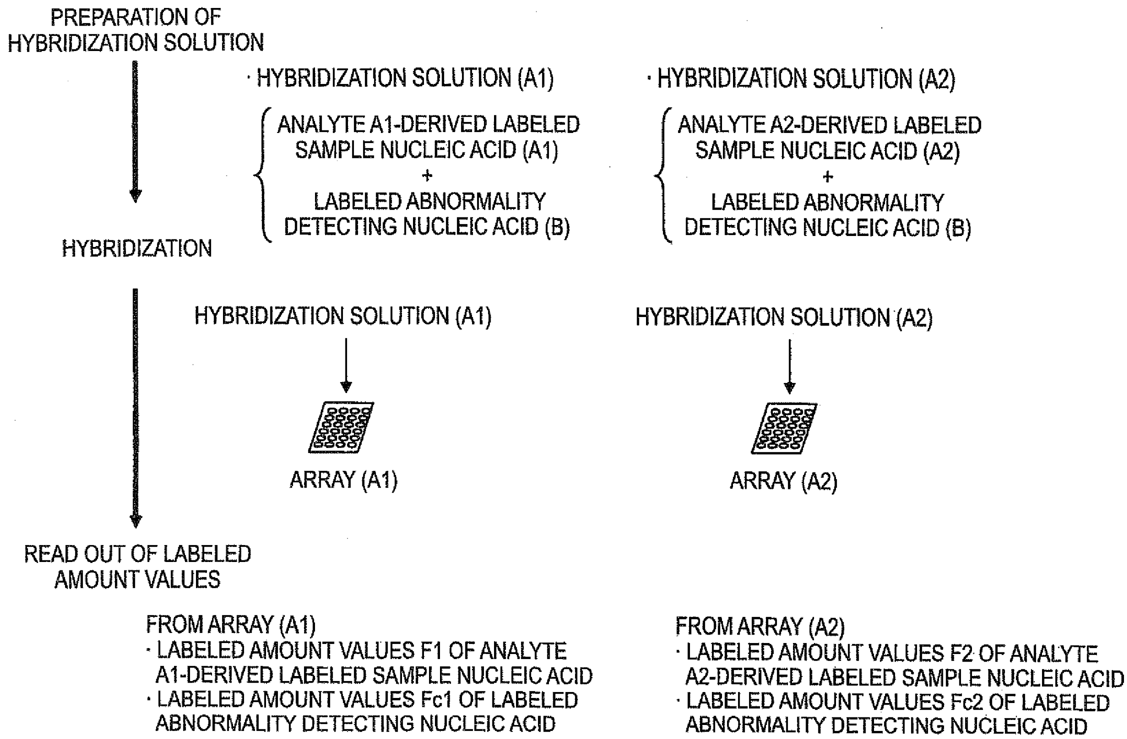
PREPARATION OF HYBRIDIZATION SOLUTION
· HYBRIDIZATION SOLUTION (A1)
ANALYTE A1-DERIVED LABELED SAMPLE NUCLEIC ACID (A1)
+
LABELED ABNORMALITY DETECTING NUCLEIC ACID (B)
· HYBRIDIZATION SOLUTION (A2)
ANALYTE A2-DERIVED LABELED SAMPLE NUCLEIC ACID (A2)
+
LABELED ABNORMALITY DETECTING NUCLEIC ACID (B)
HYBRIDIZATION
HYBRIDIZATION SOLUTION (A1)
ARRAY (A1)
HYBRIDIZATION SOLUTION (A2)
ARRAY (A2)
READ OUT OF LABELED AMOUNT VALUES
FROM ARRAY (A1)
· LABELED AMOUNT VALUES F1 OF ANALYTE A1-DERIVED LABELED SAMPLE NUCLEIC ACID
· LABELED AMOUNT VALUES Fc1 OF LABELED ABNORMALITY DETECTING NUCLEIC ACID
FROM ARRAY (A2)
· LABELED AMOUNT VALUES F2 OF ANALYTE A2-DERIVED LABELED SAMPLE NUCLEIC ACID
· LABELED AMOUNT VALUES Fc2 OF LABELED ABNORMALITY DETECTING NUCLEIC ACID

*FIG. 2A*

CALCULATION OF Log VALUES ON ALL SPOTS BASED ON LABELED ABNORMALITY DETECTING NUCLEIC ACIDS

$$\mathrm{Log}_2 \frac{\text{LABELED AMOUNT VALUE Fc2 OF LABELED ABNORMALITY DETECTING NUCLEIC ACID}}{\text{LABELED AMOUNT VALUE Fc1 OF LABELED ABNORMALITY DETECTING NUCLEIC ACID}}$$

*FIG. 2B*

DETERMINATION OF ABNORMAL SPOT

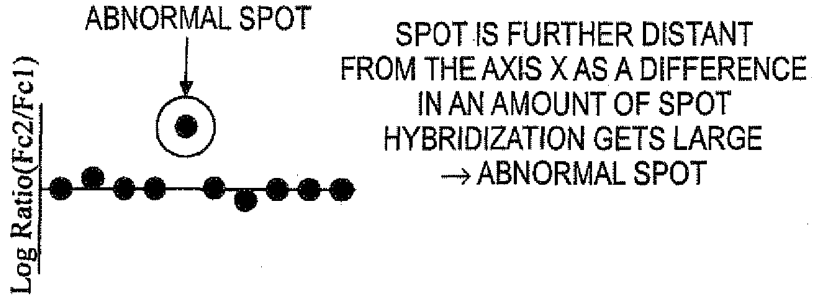


*FIG. 2C*

CALCULATION OF Log VALUES (Log Ratios) OF SAMPLES OF ALL SPOTS EXCLUDING ABNORMAL SPOTS

$$\mathrm{Log} \frac{\text{LABELED AMOUNT VALUE F2 OF LABELED SAMPLE NUCLEIC ACID}}{\text{LABELED AMOUNT VALUE F1 OF LABELED SAMPLE NUCLEIC ACID}}$$

*FIG. 2D*

PLOTTING OF Log VALUES OF SAMPLE NUCLEIC ACID

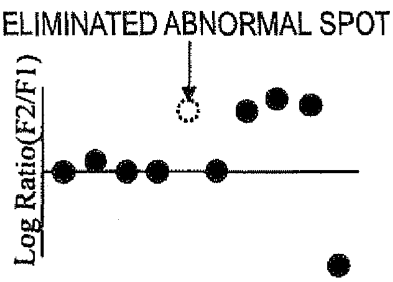


*FIG. 2E*

FURTHER CORRECTION OF LABELED ABNORMALITY DETECTING NUCLEIC ACID (LABELED CORRECTION NUCLEIC ACID) USING LABELED AMOUNT VALUES

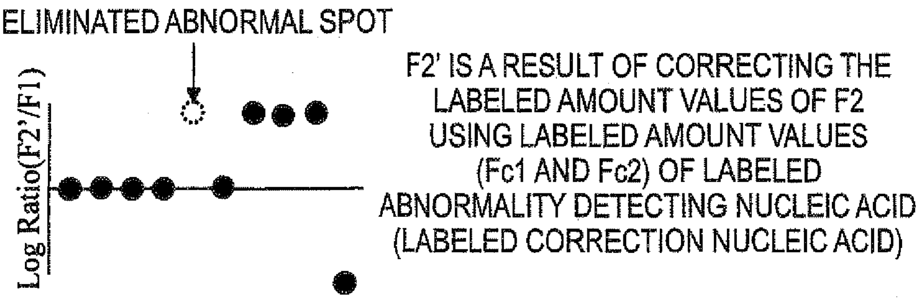

FIG. 3

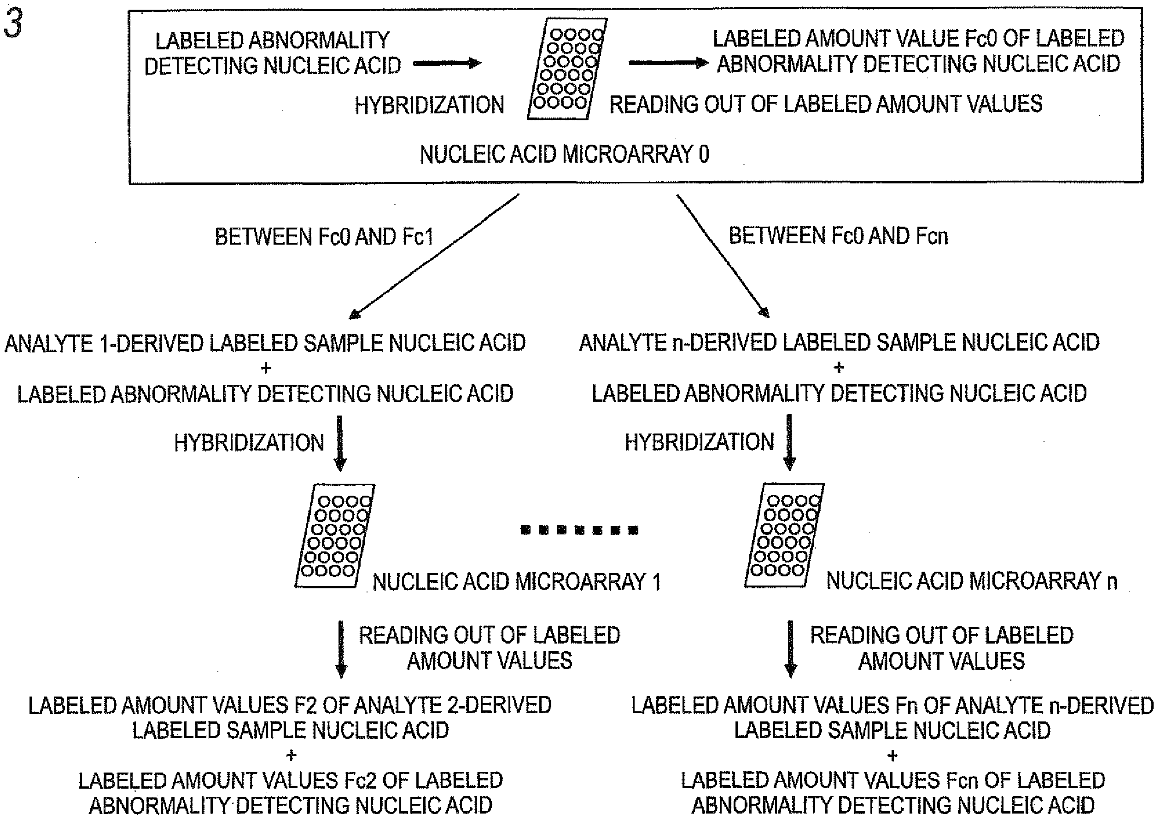
LABELED ABNORMALITY DETECTING NUCLEIC ACID
HYBRIDIZATION
LABELED AMOUNT VALUE Fc0 OF LABELED ABNORMALITY DETECTING NUCLEIC ACID
READING OUT OF LABELED AMOUNT VALUES
NUCLEIC ACID MICROARRAY 0
BETWEEN Fc0 AND Fc1
BETWEEN Fc0 AND Fcn
ANALYTE 1-DERIVED LABELED SAMPLE NUCLEIC ACID
+
LABELED ABNORMALITY DETECTING NUCLEIC ACID
ANALYTE n-DERIVED LABELED SAMPLE NUCLEIC ACID
+
LABELED ABNORMALITY DETECTING NUCLEIC ACID
HYBRIDIZATION
HYBRIDIZATION
NUCLEIC ACID MICROARRAY 1
NUCLEIC ACID MICROARRAY n
READING OUT OF LABELED AMOUNT VALUES
READING OUT OF LABELED AMOUNT VALUES
LABELED AMOUNT VALUES F2 OF ANALYTE 2-DERIVED LABELED SAMPLE NUCLEIC ACID
+
LABELED AMOUNT VALUES Fc2 OF LABELED ABNORMALITY DETECTING NUCLEIC ACID
LABELED AMOUNT VALUES Fn OF ANALYTE n-DERIVED LABELED SAMPLE NUCLEIC ACID
+
LABELED AMOUNT VALUES Fcn OF LABELED ABNORMALITY DETECTING NUCLEIC ACID

FIG. 4

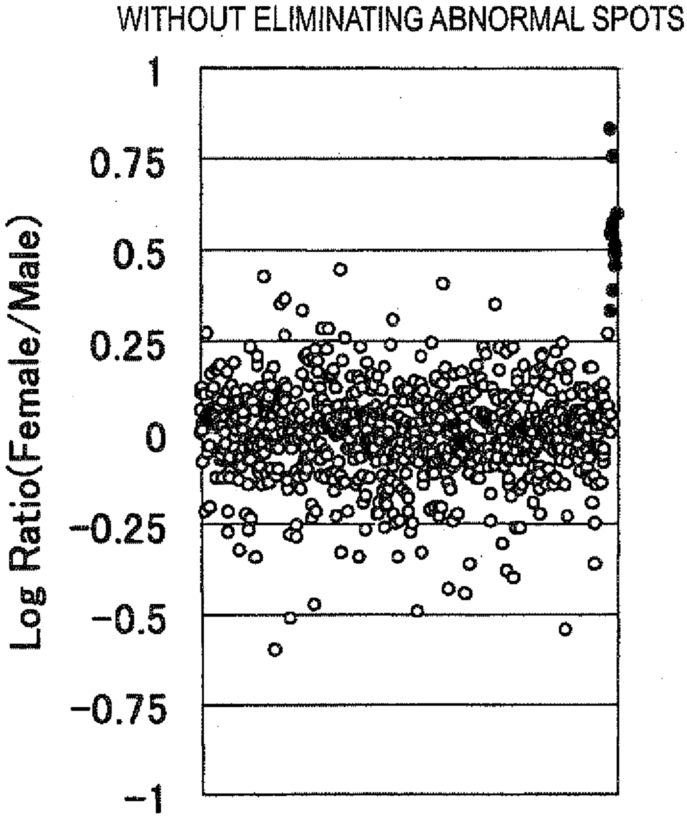
WITHOUT ELIMINATING ABNORMAL SPOTS
1
0.75
0.5
0.25
0
-0.25
-0.5
-0.75
-1
Log Ratio(Female/Male)

FIG. 5

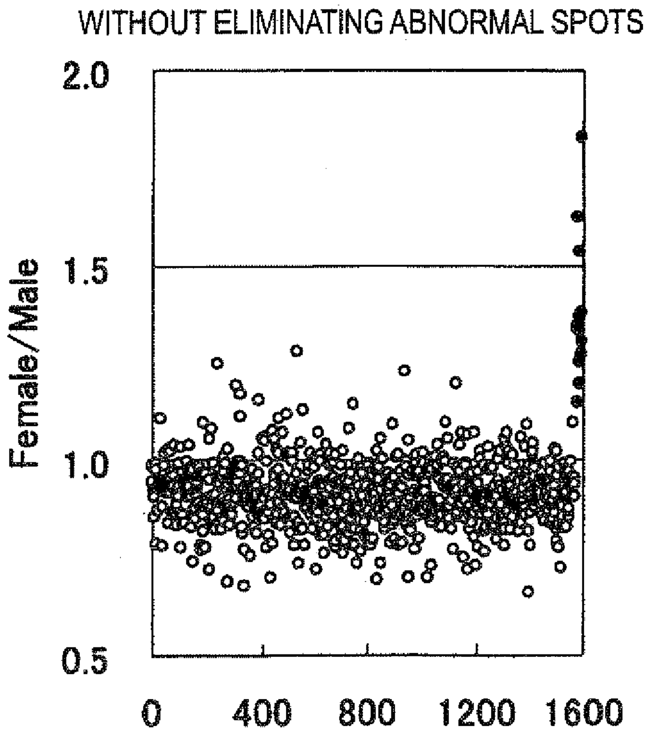
WITHOUT ELIMINATING ABNORMAL SPOTS
2.0
1.5
1.0
0.5
Female/Male
0
400
800
1200
1600

FIG. 6

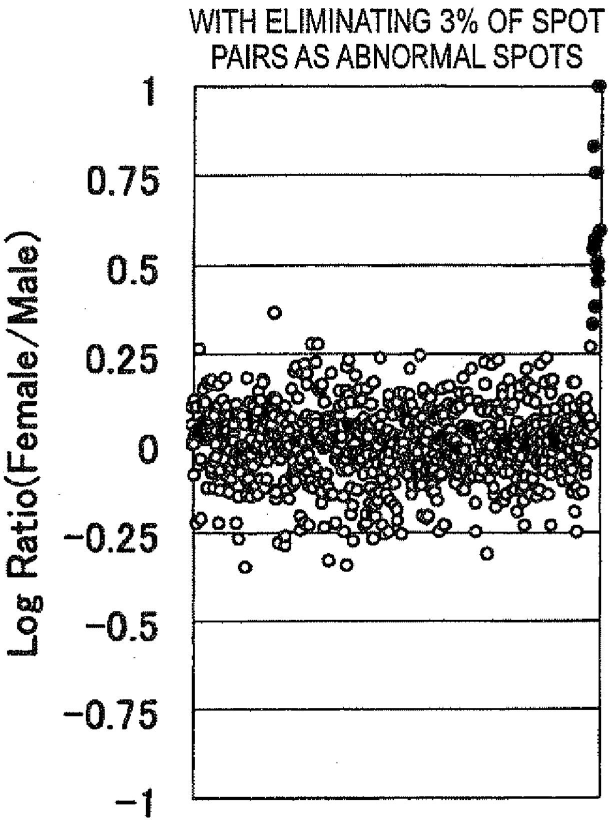
WITH ELIMINATING 3% OF SPOT PAIRS AS ABNORMAL SPOTS
Log Ratio(Female/Male)
1
0.75
0.5
0.25
0
−0.25
−0.5
−0.75
−1

FIG. 7

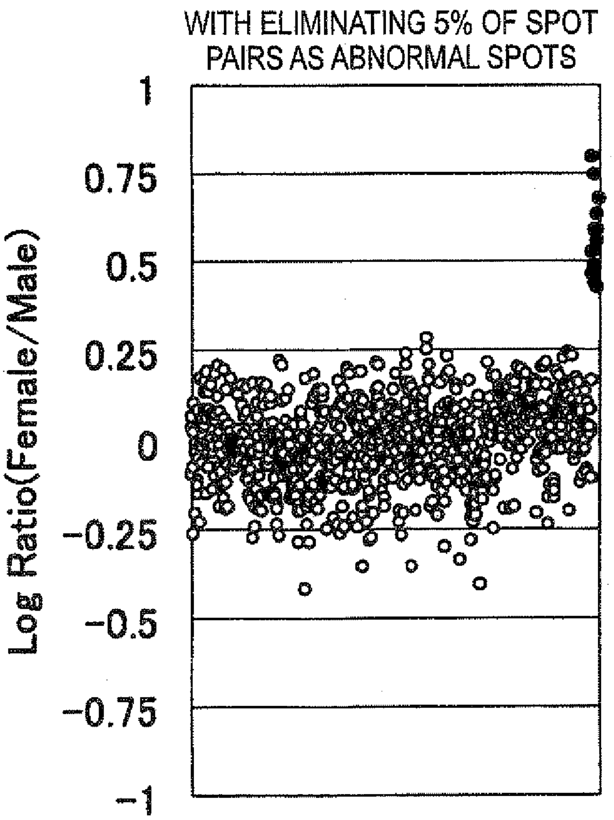
WITH ELIMINATING 5% OF SPOT PAIRS AS ABNORMAL SPOTS
Log Ratio(Female/Male)
1
0.75
0.5
0.25
0
−0.25
−0.5
−0.75
−1

FIG. 8

WITH ELIMINATING 10% OF SPOT PAIRS AS ABNORMAL SPOTS

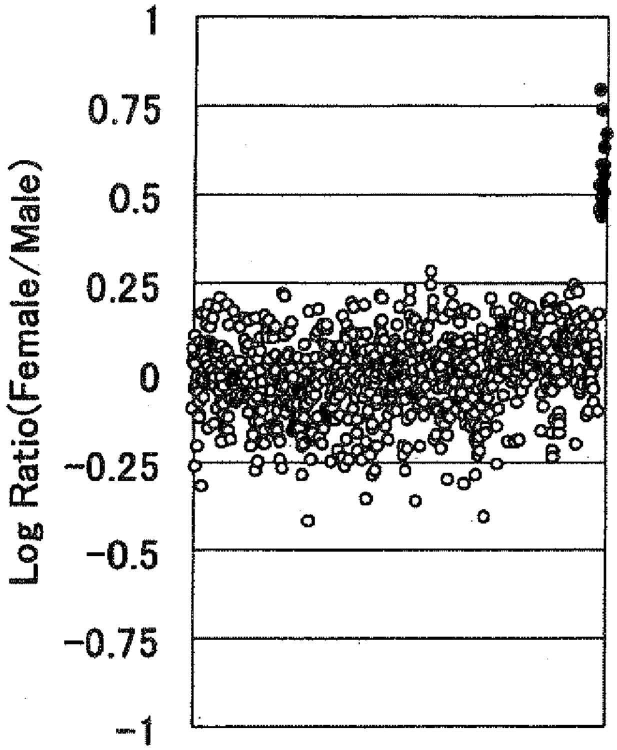


FIG. 9

WITH ELIMINATING, AS ABNORMAL SPOTS, SPOT PAIRS HAVING 0.6 OR MORE IN A DIFFERENCE BETWEEN AN INTERMEDIATE VALUE AND A COMPARISON VALUE

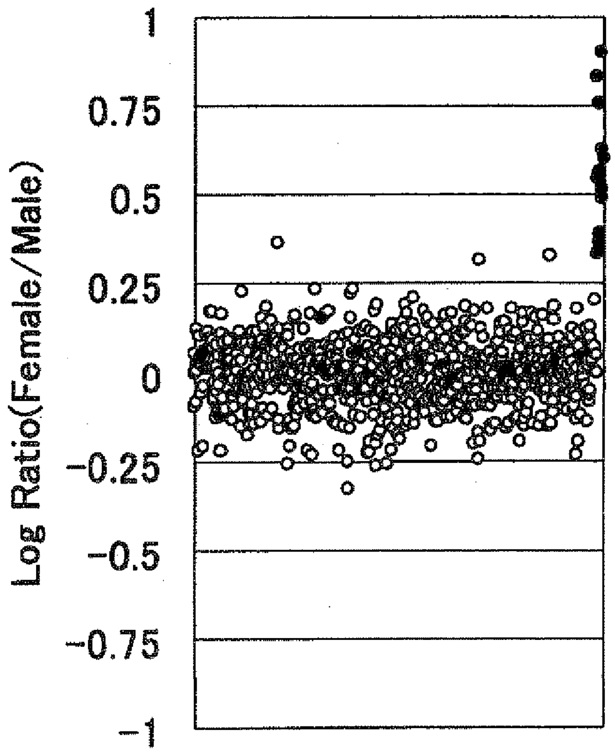

FIG. 10

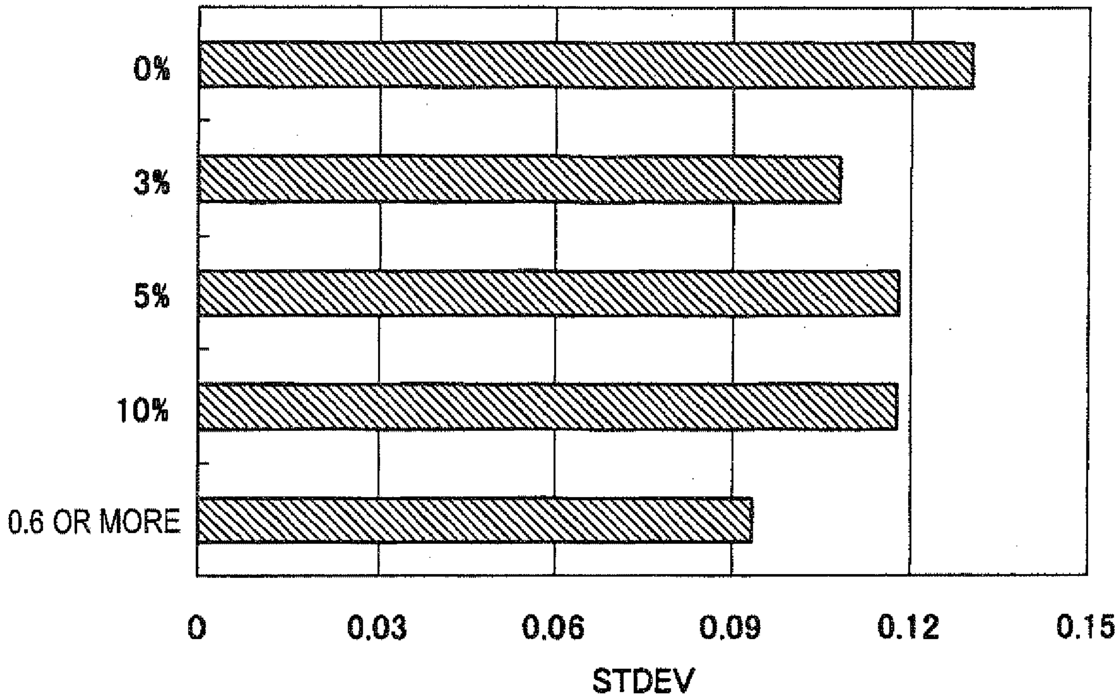
0%
3%
5%
10%
0.6 OR MORE
0
0.03
0.06
0.09
0.12
0.15
STDEV

FIG. 11

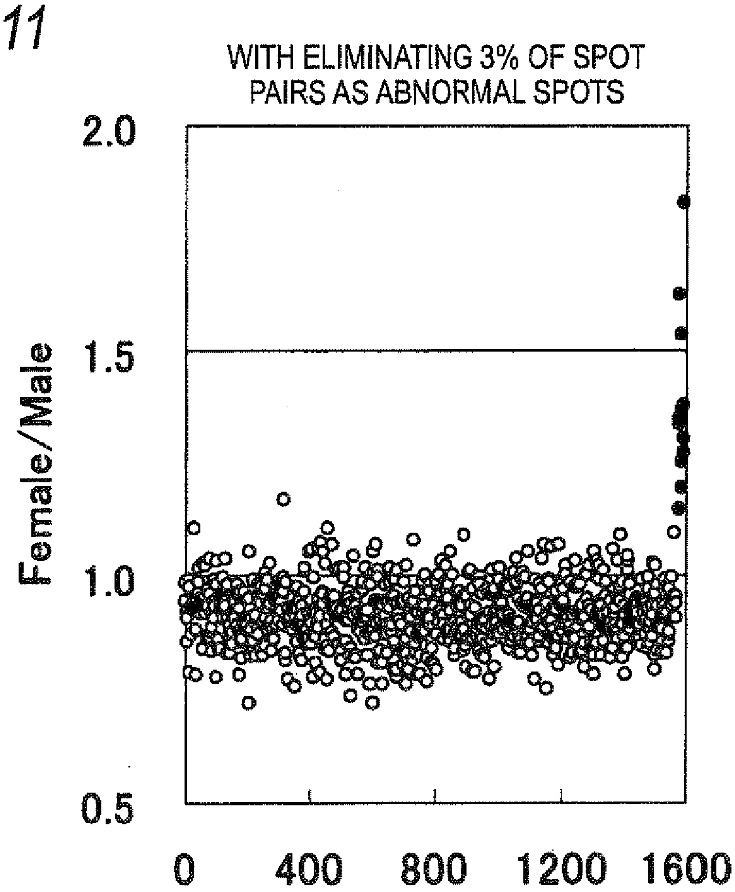
WITH ELIMINATING 3% OF SPOT PAIRS AS ABNORMAL SPOTS
2.0
1.5
1.0
0.5
Female/Male
0
400
800
1200
1600

FIG. 12

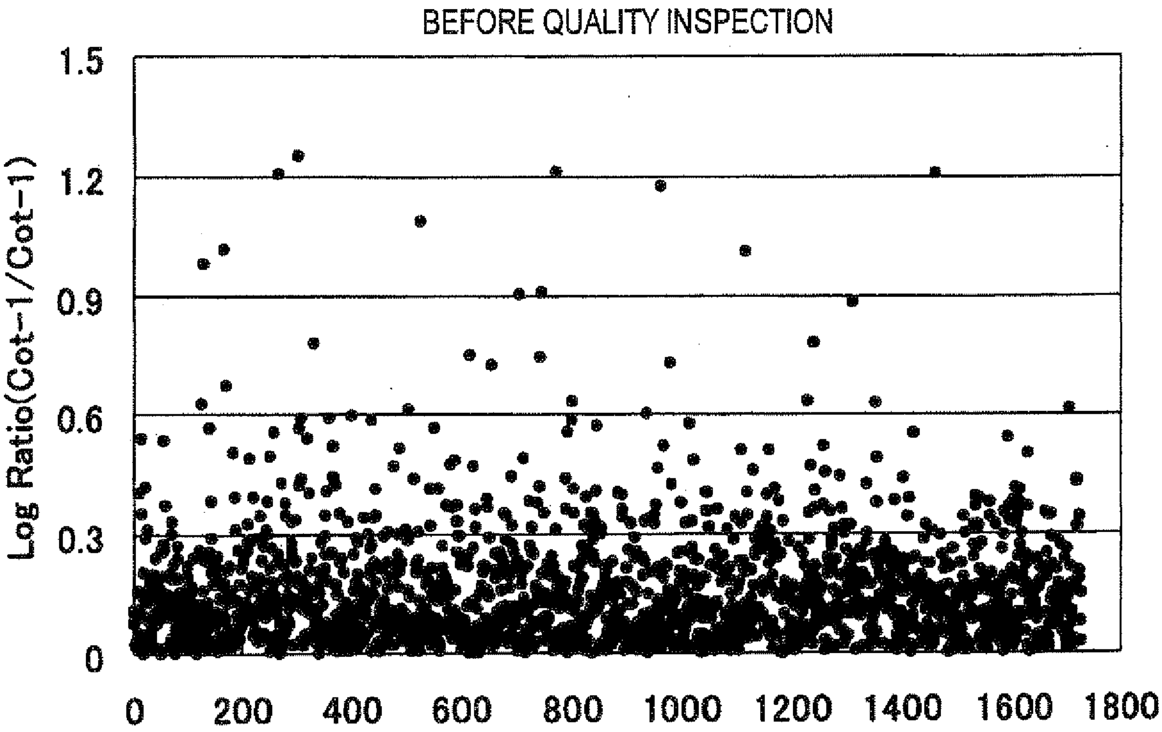
BEFORE QUALITY INSPECTION
Log Ratio(Cot-1/Cot-1)
1.5
1.2
0.9
0.6
0.3
0
0
200
400
600
800
1000
1200
1400
1600
1800

FIG. 13

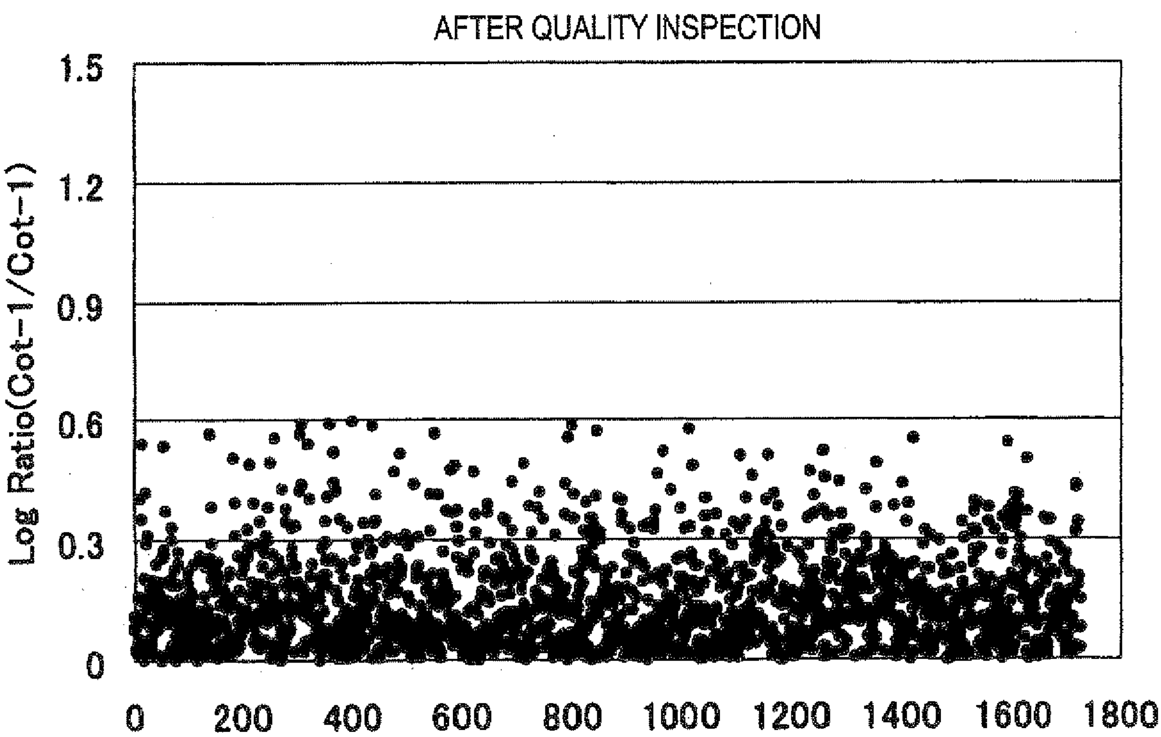
AFTER QUALITY INSPECTION
Log Ratio(Cot-1/Cot-1)
1.5
1.2
0.9
0.6
0.3
0
0
200
400
600
800
1000
1200
1400
1600
1800

FIG. 14

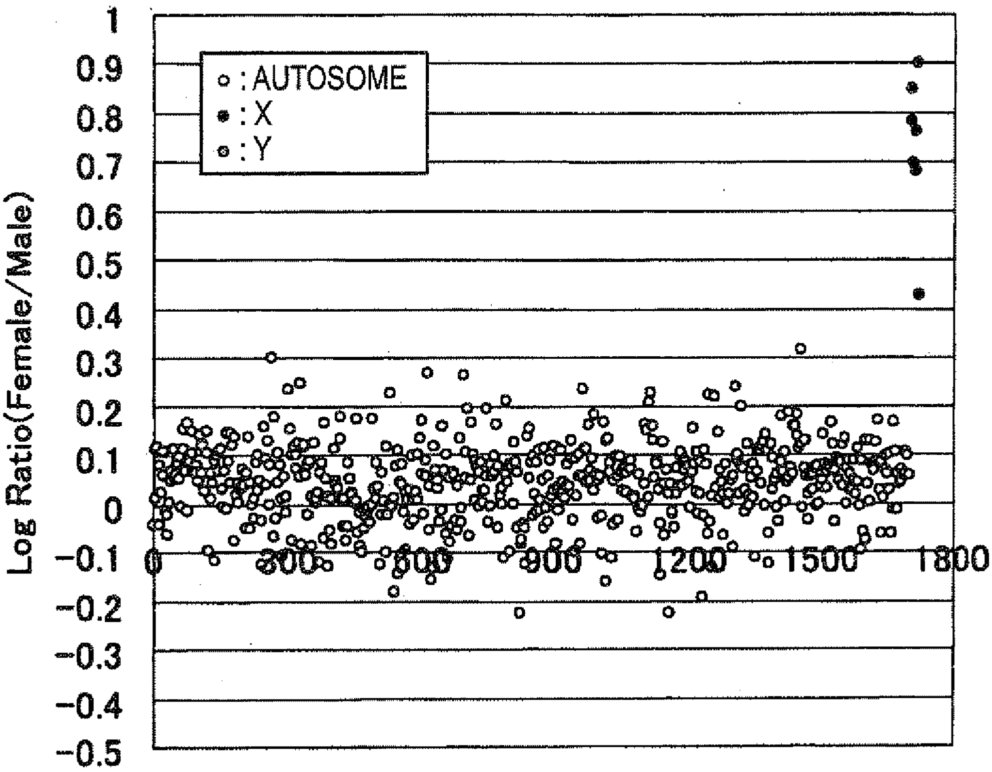
○ : AUTOSOME
● : X
◎ : Y
Log Ratio(Female/Male)
1
0.9
0.8
0.7
0.6
0.5
0.4
0.3
0.2
0.1
0
-0.1
-0.2
-0.3
-0.4
-0.5
0
300
600
900
1200
1500
1800

FIG. 15

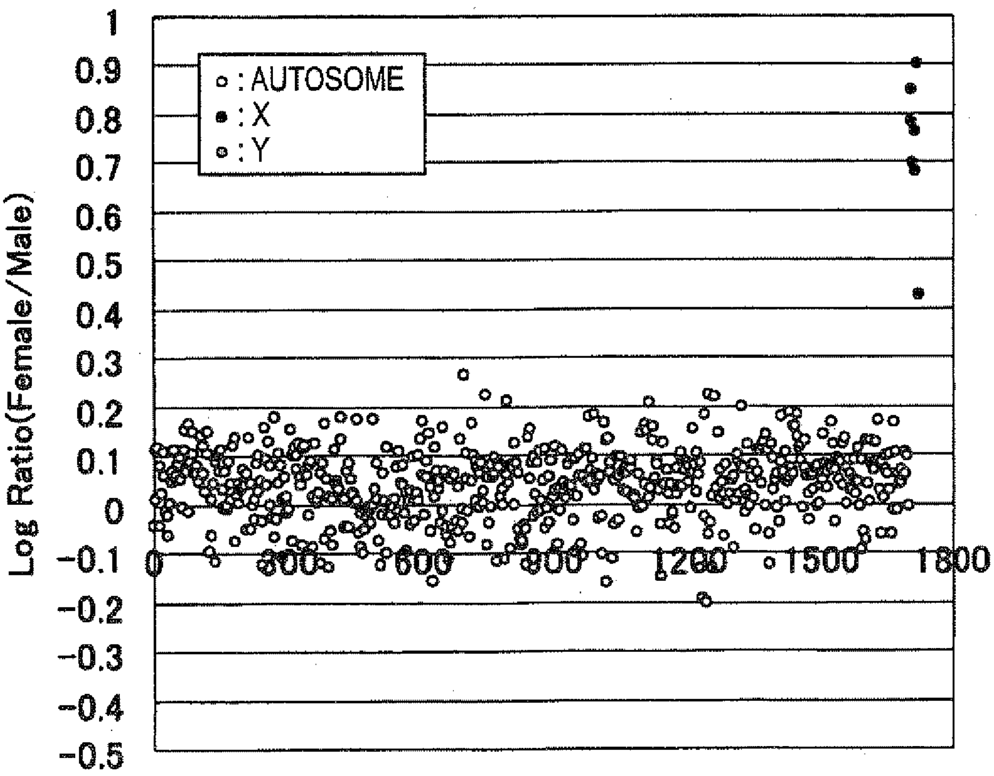
○ : AUTOSOME
● : X
◎ : Y
Log Ratio(Female/Male)
1
0.9
0.8
0.7
0.6
0.5
0.4
0.3
0.2
0.1
0
-0.1
-0.2
-0.3
-0.4
-0.5
0
300
600
900
1200
1500
1800

FIG. 16

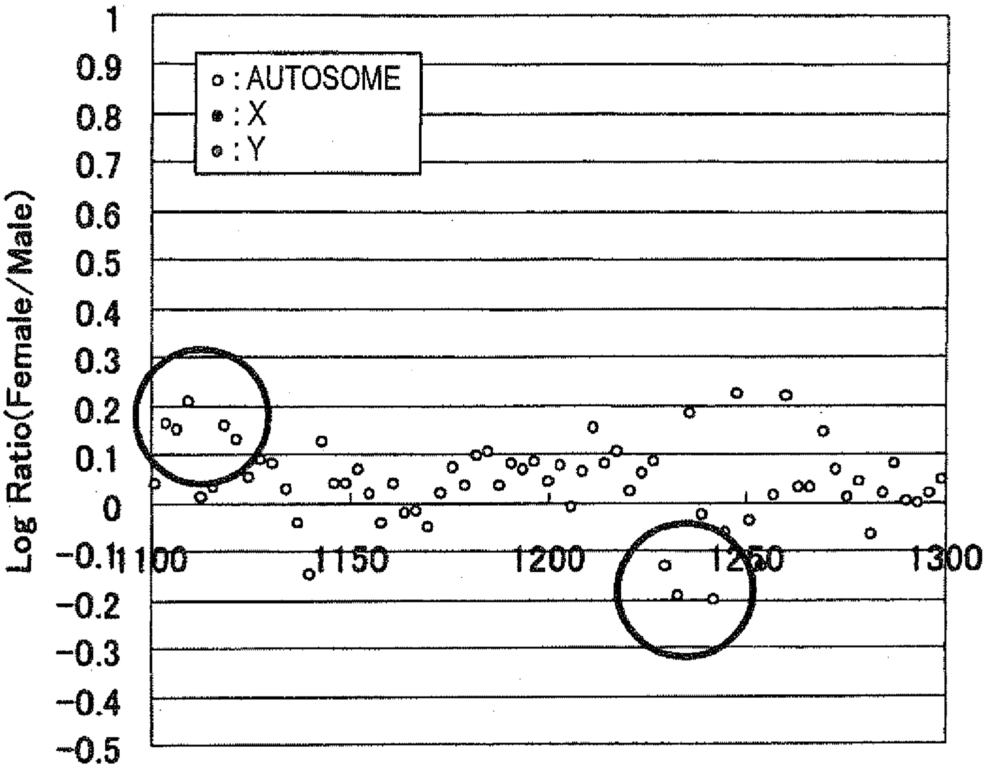

: AUTOSOME
: X
: Y
Log Ratio(Female/Male)
1
0.9
0.8
0.7
0.6
0.5
0.4
0.3
0.2
0.1
0
-0.1
-0.2
-0.3
-0.4
-0.5
1100
1150
1200
1250
1300


FIG. 17

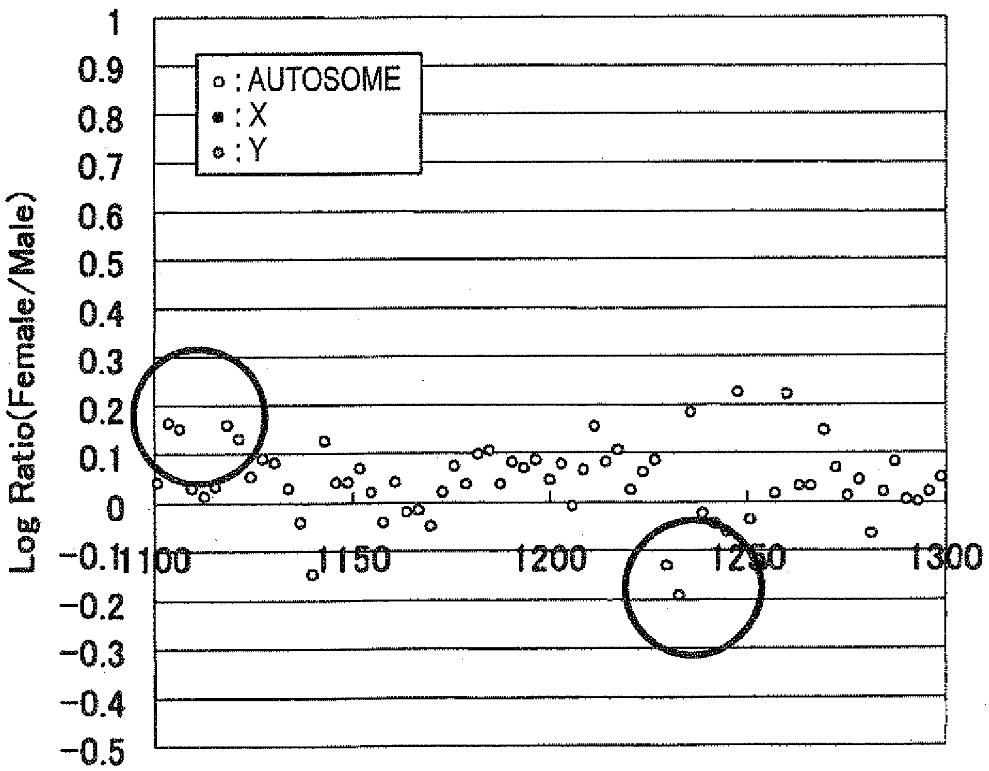

: AUTOSOME
: X
: Y
Log Ratio(Female/Male)
1
0.9
0.8
0.7
0.6
0.5
0.4
0.3
0.2
0.1
0
-0.1
-0.2
-0.3
-0.4
-0.5
1100
1150
1200
1250
1300

FIG. 18

| CHROMOSOME NUMBER | Log Ratio (Cot-1 / Cot-1) | UN-CORRECTED Log Ratio (Sample A / Sample B) | CORRECTED Log Ratio (Sample A / sample B) | SPOT PAIRS TO BE LEFT |
|---|---|---|---|---|
| 834 | 0.687 | −0.563 | 0.112 | ○ |
| 835 | 0.772 | −0.505 | 0.255 | |
| 836 | 0.907 | −0.672 | 0.223 | |
| 1536 | 0.654 | 0.467 | −0.198 | ○ |
| 1537 | 0.896 | 0.479 | −0.428 | |
| 1538 | 1.053 | 0.712 | −0.354 | |
| 1704 | 0.664 | 0.519 | −0.156 | ○ |
| 1705 | 0.700 | 0.456 | −0.256 | |
| 1706 | 0.724 | 0.563 | −0.173 | |
| 1863 | 0.775 | 0.499 | −0.288 | ○ |
| 1864 | 0.855 | 0.414 | −0.453 | |
| 1865 | 0.923 | 0.404 | −0.531 | |

OPEN CIRCLES INDICATE THE SPOT PAIRS TO BE LEFT

FIG. 19

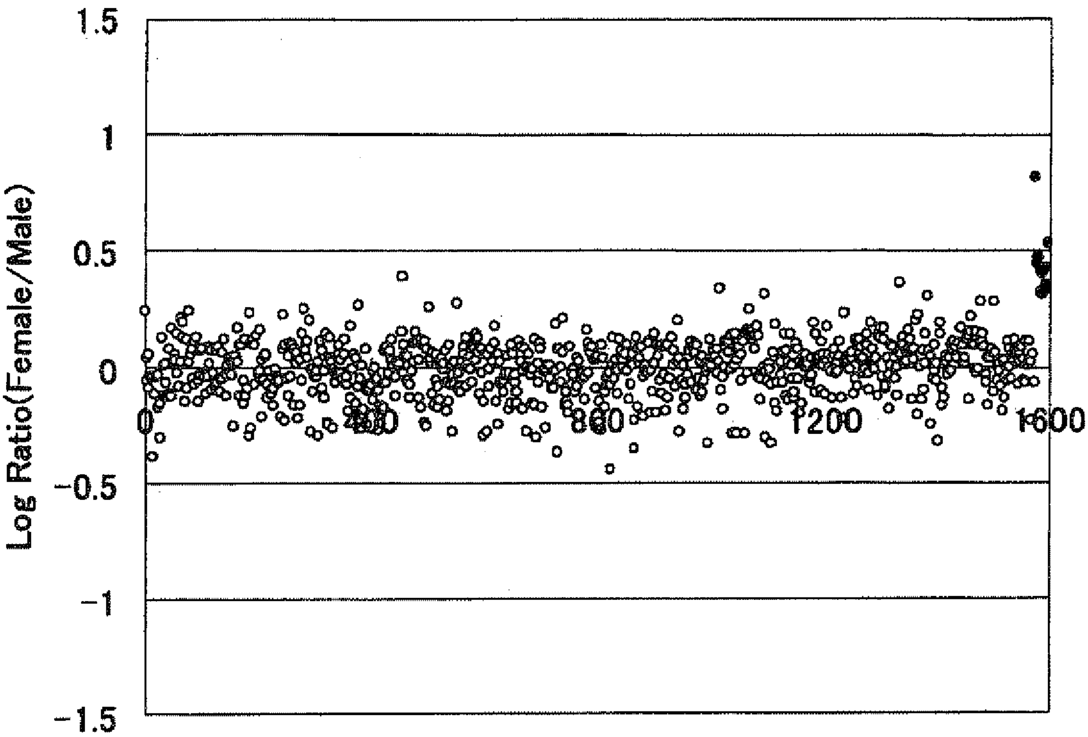

FIG. 20

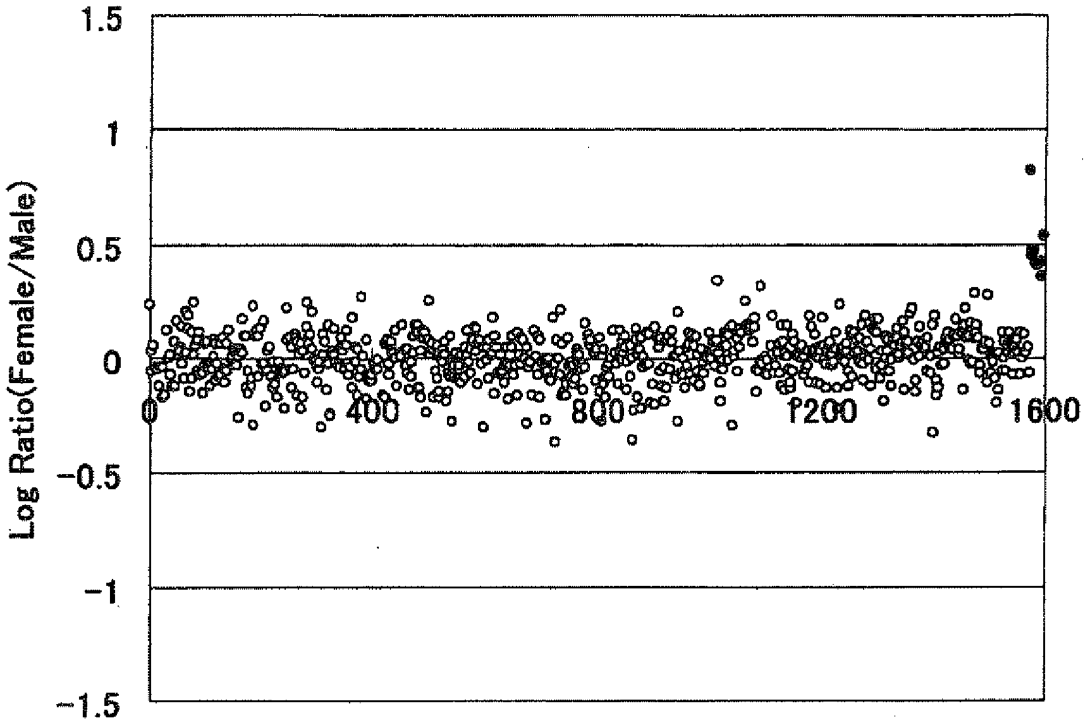
1.5
1
0.5
0
-0.5
-1
-1.5
Log Ratio(Female/Male)
0
400
800
1200
1600

FIG. 21

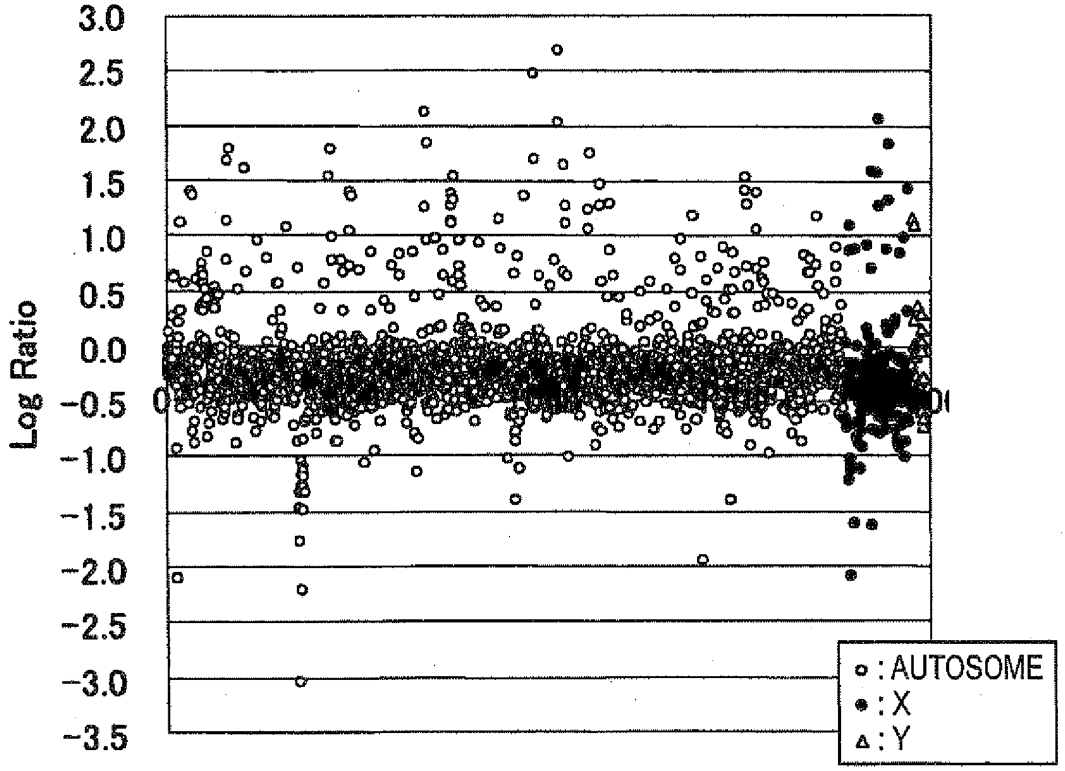
3.0
2.5
2.0
1.5
1.0
0.5
0.0
-0.5
-1.0
-1.5
-2.0
-2.5
-3.0
-3.5
Log Ratio
○ : AUTOSOME
● : X
△ : Y

FIG. 22

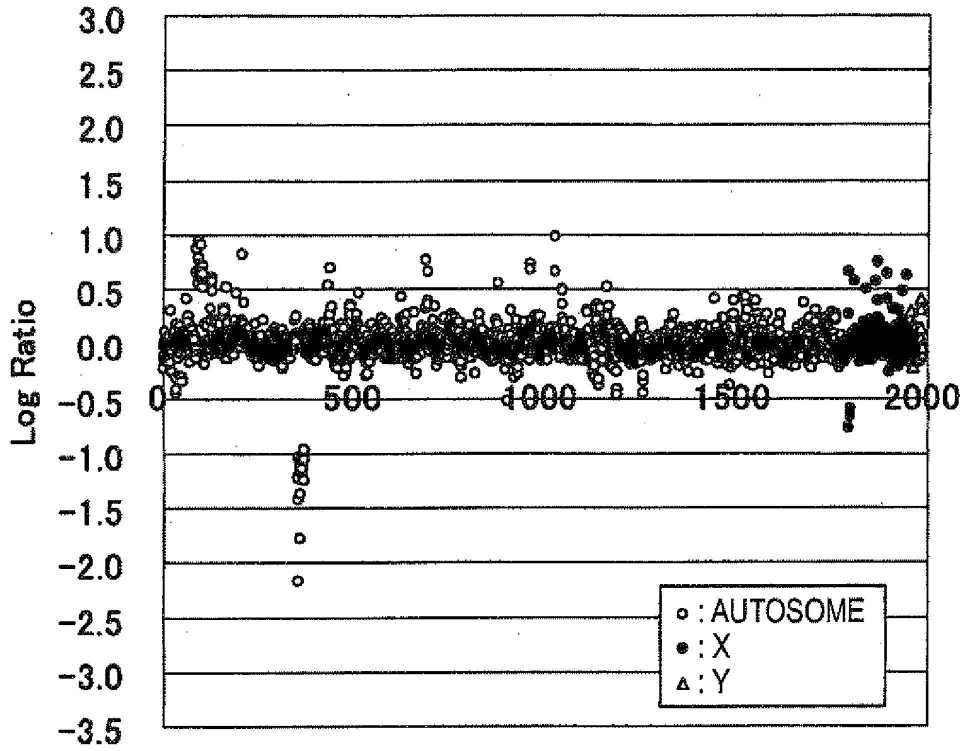
3.0
2.5
2.0
1.5
1.0
0.5
0.0
-0.5
-1.0
-1.5
-2.0
-2.5
-3.0
-3.5
Log Ratio
0
500
1000
1500
2000
: AUTOSOME
: X
: Y

FIG. 23

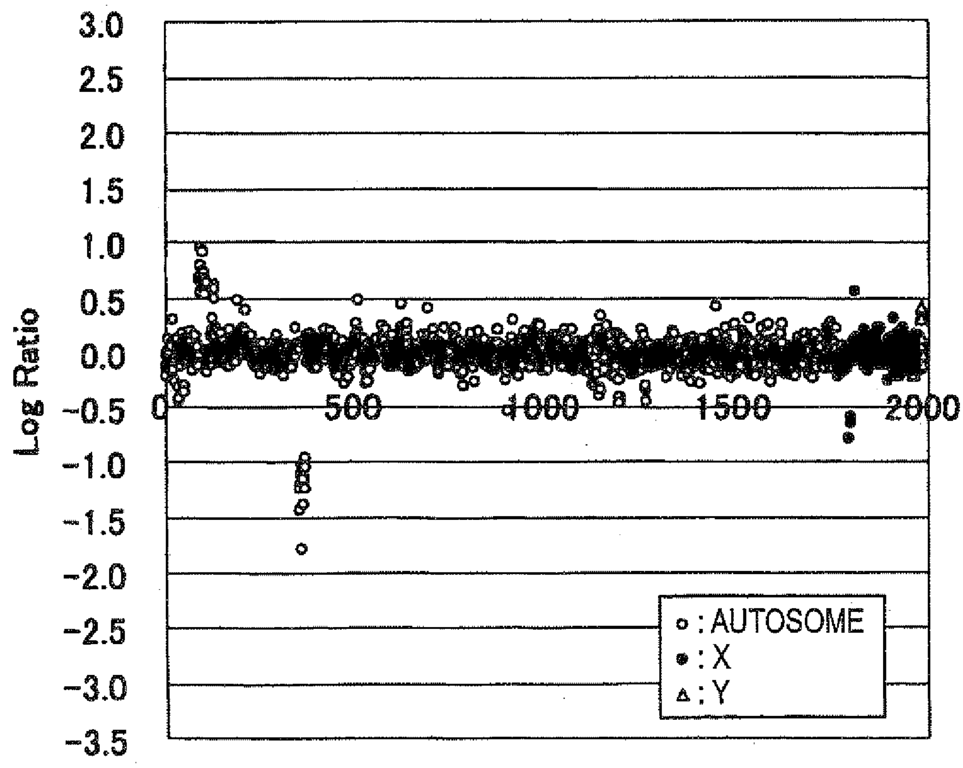
3.0
2.5
2.0
1.5
1.0
0.5
0.0
-0.5
-1.0
-1.5
-2.0
-2.5
-3.0
-3.5
Log Ratio
0
500
1000
1500
2000
: AUTOSOME
: X
: Y

FIG. 24

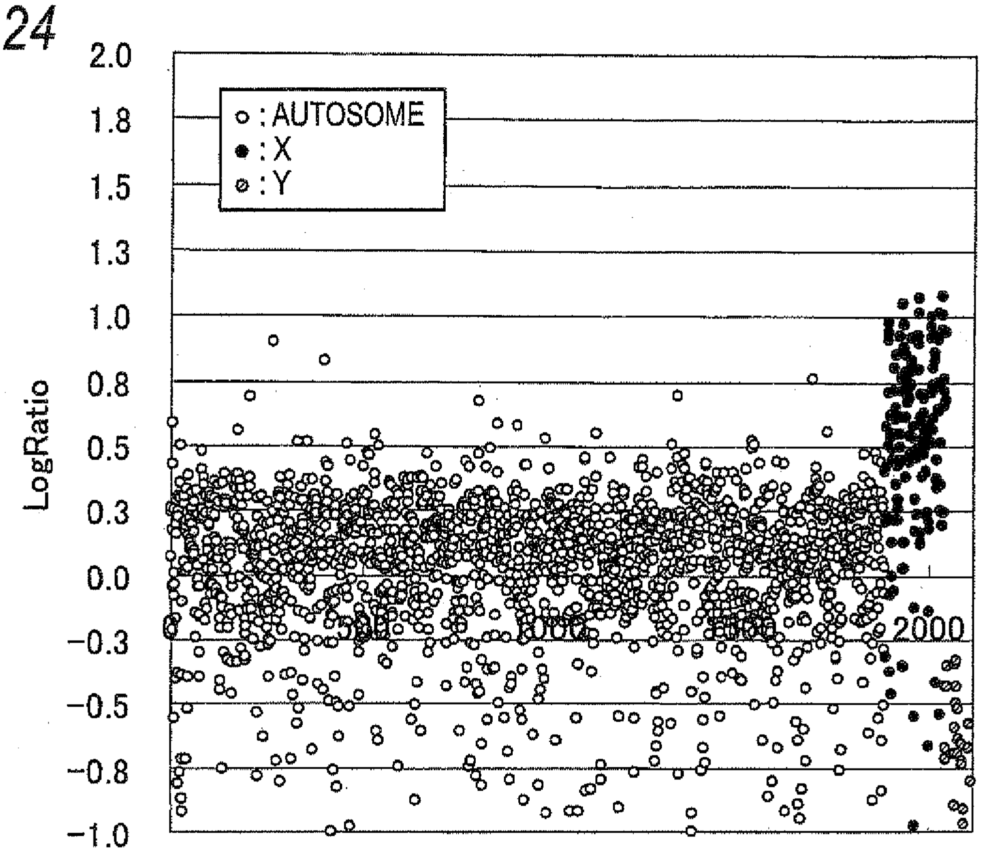
2.0
1.8
1.5
1.3
1.0
0.8
0.5
0.3
0.0
−0.3
−0.5
−0.8
−1.0
LogRatio
○ : AUTOSOME
● : X
⊘ : Y
500
1000
1500
2000

FIG. 25

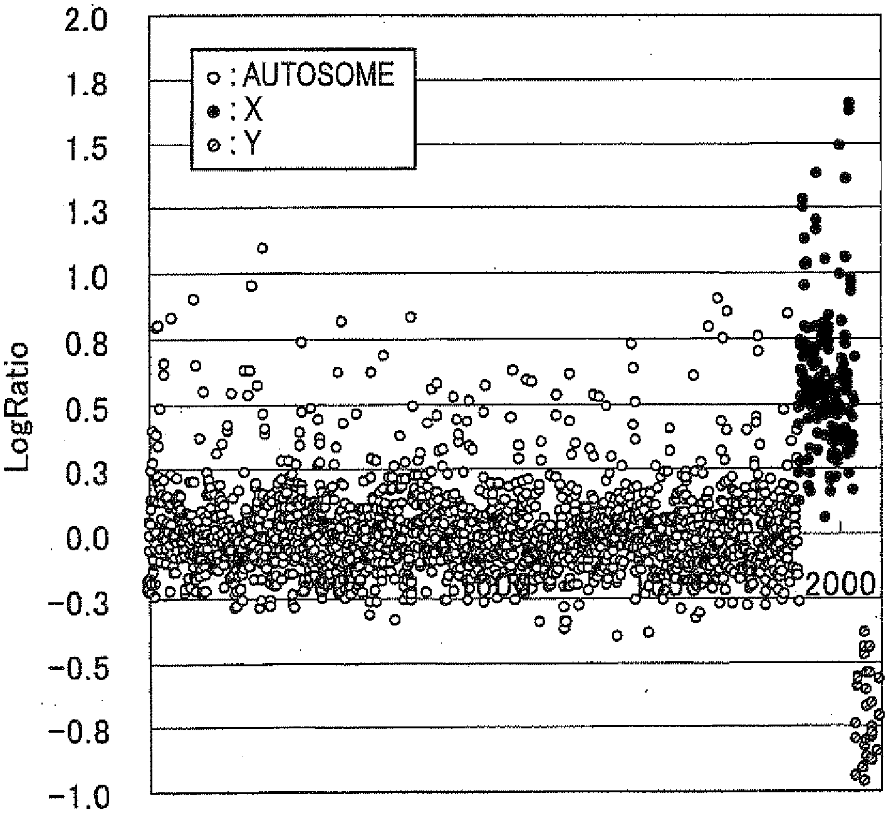
2.0
1.8
1.5
1.3
1.0
0.8
0.5
0.3
0.0
−0.3
−0.5
−0.8
−1.0
LogRatio
○ : AUTOSOME
● : X
⊘ : Y
1000
2000

FIG. 26

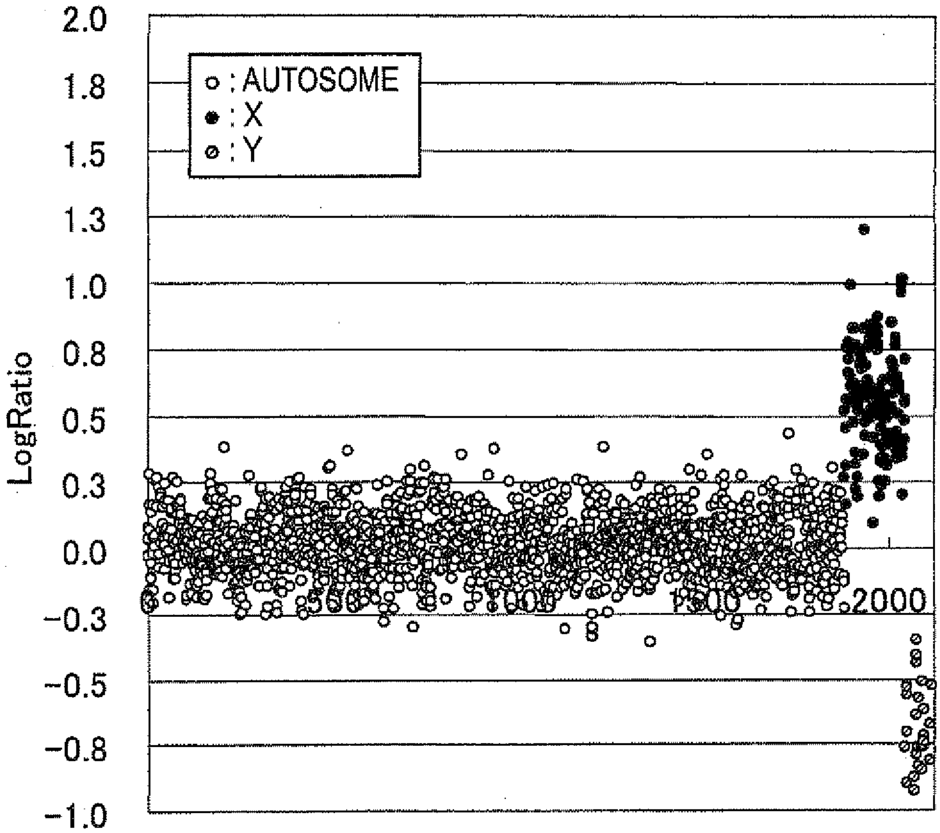
○ : AUTOSOME
● : X
⊘ : Y
LogRatio
2.0
1.8
1.5
1.3
1.0
0.8
0.5
0.3
0.0
−0.3
−0.5
−0.8
−1.0
0
500
1000
1500
2000

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007033210 A
- JP 2000325099 A
- JP 2005087109 A

### Non-patent literature cited in the description


- **A. KALLIONIEMI et al.** *Science,* 1992, vol. 258, 818-821
- **J. INASAWA ; M. MIZUKUCHI ; RINSHO KENSA.** *Clinical Inspection,* 2005, vol. 49, 497-502
- **D. PINKEL et al.** *Nat. Genet.,* 1998, vol. 20, 207-211
- **I. INOUE ; J. INASAWA.** *Saibo Kogaku (Cell Engineering,* 2004, vol. 23, 355-361
- **NARAYANAN et al.** *Journal of Microbiological Methods,* 2009, vol. 77, 261-266
- **I. INOUE ; J. INASAWA.** *Cell Engineering,* 2004, vol. 123, 355
- *Biochemistry,* 1997, vol. 32, 137-144
- *Proc. Natl. Acad. Sci. USA,* 1985, vol. 82, 1585-1588